# EUROPEAN PATENT SPECIFICATION

(11) **EP 3 684 925 B1**
(45) Date of publication and mention of the grant of the patent: **18.08.2021**
(21) Application number: 18769378.3
(22) Date of filing: 19.09.2018
(51) Int. Cl.: C12N 15/00

(54) **NON-INTEGRATING DNA VECTORS FOR THE GENETIC MODIFICATION OF CELLS**
NICHT INTEGRIERENDE DNA-VEKTOREN ZUR GENETISCHEN MODIFIKATION VON ZELLEN
VECTEURS D'ADN NON INTÉGRANTS DESTINÉS À LA MODIFICATION GÉNÉTIQUE DE CELLULES

(30) Priority: 19.09.2017 EP 17191829; 17.09.2018 WO PCT/US2018/051381
(43) Date of publication of application: 29.07.2020
(73) Proprietor: Deutsches Krebsforschungszentrum, 69120 Heidelberg (DE); Nature Technology Corporation, Lincoln, NE 68521 (US)
(72) Inventor: HARBOTTLE, Richard, 68526 Ladenburg (DE); BOZZA, Matthias, 69121 Heidelberg (DE); WILLIAMS, James, A., Lincoln, Nebraska 68516 (US)
(74) Representative: Altmann Stößel Dick Patentanwälte PartG mbB
(86) International application number: PCT/EP2018/075354
(87) International publication number: WO 2019/057774

(56) References cited:
- WO-A1-2014/077863
- WO-A2-2010/018444
- US-A- 5 985 607
- ANJA EHRHARDT ET AL: "Episomal Vectors for Gene Therapy", CURRENT GENE THERAPY, vol. 8, no. 3, 1 June 2008 (2008-06-01), pages 147-161, XP055436780, NL ISSN: 1566-5232, DOI: 10.2174/156652308784746440
- S. C. VERGHESE ET AL: "S/MAR sequence confers long-term mitotic stability on non-integrating lentiviral vector episomes without selection", NUCLEIC ACIDS RESEARCH, 27 January 2014 (2014-01-27), XP055102696, ISSN: 0305-1048, DOI: 10.1093/nar/gku082
- Kamil J Cygan ET AL: "Messenger RNA Splicing Signals" In: "eLS", 15 February 2017 (2017-02-15), John Wiley & Sons, Ltd, Chichester, UK, XP055516645, ISBN: 978-0-470-01617-6 pages 1-8, DOI: 10.1002/9780470015902.a0000888.pub2, the whole document

## Description

The present invention relates to the field of self-replicating non-integrative episomal vertebrate expression vectors useful for in gene therapy, ex vivo cell therapy, stem cell therapy, and more particularly, for improving the expression of vector encoded antigens or therapeutic genes. Such recombinant DNA molecules are useful in biotechnology, transgenic organisms, gene therapy, stem cell therapy, therapeutic vaccination, agriculture and DNA vaccines. More specifically, relates to a polynucleotide comprising at least one promoter and an S/MAR element, wherein said S/MAR element is located downstream of said promoter and wherein the nucleic acid sequence of said S/MAR element (S/MAR sequence) comprises at least 3 sequence motifs ATTA (SEQ ID NO:1) per 100 nucleotides over a stretch of at most 200 nucleotides; the present invention further relates to a composition and to a host cell comprising said polynucleotide, and to the polynucleotide for use in medicine and for use in treating genetic disease. The present invention also relates to a kit and to a device comprising said polynucleotide, and to methods and uses related to the polynucleotide.

Genetic modification of cells is used routinely in modern cell culture for scientific purposes. However, use of corresponding techniques in treatment of inherited diseases caused by mutations of genes, while being highly desirable, still is hampered by the problem that methods available usually only provide transient modification, such as transient transfection protocols, whereas methods providing stable modification of cells such as with viral lentiviral vectors or non-viral transposon vectors usually rely on integration of the transgene into the genome of the host cell. Integration of a transgene, however, even if targeted to a specific locus, bears the risk of inducing a deleterious mutation, which may lead e.g. to cancer as a side effect of treatment.

Scaffold/matrix attachment regions (S/MARs), which are also known as scaffold-attachment regions (SARs) or matrix-associated regions (MARs) are known as sequences in the genome of eukaryotic organisms mediating attachment of the nuclear matrix. The S/MARS are AT rich sequences, and some AT-rich motifs were found to be further enriched (Liebeich et al., (2002), NAR 30(15): 3433). A variety of vectors has been proposed for stable maintenance in cells based on S/MAR motifs, e.g. in US 6,410,314 B1 and in Haase et al., (2010), BMC Biotechnology 10:20; moreover, epigenetic effects having an influence on replication of such vectors were identified (Haase et al., (013), PLOS One 8(11):e79262). Nonetheless, S/MAR based vectors being stable enough for use in gene therapy are needed.

Suboptimal expression level, gene silencing and low establishment rate represent the major limitations of S/MAR based vectors described in the art.

There is, therefore a need for improved means and methods for stable transfection of cells, in particular using S/MAR elements and avoiding the risks involved with integration of the transgene into the genome of the host cell. This problem is solved by the means and methods disclosed herein.

The present invention relates to vectors useful for non-integrative episomal gene therapy and stem cell therapy, and more particularly, for improving transgene expression and vector establishment efficiency of a self-replicating non-integrative episomal S/MAR expression vector, and for eliminating antibiotic resistance marker gene transfer by non-viral vectors.

Improved vector methods and compositions that improve the expression and establishment efficiency of a self-replicating non-integrative episomal S/MAR expression vector in a target vertebrate cell are disclosed.

One object of the invention is to provide improved expression of a self-replicating non-integrative episomal S/MAR expression vector in a target vertebrate cell.

Another object of the invention is to provide improved establishment efficiency of a self-replicating non-integrative episomal S/MAR expression vector in a target vertebrate cell.

In one embodiment, the present technology provides a method for improving the expression and establishment efficiency of a self-replicating non-integrative episomal S/MAR expression vector in a target vertebrate cell comprising the following steps: a) providing a episomal S/MAR expression vector comprising: i) a bacterial replication-selection region comprising a bacterial origin of replication and a selectable marker; ii) a transcription unit for expression of a transgene in a vertebrate cell, comprising a promoter, a 5' UTR, a transgene, and a 3' UTR; iii) an S/MAR insert located within said 3' UTR; and b) modifying the episomal S/MAR expression vector such that the S/MAR is flanked by a 5' splice donor site and a 3' splice acceptor site within said 3' UTR, whereby the resultant self-replicating non-integrative episomal S/MAR expression vector has improved the expression and establishment efficiency after transfection of a vertebrate cell. In a further embodiment said S/MAR contains internal AATAAA transcription termination motifs. In a further embodiment said AATAAA transcription termination motifs in said S/MAR are replaced with AATATT motifs. In a further embodiment said S/MAR is selected from the group consisting of human Interferon beta S/MAR, M18 S/MAR, ApoL1 S/MAR. In a further embodiment said SMAR flanked by a 5' splice donor site and a 3' splice acceptor site has at least 95% sequence identity to a sequence selected from the group consisting of SEQ ID NO: 19, SEQ ID NO: 20, SEQ ID NO: 21, SEQ ID NO: 22, and SEQ ID NO: 23. In a further embodiment said bacterial origin of replication is an R6K gamma replication origin. In a further embodiment said bacterial origin of replication is an R6K gamma replication origin with at least 95% sequence identity to a sequence selected from the group consisting of SEQ ID NO: 1, SEQ ID NO: 2, SEQ ID NO: 3, and SEQ ID NO: 4. In a further embodiment said selectable marker is an RNA-IN regulating RNA-OUT functional variant with at least 95% sequence identity to a sequence selected from the group consisting of SEQ ID NO: 5, and SEQ ID NO: 7. In a further embodiment said selectable marker is an RNA-OUT RNA selectable marker that encodes an RNA-IN regulating RNA-OUT RNA with at least 95% sequence identity to SEQ ID NO: 6. In a further embodiment said bacterial replication-selection region comprising a bacterial origin of replication and a selectable marker is a R6K origin-RNA-OUT RNA selectable marker bacterial replication-selection region with at least 95% sequence identity to a sequence selected from the group consisting of SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, and SEQ ID NO: 17. In a further embodiment said 5' UTR further encodes an intron. In a further embodiment said transcription unit further encodes an expression enhancer positioned upstream of the promoter. In a further embodiment said expression enhancer has at least 95% sequence identity to a sequence selected from the group consisting of SEQ ID NO: 27, and SEQ ID NO: 28. In a further embodiment said splice donor site has at least 95% sequence identity to SEQ ID NO:25. In a further embodiment said splice acceptor site has at least 95% sequence identity to SEQ ID NO: 26. In a further embodiment said self-replicating non-integrative episomal S/MAR expression vector is selected from the group consisting of plasmid vector, Nanoplasmid vector, Integration-Deficient Lentivirus vector, and Non-integrating Lentiviral vectors.

In another embodiment, the present technology provides an antibiotic marker free covalently closed circular recombinant DNA molecule comprising: a) an antibiotic marker free transcription unit for expression of a transgene in a vertebrate cell, comprising a promoter, a 5' UTR, a transgene, and a 3' UTR; b) an S/MAR located within said 3' UTR wherein said S/MAR is flanked by a 5' splice donor site and a 3' splice acceptor site; c) an R6K gamma replication origin with at least 95% sequence identity to a sequence selected from the group consisting of SEQ ID NO: 1, SEQ ID NO: 2, SEQ ID NO: 3, and SEQ ID NO: 4; and d) an RNA-OUT RNA selectable marker comprising an RNA-IN regulating RNA-OUT functional variant with at least 95% sequence identity to a sequence selected from the group consisting of SEQ ID NO: 5, and SEQ ID NO: 7. In a further embodiment said R6K gamma replication origin and said RNA-OUT RNA selectable marker comprise a R6K origin-RNA-OUT RNA selectable marker bacterial replication-selection region with at least 95% sequence identity to a sequence selected from the group consisting of SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, and SEQ ID NO: 17. In a further embodiment said S/MAR is selected from the group consisting of human Interferon beta S/MAR, M18 S/MAR, ApoL1 S/MAR. In a further embodiment said S/MAR contains internal AATAAA transcription termination motifs. In a further embodiment said AATAAA transcription termination motifs in said S/MAR are replaced with AATATT motifs. In a further embodiment said S/MAR is selected from the group consisting of human Interferon beta S/MAR, M18 S/MAR, ApoL1 S/MAR. In a further embodiment said SMAR flanked by a 5' splice donor site and a 3' splice acceptor site has at least 95% sequence identity to a sequence selected from the group consisting of SEQ ID NO: 19, SEQ ID NO: 20, SEQ ID NO: 21, SEQ ID NO: 22, and SEQ ID NO: 23. In a further embodiment said 5' UTR further encodes an intron. In a further embodiment said transcription unit further encodes an expression enhancer positioned upstream of the promoter. In a further embodiment said expression enhancer has at least 95% sequence identity to a sequence selected from the group consisting of SEQ ID NO: 27, and SEQ ID NO: 28. In a further embodiment said splice donor site has at least 95% sequence identity to SEQ ID NO:25. In a further embodiment said splice acceptor site has at least 95% sequence identity to SEQ ID NO: 26.

In another embodiment, the present technology provides an covalently closed circular recombinant DNA molecule comprising: a) an transcription unit for expression of a transgene in a vertebrate cell, comprising a promoter, a 5' UTR, a transgene, and a 3' UTR; b) an S/MAR located within said 3' UTR wherein said S/MAR is flanked by a 5' splice donor site and a 3' splice acceptor site; c) an R6K gamma replication origin with at least 95% sequence identity to a sequence selected from the group consisting of SEQ ID NO: 1, SEQ ID NO: 2, SEQ ID NO: 3, and SEQ ID NO: 4; and d) an RNA-OUT RNA selectable marker comprising an RNA-IN regulating RNA-OUT functional variant with at least 95% sequence identity to a sequence selected from the group consisting of SEQ ID NO: 5, and SEQ ID NO: 7. In a further embodiment said R6K gamma replication origin and said RNA-OUT RNA selectable marker comprise a R6K origin-RNA-OUT RNA selectable marker bacterial replication-selection region with at least 95% sequence identity to a sequence selected from the group consisting of SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, and SEQ ID NO: 17. In a further embodiment said S/MAR is selected from the group consisting of human Interferon beta S/MAR, M18 S/MAR, ApoL1 S/MAR. In a further embodiment said S/MAR contains internal AATAAA transcription termination motifs. In a further embodiment said AATAAA transcription termination motifs in said S/MAR are replaced with AATATT motifs. In a further embodiment said S/MAR is selected from the group consisting of human Interferon beta S/MAR, M18 S/MAR, ApoL1 S/MAR. In a further embodiment said SMAR flanked by a 5' splice donor site and a 3' splice acceptor site has at least 95% sequence identity to a sequence selected from the group consisting of SEQ ID NO: 19, SEQ ID NO: 20, SEQ ID NO: 21, SEQ ID NO: 22, and SEQ ID NO: 23. In a further embodiment said 5' UTR further encodes an intron. In a further embodiment said transcription unit further encodes an expression enhancer positioned upstream of the promoter. In a further embodiment said expression enhancer has at least 95% sequence identity to a sequence selected from the group consisting of SEQ ID NO: 27, and SEQ ID NO: 28. In a further embodiment said splice donor site has at least 95% sequence identity to SEQ ID NO:25. In a further embodiment said splice acceptor site has at least 95% sequence identity to SEQ ID NO: 26.

The resultant plasmids with a S/MAR flanked by a 5' splice donor site and a 3' splice acceptor site within the 3' UTR have surprisingly improved establishment and transgene expression than plasmids with a S/MAR within the 3' UTR without flanking splice donor and acceptor sites.

### BRIEF DESCRIPTION OF THE FIGURES

FIG. 1 depicts the pCI intron, with splice donor (SD) branch point and splice acceptor (SA) regions. FIG. 2 depicts the interferon beta S/MAR (top), and a SD interferon beta S/MAR SA derivative (middle), as well as a SD interferon beta S/MAR SA derivative in which the internal AATAAA polyadenylation signals were mutated (bottom).
FIG. 3 depicts the interferon beta S/MAR derivative M18 with flanking SD and SA sites.
FIG. 4 depicts the 805 bp (top) or 525 bp (bottom) apoB S/MAR with flanking SD and SA sites.
FIG. 5 depicts the pMAX-UCOE-coGFP P2A-PuroR-NP (pSMARt UCOE) vector.
FIG. 6 depicts the NTC9385R-UCOE-CMV- coGFP P2A-PuroR -SMAR-SV40 pA (NP-UCOE) and NTC9385R-UCOE-CMV- coGFP P2A-PuroR - SD SMAR- SA SV40 pA (NP-UCOE-SP) vectors.
FIG. 7 depicts the NTC9385R-SP-UCOE-CMV-GFP SMARter (NP-SMARter-SP) and NTC9385R- SP-UCOE-CMV-GFP CMARter (NP-CMARter-SP) vectors.
FIG. 8 depicts the NTC9385R- UCOE EF1 -coGFP SD -SMAR SA SV40 pA (NP-UCOE-EF1-SP) and NTC9385R- UCOE EFl-coGFP-SD SMAR R6K-R-OUT-SA pA (UCOE-EF1-SP-NP) vectors.
FIG. 9 depicts the NTC9385R-SP-ELE40-CMV-GFP CMARter (NP-Ele40-CMARter-SP) vector.

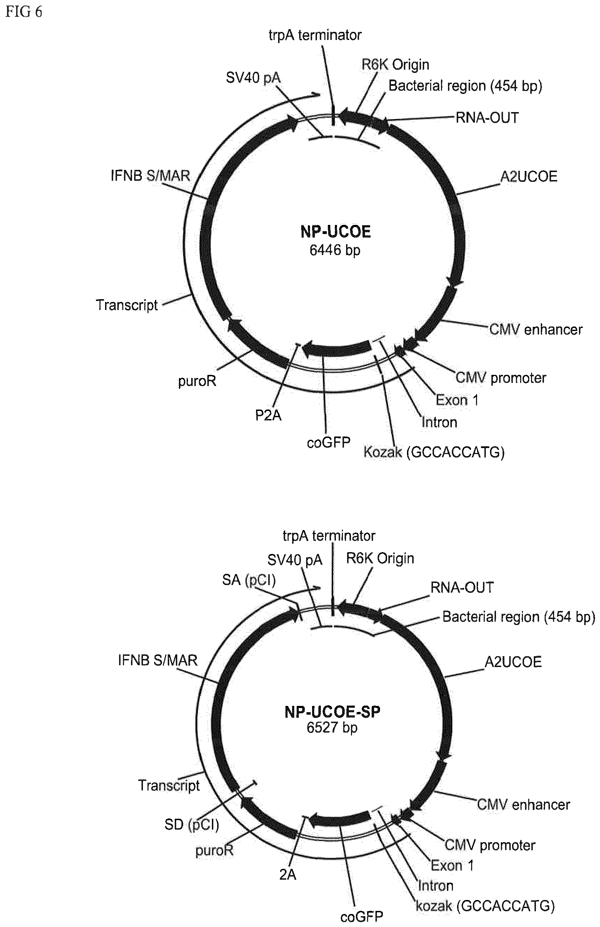

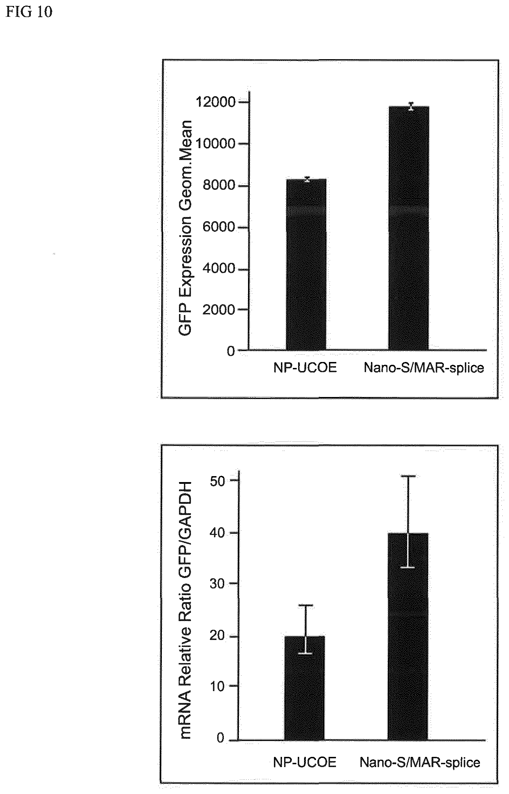

FIG. 10 depicts improved expression of established S/MAR vectors with and without flanking SD and SA sites. Left panel: MFI of HEK293T cells established with a S/MAR vector with and without splice junctions. The vectors contain NP bacterial region, the genomic insulator UCOE, the expression cassette GFP -2A-PuroR driven by the CMV promoter and the interferon beta S/MAR in the 3' UTR with (Nano-S/MAR-splice = NP-UCOE-SP; NTC9385R-UCOE-CMV- coGFP P2A- PuroR - SD SMAR- SA SV40 pA Figure 6) or without (NP-UCOE; NTC9385R-UCOE-CMV- coGFP P2A-PuroR -SMAR-SV40 pA, Figure 6) S/MAR flanking SD and SA sites. Right panel: the improved transcription expression is confirmed by real time PCR analysis. The expression of the transgene GFP was normalized to the housekeeping gene GAPDH.
FIG. 11 depicts improved expression of established S/MAR vectors with and without flanking SD and SA sites. MFI of established cells (HEK293T and primary Mouse Embryonic Fibroblast) with vectors harboring different S/MARs flanked by splicing junctions. Vector names are as in Figures 5, 6 and 7.
FIG. 12 depicts improved establishment of S/MAR vectors with and without flanking SD and SA sites. Colony forming assay conducted in HEK293T with vectors harboring two different S/MARs with and without flanking SD and SA sites. pEPI is a CMV promoter plasmid vector with a 3' UTR interferon beta S/MAR.

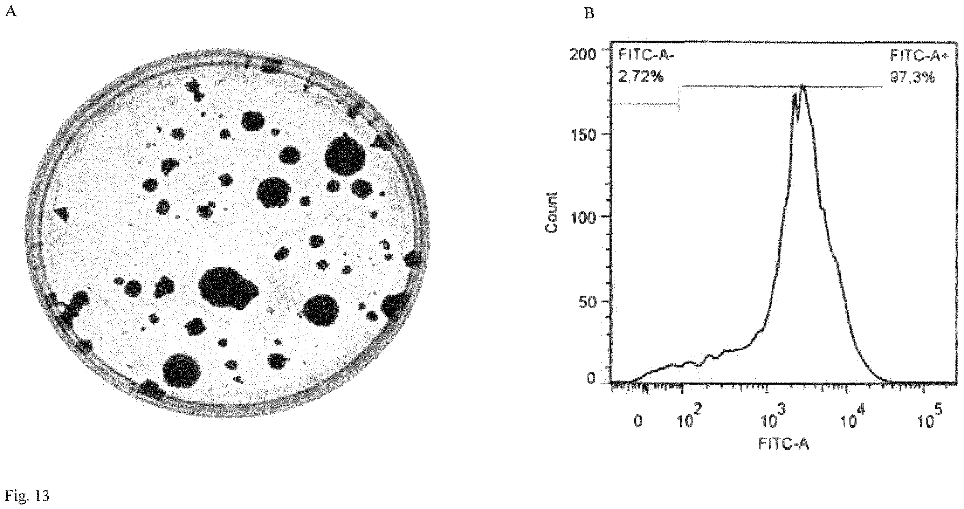

FIG. 13: Efficiency of establishment and analysis of the genetically modified cell population: A) A cell culture plate with Crystal Violet stained colonies having formed after 4 weeks selection with Puromycin; the efficiency of vector establishment was approximately 40%; b) FACS detection of GFP fluorescence in Puromycin selected cells; fluorescence is very homogenous and the number of non-fluorescing cells is extremely low.

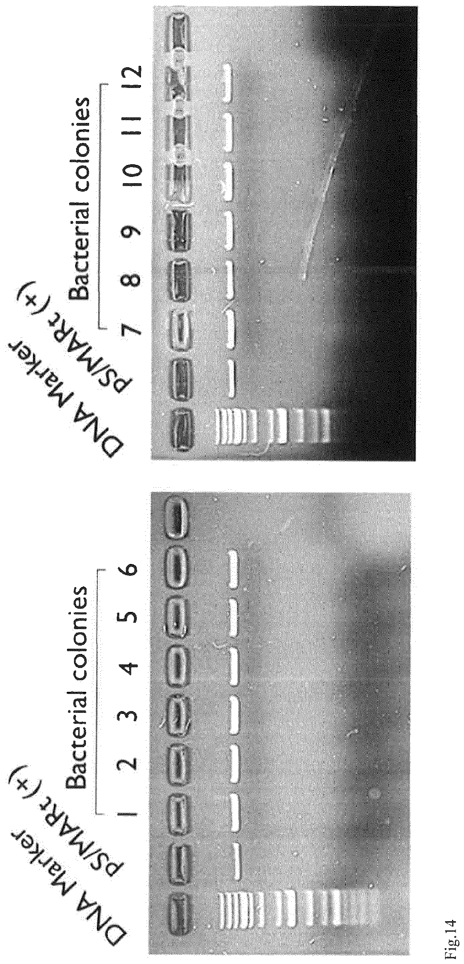

FIG.14: Result of plasmid rescue of pS/MARt vectors from established cell populations: DNA from bacterial colonies (numbers 1 to 12) obtained in a plasmid rescue experiment were digested with BamHI and were resolved by agarose gel electrophoresis; the lanes labeled with "p/SMART" contain DNA from a colony of bacteria carrying the original plasmid treated the same as above.

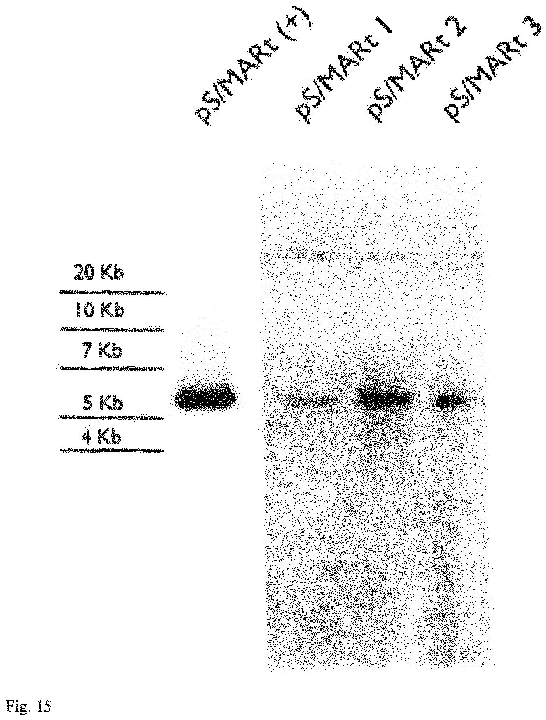

FIG. 15: Southern blot of pSMARt vectors maintained in selected cells: oligonucleotides hybridizing to the GFP gene of pS/MART were used as probes to detect BamHI restricted vector DNA in extracts from host cells (pS/MARt1 to 3); the non-transfected vector was used as a control ("pS/MARt(+)").

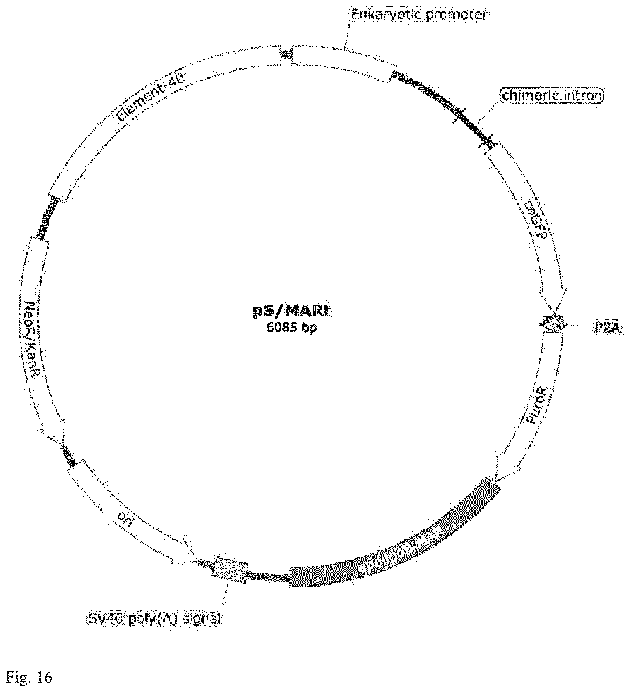

FIG. 16: Vector map of pS/MART; ori: bacterial origin of replication, P2A: sequence encoding the self-cleaving 2A peptide from porcine teschovirus-1, apolipoB MAR: S/MAR sequence from the apolipoprotein B gene.

Table 1: pNTC multiple cloning site flanked R6K Origin-RNA-OUT selection marker vectors.
Table 2: Transient expression of S/MAR vectors after transfection into A549 and HEK293 cell lines.
Table 3: Transient expression of S/MAR vectors after transfection into A549 and HEK293 cell lines.
Table 4: Transient expression of S/MAR vectors after transfection into A549 and HEK293 cell lines.

SEQ ID NO:1: R6K gamma origin
SEQ ID NO:2: 1 CpG R6K gamma origin
SEQ ID NO:3: CpG free R6K gamma origin
SEQ ID NO:4: Extended R6K gamma origin
SEQ ID NO:5: RNA-OUT Selectable Marker
SEQ ID NO:6: RNA-OUT antisense repressor RNA
SEQ ID NO:7: 2 CpG RNA-OUT Selectable Marker SEQ ID NO:8: R6K gamma origin-RNA-OUT bacterial region flanked by NheI and KpnI restriction sites
SEQ ID NO:9: 1 CpG R6K gamma origin-2 CpG RNA-OUT bacterial region flanked by NheI and KpnI restriction sites
SEQ ID NO:10: pNTC-NP1 polylinker trpA R6K-RNA-OUT polylinker cloning cassette: EcoRI/HindIII
SEQ ID NO:11: pNTC-NP2 polylinker trpA R6K-RNA-OUT polylinker cloning cassette: EcoRI/HindIII
SEQ ID NO:12: pNTC-NP3 polylinker trpA R6K-RNA-OUT polylinker cloning cassette: EcoRI/HindIII
SEQ ID NO:13: pNTC-NP4 polylinker trpA R6K-RNA-OUT polylinker cloning cassette: EcoRI/HindIII
SEQ ID NO:14: pNTC-NP5 polylinker trpA R6K-RNA-OUT polylinker cloning cassette: KasI/HindIII
SEQ ID NO:15: pNTC-NP6 polylinker trpA R6K-RNA-OUT polylinker cloning cassette: EcoRI/SacI
SEQ ID NO:16: pNTC-NP7 polylinker trpA R6K-RNA-OUT polylinker cloning cassette: BssHII/BssHII
SEQ ID NO:17: pNTC-3xCpG NP1 polylinker R6K-RNA-OUT polylinker cloning cassette: HindIII/EcoRI
SEQ ID NO:18: Human Interferon beta S/MAR flanked by 5' BglII-XhoI site and 3' EcoRI restriction enzyme sites
SEQ ID NO:19: Splice donor-human Interferon beta S/MAR-splice acceptor flanked by 5' BglII site and 3' BamHI restriction enzyme sites
SEQ ID NO:20: Splice donor-human Interferon beta S/MAR (-AATAAA)-splice acceptor flanked by 5' BglII site and 3' BamHI restriction enzyme sites
SEQ ID NO:21: Splice donor-human Interferon beta M18 S/MAR-splice acceptor flanked by 5' BglII site and 3' BamHI restriction enzyme sites
SEQ ID NO:22: Splice donor-805 bp human Apolipoprotein B S/MAR-splice acceptor flanked by 5' BglII site and 3' BamHI restriction enzyme sites
SEQ ID NO:23: Splice donor-525 bp human Apolipoprotein B S/MAR-splice acceptor flanked by 5' NsiI site and 3' BamHI restriction enzyme sites
SEQ ID NO:24: pCI intron
SEQ ID NO:25: pCI Splice donor
SEQ ID NO:26: pCI Splice acceptor (murine IgG)
SEQ ID NO:27: Ele40 expression enhancer
SEQ ID NO:28: A2UCOE expression enhancer
SEQ ID NO:29: Splice acceptor consensus sequence
SEQ ID NO: 30: sequence motif
SEQ ID NO: 31: sequence motif
SEQ ID NO: 32: sequence motif
SEQ ID NO: 33: sequence motif
SEQ ID NO: 34: sequence motif
SEQ ID NO: 35: sequence motif
SEQ ID NO: 36: puromycin acetyltransferase, synthetic construct
SEQ ID NO: 37: puromycin acetyltransferase encoding sequence, synthetic
SEQ ID NO: 38: anti-repressive element40
SEQ ID NO: 39: CMV Promoter - S/MAR sequence
SEQ ID NO: 40: CMV Promoter - Puromycin - S/MAR sequence
SEQ ID NO: 41: Element40-GPF-P2A-Puromycin-S/MAR

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

Moreover, if not otherwise indicated, the term "about" relates to the indicated value with the commonly accepted technical precision in the relevant field, preferably relates to the indicated value ± 20%, more preferably ± 10%, most preferably ± 5%. Further, the term "essentially" indicates that deviations having influence on the indicated result or use are absent, i.e. potential deviations do not cause the indicated result to deviate by more than ± 20%, more preferably ± 10%, most preferably ± 5%. Thus, "consisting essentially of' means including the components specified but excluding other components except for materials present as impurities, unavoidable materials present as a result of processes used to provide the components, and components added for a purpose other than achieving the technical effect of the invention. For example, a composition defined using the phrase "consisting essentially of' encompasses any known acceptable additive, excipient, diluent, carrier, and the like. Preferably, a composition consisting essentially of a set of components will comprise less than 5% by weight, more preferably less than 3% by weight, even more preferably less than 1%, most preferably less than 0.1% by weight of non-specified component(s). In the context of nucleic acid sequences, the term "essentially identical" indicates a %identity value of at least 80%, preferably at least 90%, more preferably at least 98%, most preferably at least 99%. As will be understood, the term essentially identical includes 100% identity. The aforesaid applies to the term "essentially complementary" mutatis mutandis.

The term "polynucleotide", as used herein, refers to a linear or circular nucleic acid molecule. The term encompasses single as well as partially or completely double-stranded polynucleotides. Preferably, the polynucleotide is RNA or is DNA, including cDNA. Moreover, comprised are also chemically modified polynucleotides including naturally occurring modified polynucleotides such as glycosylated or methylated polynucleotides or artificially modified derivatives such as biotinylated polynucleotides. The polynucleotide of the present invention shall be provided, preferably, either as an isolated polynucleotide (i.e. isolated from its natural context) or in genetically modified form. The polynucleotide of the invention comprises at least one promoter active in a host cell and an S/MAR element; moreover, the polynucleotide has the biological activity of replicating episomally in a host cell, all as specified herein below. Preferably, the polynucleotide has a length of at most 1 Mb, more preferably at most 500 kb, even more preferably at most 200 kb, most preferably at most 100 kb. Preferably, the polynucleotide is a non-naturally occurring polynucleotide; thus, preferably, the nucleotide is an artificial polynucleotide. Also preferably, the polynucleotide is a chimeric polynucleotide; more preferably, the polynucleotide comprises at least one nucleic acid sequence heterologous to the remaining nucleic acid sequences it comprises.

As used herein, the term polynucleotide, preferably, includes variants of the specifically indicated polynucleotides. More preferably, the term polynucleotide relates to the specific polynucleotides indicated. The term "polynucleotide variant", as used herein, relates to a variant of a polynucleotide related to herein comprising a nucleic acid sequence characterized in that the sequence can be derived from the aforementioned specific nucleic acid sequence by at least one nucleotide substitution, addition and/or deletion, wherein the polynucleotide variant shall have the biological activity or activities as specified for the specific polynucleotide. Thus, it is to be understood that a polynucleotide variant as referred to in accordance with the present invention shall have a nucleic acid sequence which differs due to at least one nucleotide substitution, deletion and/or addition. Preferably, said polynucleotide variant comprises an ortholog, a paralog or another homolog of the specific polynucleotide or of a functional subsequence thereof, e.g. of an S/MAR element. Also preferably, said polynucleotide variant comprises a naturally occurring allele of the specific polynucleotide or of a functional subsequence thereof. Polynucleotide variants also encompass polynucleotides comprising a nucleic acid sequence which is capable of hybridizing to the aforementioned specific polynucleotides or functional subsequences thereof, preferably, under stringent hybridization conditions. These stringent conditions are known to the skilled worker and can be found in standard textbooks A preferred example for stringent hybridization conditions are hybridization conditions in 6x sodium chloride/sodium citrate (= SSC) at approximately 45°C, followed by one or more wash steps in 0.2x SSC, 0.1% SDS at 50 to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of 0.1x to 5x SSC (pH 7.2). If organic solvent is present in the abovementioned buffer, for example 50% formamide, the temperature under standard conditions is approximately 42°C. The hybridization conditions for DNA:DNA hybrids are preferably for example 0.1x SSC and 20°C to 45°C, preferably between 30°C and 45°C. The hybridization conditions for DNA:RNA hybrids are preferably, for example, 0.1x SSC and 30°C to 55°C, preferably between 45°C and 55°C. The abovementioned hybridization temperatures are determined for example for a nucleic acid with approximately 100 bp (= base pairs) in length and a G+C content of 50% in the absence of formamide; accordingly, other conditions more suitable for low-G+C DNA, which are in principle known to the skilled person, may be found to be more appropriate by the skilled person. The skilled worker knows how to determine the hybridization conditions required by referring to standard textbooks. Alternatively, polynucleotide variants are obtainable by PCR-based techniques such as mixed oligonucleotide primer- based amplification of DNA, i.e. using degenerated primers against conserved domains of a polypeptide of the present invention. Conserved domains of a polypeptide may be identified by a sequence comparison of the nucleic acid sequence of the polynucleotide or the amino acid sequence of the polypeptide of the present invention with sequences of other organisms. As a template, DNA or cDNA from bacteria, fungi, plants or, preferably, from animals may be used. Further, variants include polynucleotides comprising nucleic acid sequences which are at least 70%, at least 75%, at least 80%, at least 85%, at least 90%, at least 95%, at least 98% or at least 99% identical to the specifically indicated nucleic acid sequences or functional subsequences thereof. Moreover, also encompassed are polynucleotides which comprise nucleic acid sequences encoding amino acid sequences which are at least 70%, at least 75%, at least 80%, at least 85%, at least 90%, at least 95%, at least 98% or at least 99% identical to the amino acid sequences specifically indicated. The percent identity values are, preferably, calculated over the entire amino acid or nucleic acid sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (J. Mol. Evolution., 25, 351-360, 1987, Higgins et al., CABIOS, 5 1989: 151-153) or the programs Gap and BestFit (Needleman and Wunsch (J. Mol. Biol. 48; 443-453 (1970)) and Smith and Waterman (Adv. Appl. Math. 2; 482-489 (1981))), are preferably used. Preferably, said programs are used with their standard parameters. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

A polynucleotide comprising a fragment of any of the specifically indicated nucleic acid sequences, said polynucleotide retaining the indicated activity or activities, is also encompassed as a variant polynucleotide of the present invention. A fragment as meant herein, preferably, comprises at least 200, preferably at least 300, more preferably at least 400 consecutive nucleotides of any one of the specific nucleic acid sequences; or encodes an amino acid sequence comprising at least 100, preferably at least 200, more preferably at least 300 consecutive amino acids of any one of the specific amino acid sequences and still having the indicated activity.

The polynucleotides of the present invention either consist, essentially consist of, or comprise the aforementioned nucleic acid sequences. Thus, they may contain further nucleic acid sequences as well. Specifically, the polynucleotides of the present invention may encode e.g. fusion proteins or selectable markers. Such fusion proteins may comprise as additional part polypeptides for monitoring expression (e.g., green, yellow, blue or red fluorescent proteins, alkaline phosphatase and the like) or so called "tags" which may serve as a detectable marker or as an auxiliary measure for purification purposes. Tags for the different purposes are well known in the art and are described elsewhere herein.

Also preferably, the polynucleotide comprises at least one cargo sequence. The term "cargo sequence", as used herein, relates to a nucleic acid sequence of interest of being transferred into and stably maintained in a host cell. Preferably, the cargo sequence is a nucleic acid sequence encoding a polynucleotide, e.g. an RNA, and/or a polypeptide of interest. Preferably, the polypeptide of interest is a therapeutic polypeptide, more preferably a T Cell Receptor (TCR), more preferably a human or chimeric T Cell receptor, a Chimeric Antigen Receptor (CAR), preferably MART1 TCR, or a polypeptide lacking in cells affected with a genetic disease as specified elsewhere herein. Thus, e.g. preferably, the polynucleotide comprises at least one cargo sequence encoding a polypeptide providing phenylalanine-hydroxylase activity (EC 1.14.16.1) for treatment of phenylketonuria.

Preferably, the sequence encoding a selectable marker and the cargo sequence are intervened by a sequence enabling expression of two (or more) polypeptides in a eukaryotic cell from one mRNA, e.g. an internal ribosomal entry sequence (IRES) or, more preferably, a self-cleaving peptide sequence such as, most preferably, a peptide 2A (P2A) sequence from porcine teschovirus-1. Appropriate sequences are known in the art, e.g. from Kim et al. (2011) PLoS ONE 6(4): e18556.

Preferably, the polynucleotide is a DNA. Preferably, the polynucleotide comprises further expression control sequences allowing expression of genes in prokaryotic and/or eukaryotic, preferably in eukaryotic host cells or isolated fractions thereof. Expression of said polynucleotide comprises transcription of the polynucleotide, preferably into a translatable mRNA. Regulatory elements ensuring expression in eukaryotic cells, preferably mammalian cells, are well known in the art. They, preferably, comprise regulatory sequences ensuring initiation of transcription and, optionally, poly-A signals ensuring termination of transcription and stabilization of the transcript. Additional regulatory elements may include transcriptional as well as translational enhancers. Examples for regulatory elements permitting expression in eukaryotic host cells are the AOX1 or GAL1 promoter in yeast or the SMVP-, U6-, H1-, 7SK-, CMV- EFS-, SV40-, or RSV-promoter (Rous sarcoma virus), CMV-enhancer, SV40-enhancer or a globin intron in mammalian and other animal cells. Moreover, inducible or cell type-specific expression control sequences may be comprised in a polynucleotide of the present invention. Inducible expression control sequences may comprise tet or lac operator sequences or sequences inducible by heat shock or other environmental factors. Suitable expression control sequences are well known in the art. Besides elements which are responsible for the initiation of transcription, such regulatory elements may also comprise transcription termination signals, such as the SV40-poly-A site or the tk-poly-A site, downstream of the polynucleotide.

The term "host cell", as used herein, relates to any cell capable of receiving and stably replicating the polynucleotide. Preferably, the host cell is a eukaryotic cell, preferably a plant or yeast cell, e.g. a cell of a strain of baker's yeast, or is an animal cell. More preferably, the host cell is an insect cell or a mammalian cell, in particular a mouse or rat cell. Even more preferably, the host cell is a mammalian cell, most preferably is a human cell. Preferably, the host cell is a CD34+ Progenitor Cell; a CD61+ Thrombocyte; a CD19+ B-Lymphocyte; a CD14+ Monocyte; a CD15+ Granulocyte; a CD3+ Cytotoxic T-Lymphocyte, preferably also positive for CD8 and CD45; a CD3+ Helper T-Lymphocyte, preferably also positive for CD4 and CD45; a CD3+ activated T-Lymphocyte, preferably also positive for CD25 and CD45, a Tumor infiltrating Lymphocyte, or a Natural Killer (NK) cell. As will be understood by the skilled person, the polynucleotide may in addition have sequences permitting replication in a bacterial cell, in particular a bacterial origin of replication. Preferably, the bacterial cell is a cell of a laboratory strain of bacteria, more preferably an Escherichia coli cell.

The term "promoter" is, in principle, known to the skilled person as a genetic element directing, optionally in concert with further regulatory elements, the level of transcription of a given gene. A promoter may be constitutive, i.e. providing a constant level of transcription essentially independent of a host cell's state, or may be regulated, i.e. provide levels of transcription in dependence of a host cell's state. Moreover, a promoter may be cell type and/or tissue specific, i.e. provide a detectable level of transcription only in a few or only one cell type. Preferably, the promoter according to the present invention is active in the host cell as specified herein above. As will be understood by the skilled person, the selection of promoter may depend on the type of host cell intended for targeting; suitable promoters for specific cell types as well as constitutive promoters are known in the art. Preferably, the promoter is a eukaryotic promoter, more preferably a constitutive eukaryotic promoter, even more a strong eukaryotic promoter. Preferably, the promoter is an EF1alpha (elongation factor 1 alpha) promoter, an UbiC (ubiquitin C) promoter, a ROSA 26 promoter, a PGK (phosphoglycerate kinase) promoter, and/or a CAG (chicken alpha-actin) promoter, more preferably is an EF1alpha promoter. Also preferably, the promoter is a cell- and/or tissue-specific eukaryotic promoter. As used herein, the term "promoter" is used for the promoter as specified above, whereas any other promoter potentially present on the polynucleotide in addition is referred to as "secondary promoter". Thus, preferably, the promoter is a promoter directing transcription into the S/MAR sequence in a host cell; also preferably, a promoter not directing transcription into the S/MAR sequence of the polynucleotide, e.g. for being a prokaryotic promoter, for being transcriptionally insulated from the S/MAR sequence, and/or for being a promoter directing transcription away from the S/MAR sequence, is a secondary promoter. Preferably, the promoter comprises less than 1000, more preferably less than 250, even more preferably less than 100, most preferably less than 20 contiguous base pairs corresponding to an Apolipoprotein B promoter; thus, preferably, the polynucleotide does not comprise a human Apolipoprotein B promoter, more preferably does not comprise an Apolipoprotein B promoter.

Preferably, the S/MAR sequence is located immediately downstream of the promoter and, if present, of the selectable marker gene as specified herein below. Preferably, being located "immediately downstream" is lacking an intervening transcription termination signal, more preferably is lacking an intervening gene. Thus, preferably, transcripts initiated at the promoter and, if encoded, including the detectable marker sequence preferably comprise a transcribed S/MAR sequence, more preferably comprise the complete S/MAR sequence comprised in the polynucleotide; as will be understood by the skilled person in view of the description elsewhere herein, the polynucleotide may further include splicing sites mediating excision of the S/MAR sequence from the primary transcript; thus, more preferably, preferably, at least primary transcripts initiated at the promoter and, if encoded, including the detectable marker sequence preferably comprise a transcribed S/MAR sequence, more preferably comprise the complete S/MAR sequence comprised in the polynucleotide. Also preferably, the term "immediately downstream" includes a polynucleotide in which the promoter and the S/MAR sequence are separated by elongated nucleic acid sequences, provided a transcription termination signal is not intervening the promoter and the S/MAR.

Preferably, the sequence intervening the promoter or, if present, the stop codon of the selectable marker gene and the S/MAR sequence has a length of at most 2 kb, more preferably at most 0.5 kb, even more preferably at most 0.2 kb, still more preferably at most 0.1 kb, most preferably at most 50 bp.

The term "S/MAR element", also known under the designation "scaffold/matrix attachment region", is, in principle, known to the skilled person to relate to a DNA sequence mediating attachment of the nuclear matrix of a eukaryotic cell to said DNA. S/MAR sequences typically are derived from sequences in the DNA of eukaryotic chromosomes. A variety of S/MAR sequences is available, and sequences are available from public databases, e.g. as described in Liebich et al. (2002), Nucleic Acids Res. 30, 312-374. According to the present invention, the nucleic acid sequence of said S/MAR element (hitherto referred to as S/MAR sequence) comprises at least 3 sequence motifs ATTA per 100 nucleotides over a stretch of at most 200 nucleotides. Thus, the motif comprised in the S/MAR sequence comprises a multitude of the four-nucleotide motif 5'-ATTA-3'. Preferably, the S/MAR sequence has a length of at least 200 nucleotide, more preferably at least 300 nucleotides, even more preferably at least 400 nucleotides, most preferably at least 500 nucleotides. Preferably, the S/MAR sequence has a length of at most 3 kb, more preferably at most 2 kb, even more preferably at most 1.5 kb, still more preferably at most 1 kb, most preferably at most 0.9 kb. Thus, preferably, the S/MAR sequence has a length of from 0.2 kb to 3 kb, more preferably of from 0.3 kb to 2 kb, even more preferably of from 0.4 kb to 1.5 kb, most preferably of from 0.5 kb to 1 kb. As will be understood, the indication "comprises n sequence motifs per 100 nucleotides" relates to the average number of said sequence motifs calculated per 100 base pairs of sequence and, accordingly, may be a fraction number. E.g. the number of ATTA sequence motifs per 100 base pairs in SEQ ID NO:6 is 34 / 525 base pairs * 100 base pairs = 6.5. Preferably, the number of sequence motifs per 100 base pairs is determined over the whole length of the S/MAR sequence; in case of doubt, e.g. where a boundary of the S/MAR sequence cannot be determined, the number of sequence motifs per 100 base pairs of a polynucleotide, preferably, is the highest number determinable for any window of 200 bp within said polynucleotide, more preferably is the highest number determinable for any window of 500 bp within said polynucleotide. Preferably, the S/MAR sequence comprises at least 4 sequence motifs ATTA per 100 nucleotides over a stretch of at most 200 nucleotides, more preferably at least 5 sequence motifs ATTA per 100 nucleotides over a stretch of at most 200 nucleotides, still more preferably at least 6 sequence motifs ATTA per 100 nucleotides over a stretch of at most 200 nucleotides. Also preferably, the S/MAR sequence comprises at least 3 sequence motifs ATTA per 100 nucleotides over a stretch of at most 400 nucleotides, more preferably at least 4 sequence motifs ATTA per 100 nucleotides over a stretch of at most 400 nucleotides, even more preferably at least 5 sequence motifs ATTA per 100 nucleotides over a stretch of at most 400 nucleotides, most preferably at least 6 sequence motifs ATTA per 100 nucleotides over a stretch of at most 400 nucleotides. Also preferably, the S/MAR sequence comprises at least 3 sequence motifs ATTA per 100 nucleotides over a stretch of at most 500 nucleotides, more preferably at least 4 sequence motifs ATTA per 100 nucleotides over a stretch of at most 500 nucleotides, still more preferably at least 5 sequence motifs ATTA per 100 nucleotides over a stretch of at most 500 nucleotides, most preferably at least 6 sequence motifs ATTA per 100 nucleotides over a stretch of at most 500 nucleotides. Thus, preferably, the S/MAR sequence comprises at least 10 sequence motifs ATTA over a sequence of 500 nucleotides, more preferably at least 20 sequence motifs ATTA over a sequence of 500 nucleotides, still more preferably at least 30 sequence motifs ATTA over a sequence of 500 nucleotides. Preferably, at least 80%, more preferably at least 90%, most preferably at least 95% of the ATTA motifs in the S/MAR sequence are separated by of from 9 to 13, preferably by 10 to 12, most preferably by 11 base pairs, respectively.

Preferably, the S/MAR element comprises additional sequence motifs, preferably within the sequence comprising the ATTA motifs described herein above. Preferably, the sequence stretch of said S/MAR element comprising said ATTA sequence motifs further comprises at least one sequence motif ATTTA, preferably at least 2 sequence motifs ATTTA, more preferably at least 4 sequence motifs ATTTA, most preferably at least 8 sequence motifs ATTTA. Also preferably, the sequence stretch of said S/MAR element comprising said ATTA sequence motifs and, optionally, said ATTTA motif(s), further comprises at least one, preferably at least two, more preferably at least four, most preferably at least six palindromic motifs, preferably motifs TAAATATTTTA (SEQ ID NO:30). Preferably, said motifs TAAATATTTTA are contiguous with at least one motif ATTA on the 5' end and/or the 3' end. Also preferably, the sequence stretch of the S/MAR element comprising said ATTA sequence motifs comprises at least one, preferably at least two, more preferably at least three, even more preferably at least four, most preferably at least five sequence motifs ATTATAAATATTTTAATTA (SEQ ID NO:31), more preferably sequence motifs ATTTAATTATAAATATTTTAATTA (SEQ ID NO:32).

Also preferably, the S/MAR sequence has a low G+C content. The skilled person knows how to calculate the C+G content of a known sequence by counting all guanine and cytidine bases in the sequence and dividing the cumulated result by the number of nucleotides in the sequence. Preferably, the sequence stretch of the S/MAR element comprising said sequence motifs ATTA has a G+C content of at most 30%, more preferably at most 20%, still more preferably at most 15%, even more preferably at most 10%, most preferably at most 5%. Preferably, in cases where the boundary of an S/MAR element cannot be determined, the sequence used for calculation of the G+C content is the same used for calculating the number of ATTA motifs per 100 base pairs, as specified herein above. Also preferably, the S/MAR sequence has a low number of CG dinucleotides. Preferably, the sequence stretch of said S/MAR element comprising said sequence motifs comprises at most 6 sequence motifs CG, more preferably at most 4, even more preferably at most 2, most preferably does not comprise a sequence motif CG.

Preferably, the S/MAR sequence comprises an S/MAR sequence of an Apolipoprotein B gene, preferably a human Apolipoprotein B gene, more preferably a 3' S/MAR sequence of a human Apolipoprotein B gene. More preferably, the S/MAR sequence comprises a variant of a human Apolipoprotein B gene, more preferably of a 3' S/MAR sequence of a human Apolipoprotein B gene. Thus, preferably, the S/MAR sequence comprises a sequence at least 70% identical to the sequence of SEQ ID NO:33, preferably of SEQ ID NO:34 or 35. More preferably, the S/MAR sequence comprises the nucleic acid sequence of SEQ ID NO:33, preferably of SEQ ID NO:34, more preferably SEQ ID NO:35.

Preferably, the polynucleotide comprises a poly-A signal downstream of the S/MAR element: More preferably, the polynucleotide comprises a poly-A signal and a transcription termination signal downstream of the S/MAR element. Also preferably, the S/MAR element is flanked by a splice donor and a splice acceptor; thus, preferably, the S/MAR sequence preferably is spliced out of the transcript encoding the selectable marker after transcription. Also preferably, the polynucleotide further comprises a (secondary) bacterial origin of replication as specified herein above and/or a bacterial selectable marker gene. Preferably, the bacterial origin of replication and the promoter driving expression of the bacterial selectable marker gene are prokaryote-specific, i.e., more preferably, are non-functional in a host cell. Also preferably, the bacterial origin of replication and/or bacterial selectable marker gene, preferably all elements active in a prokaryotic cell comprised in the polynucleotide, is/are insulated from the residual sequences comprised in the polynucleotide by the presence of at least one insulation element, more preferably by being flanked by insulation elements. preferably, the bacterial origin of replication and/or bacterial selectable marker gene, preferably all elements active in a prokaryotic cell, is/are insulated from the residual sequences comprised in the polynucleotide by the presence of at least one insulating element at the 5' end and of at least one insulating element at the 3' end. More preferably, the bacterial origin of replication and/or bacterial selectable marker gene, preferably all elements active in a prokaryotic cell comprised in the polynucleotide, is/are insulated from the promoter by the presence of at least one insulation element, more preferably by being flanked by insulation elements. Preferably, said insulation element(s) is(are) an anti-repressive element40 element (SEQ ID NO:38) or a variant thereof and/or an S/MAR element.

Thus, preferably, the polynucleotide comprises the sequence of SEQ ID NO:34 or 35 or of a sequence at least 70% identical to the sequence of SEQ ID NO:34 or 35; preferably of SEQ ID NO:39 or of a sequence at least 70% identical to the sequence of SEQ ID NO:39, more preferably of SEQ ID NO:40 or of a sequence at least 70% identical to the sequence of SEQ ID NO:40, most preferably of SEQ ID NO:40 or of a sequence at least 70% identical to the sequence of SEQ ID NO:41. Preferably, the polynucleotide comprises the sequence of SEQ ID NO:41 with the nucleic acid sequence encoding GFP replaced by a nucleic acid sequence encoding a different polypeptide, preferably a therapeutic polypeptide, more preferably human T Cell Receptor (TCR), Chimeric Antigen Receptor (CAR), preferably MART1 TCR.

Preferably, the polynucleotide further comprises a coding sequence encoding a selectable marker polypeptide, said selectable marker sequence intervening the promoter of the polynucleotide and the S/MAR element of the polynucleotide, preferably wherein said promoter and said selectable marker sequence together constitute a selectable marker gene. As used herein, the term "selectable marker sequence" is used as a shorthand for the expression "coding sequence encoding a selectable marker polypeptide". The term "selectable marker" is in principle understood by the skilled person and relates to a nucleic acid sequence conferring, when expressed in a host cell, resistance to at least one condition mediating selective pressure to a host cell when applied thereto. Selectable markers are known in the art for prokaryotic and for eukaryotic cells. Preferably, the selectable marker is a selectable marker of an eukaryotic cell. Preferably, the selectable marker is a selectable marker polypeptide, more preferably a selectable marker polypeptide having transporter and/or enzymatic activity removing a selective compound from a hot cell or modifying said selective compound to make it inactive. Preferably, the selectable marker gene further encodes at least one intron, preferably upstream of the sequence encoding the selectable marker polypeptide. Preferably, the selectable marker is a marker mediating resistance to puromycin, to blasticidin, neomycin, and/or to zeocin, more preferably to puromycin. Thus, preferably, the promoter and the selectable marker together constitute a puromycin resistance gene, a blasticidin resistance gene, a neomycin resistance gene, or a zeocin resistance gene, more preferably a puromycin resistance gene. Preferably, the selectable marker gene is devoid of a poly-A signal and of transcription termination signal(s).

Preferably the selectable marker is the puromycin acetyltransferase (Genbank Acc No. KX548903.1 (SEQ ID NO:36), encoded by nucleotides 535 to 1134 of Genbank Acc No. KX548903.1 (SEQ ID NO:37)). Thus, the selectable marker gene, preferably, comprises a nucleic acid sequence which a) causes expression of a puromycin resistance polypeptide comprising the sequence of SEQ ID NO:36; b) causes expression of a puromycin resistance polypeptide comprising a sequence at least 70% identical to the sequence of SEQ ID NO:36; c) comprises the sequence of SEQ ID NO:37; d) comprises a sequence at least 70% identical to the sequence of SEQ ID NO:37, e) comprises a nucleic acid sequence encoding a puromycin resistance polypeptide comprising, preferably consisting of, the sequence of SEQ ID NO:36, and/or f) comprises a nucleic acid sequence encoding a puromycin resistance polypeptide comprising, preferably consisting of, a sequence at least 70% identical to the sequence of SEQ ID NO:36.

As used herein, the term "replicating" relates to the activity of the polynucleotide to induce production of at least two replicas of said polynucleotide in a host cell during a cell replication cycle. Thus, preferably, replication of a polynucleotide in a host cell is determined by determining the presence of the polynucleotide after a series of cell divisions, in which a non-replicating polynucleotide would have been expected to be diluted out. Preferably, replication is stable replication, i.e. is replication to such an extent that the polynucleotide still is detectable in a host cell population after on average 50 cell divisions, more preferably after on average 100 cell divisions, most preferably after on average 250 cell divisions. Preferably, detection of a polynucleotide in a host cell population is performed by PCR under standard conditions.

The term "episomal" replication is, in principle, known to the skilled person to relate to replication of a polynucleotide without being integrated into the cellular genome, i.e. without becoming covalently attached to the cellular genome. Thus, preferably, episomal replication of a polynucleotide is replication of said polynucleotide as an autonomous replication unit. Preferably, episomal replication is maintenance of the polynucleotide in the host cell in the form of a circularly closed double-stranded DNA molecule. As will be understood by the skilled person, the actual replication of said polynucleotide may involve other forms, e.g. in rolling circle replication. Episomal maintenance of circular DNA preferably is verified by the plasmid rescue procedure known to the skilled person; i.e. preferably, by preparing a lysate of host cells and transforming the DNA comprised therein into appropriate bacterial cells, e.g. E. coli cells; if a suitable number of bacterial colonies obtainable by said method comprises the circular DNA as a plasmid having the same restriction pattern and/or sequence as the original circular DNA, it is, preferably, assumed that the circular DNA was maintained episomally. A further method of verifying episomal maintenance, which is also known to the skilled person, is DNA/DNA blotting ("Southern Blot" method); thus, preferably, total DNA of host cells is prepared and digested with one or more restriction enzyme(s); if in a Southern Blot using the original plasmid as a probe only bands corresponding to the original circular DNA are visible, it is preferably concluded that the plasmid is maintained episomally. More preferably, episomal maintenance is verified as described herein in the Examples.

In accordance, the term "replicating episomally", as used herein, relates to the activity of a polynucleotide to induce production of at least two replicas of said polynucleotide in a host cell during a cell replication cycle while said polynucleotide is present in said cell as an autonomously replicating entity; and stable episomal replication is episomal replication to such an extent that the polynucleotide is still detectable in the host cell after at least 50 cell divisions, preferably after at least 100 cell divisions, more preferably, after at least 250 cell divisions, most preferably, after at least 500 cell divisions. Preferably, the aforesaid number of cell divisions is the average number of cell divisions for a population of cells.

The polynucleotide of the present invention preferably is devoid of a of a simian virus 40 (SV40) origin of replication, a bovine papillomavirus (BPV) origin of replication, and an Epstein-Barr virus (EBV) origin of replication, preferably is devoid of a polyomavirus origin of replication, a papillomavirus origin of replication, and a herpesvirus origin of replication; more preferably is devoid of an origin of replication of an eukaryote-infecting virus. More preferably, the vector is devoid of any known eukaryotic origin of replication. However, preferably, the polynucleotide further comprises a prokaryotic, preferably a bacterial origin of replication, in particular an E. coli origin of replication. Preferably, the prokaryotic origin of replication is the only origin of replication comprised in the polynucleotide.

Advantageously, it was found in the work underlying the present invention that by combining an S/MAR element as specified with a promoter reading into said S/MAR element, a polynucleotide is obtained which is highly stable in episomal form in host cells, even in the absence of a dedicated origin of replication. Moreover, it was found that efficacy of establishment of the polynucleotide could be further improved by using a puromycin resistance gene, by ensuring transcription into the S/MAR element through the resistance gene, and by insulating the promoter - S/MAR combination transcriptionally from other promoters potentially present in the polynucleotide.

The definitions made above apply mutatis mutandis to the following. Additional definitions and explanations made further below also apply for all embodiments described in this specification mutatis mutandis.

The present invention further relates to a composition comprising a polynucleotide according to the present invention.

The term "composition", as used herein, as used herein, relates to a composition of matter comprising the compounds as specified and optionally one or more acceptable carrier. Preferably, the composition is a pharmaceutically acceptable composition; thus, preferably, the carrier is a pharmaceutically acceptable carrier. The compounds of the present invention can be formulated as, preferably pharmaceutically acceptable, salts. Preferred salts comprise acetate, methylester, HCl, sulfate, chloride and the like.

The carrier(s) must be acceptable in the sense of being compatible with the other ingredients of the formulation and being not deleterious to the recipient thereof. A carrier employed may be, for example, either a solid, a gel or a liquid. Exemplary of solid pharmaceutical carriers are lactose, terra alba, sucrose, talc, gelatin, agar, pectin, acacia, magnesium stearate, stearic acid and the like. Exemplary of liquid carriers are phosphate-buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania. The diluent(s) is/are selected so as not to affect the biological activity of the compounds in the composition. Examples of such diluents are distilled water, physiological saline, Ringer's solutions, dextrose solution, and Hank's solution. In addition, the pharmaceutical composition or formulation may also include other carriers, adjuvants, or nontoxic, nontherapeutic, nonimmunogenic stabilizers and the like.

Preferably, the composition mediates entry of the polynucleotide into a host cell. Thus, preferably the composition comprises at least one transfection agent. The selection of an appropriate transfection agent may depend on the target host cell, as well as the specific application envisaged. Transfection agents, appropriate transfection conditions, as well as selection criteria therefor are well-known in the art. Also preferably, the composition comprises virus-like particles. Thus, preferably, the polynucleotide is packaged into virus-like particles, i.e. preferably, the polynucleotide is comprised in the virus-like particles.

Pharmaceutical compositions are, preferably, administered topically or systemically. Suitable routes of administration conventionally used for drug administration are oral, intravenous, or parenteral administration as well as inhalation. However, depending on the nature and mode of action of a compound, the pharmaceutical compositions may be administered by other routes as well. For example, polynucleotide compounds may be administered in a gene therapy approach by using viral vectors or viruses or liposomes, as specified herein above. Moreover, the compounds can be administered in combination with other drugs either in a common pharmaceutical composition or as separated pharmaceutical compositions wherein said separated pharmaceutical compositions may be provided in form of a kit of parts. The compounds are, preferably, administered in conventional dosage forms prepared by combining the drugs with standard pharmaceutical carriers according to conventional procedures. These procedures may involve mixing, granulating and compressing or dissolving the ingredients as appropriate to the desired preparation. It will be appreciated that the form and character of the pharmaceutically acceptable carrier or diluent is dictated by the amount of active ingredient with which it is to be combined, the route of administration and other well-known variables.

A therapeutically effective dose of a pharmaceutical composition refers to an amount of the compounds to be used in a pharmaceutical composition of the present invention which prevents, ameliorates or treats the symptoms accompanying a disease or condition referred to in this specification. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50.

The dosage regimen will be determined by the attending physician and other clinical factors; preferably in accordance with any one of the above described methods. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment. A typical dose can be, for example, in the range of 1 to 1000 µg; however, doses below or above this exemplary range are envisioned, especially considering the aforementioned factors. Generally, the regimen as a regular administration of the pharmaceutical composition should be in the range of 1 µg to 10 mg units per day. If the regimen is a continuous infusion, it should also be in the range of 1 µg to 10 mg units per kilogram of body weight per minute, respectively. Progress can be monitored by periodic assessment. However, depending on the subject and the mode of administration, the quantity of substance administration may vary over a wide range to provide from about 0.01 mg per kg body mass to about 10 mg per kg body mass. In case a viral vector, in particular adeno-associated viral vector is administered, preferred doses are from 5 x 1011, to 2 x 1013 viral particles or viral genomes / kg body weight; as will be understood, these exemplary doses may be modified depending, in addition to the factors described above, on additional factors like type of virus, target organ, and the like.

The pharmaceutical compositions and formulations referred to herein are administered at least once in order to treat or ameliorate or prevent a disease or condition recited in this specification. However, the said pharmaceutical compositions may be administered more than one time, for example from one to four times daily up to a non-limited number of days.

Specific pharmaceutical compositions are prepared in a manner well known in the pharmaceutical art and comprise at least one active compound referred to herein above in admixture or otherwise associated with a pharmaceutically acceptable carrier or diluent. For making those specific pharmaceutical compositions, the active compound(s) will usually be mixed with a carrier or the diluent, or enclosed or encapsulated in a capsule, sachet, cachet, paper or other suitable containers or vehicles. The resulting formulations are to be adopted to the mode of administration, i.e. in the forms of tablets, capsules, suppositories, solutions, suspensions or the like. Dosage recommendations shall be indicated in the prescribers or users instructions in order to anticipate dose adjustments depending on the considered recipient.

The present invention also relates to a polynucleotide according to the present invention, a composition according to the present invention, and/or a host cell according to the present invention, for use in medicine. The present invention further relates to a polynucleotide according to the present invention, a composition according to the present invention, and/or a host cell according to the present invention, for use in treating genetic disease.

The term "genetic disease", as used herein, relates to a disease causally linked to one or more modifications, preferably mutations, in the genome of an individual. Thus, preferably, the genetic disease is causally linked to one or more epigenetic changes, more preferably is causally linked to one or more genetic mutations. As will be understood, symptoms of a genetic disease often are caused by expression of a mutated gene and/or lack of expression of a gene providing normal function of the gene product in one or more specific tissue(s) and/or cell type(s). Thus, it may be preferable to treat genetic disease only in those cells in which the mutation contributes to disease. Preferably, the genetic disease is a monogenic disease, i.e. is caused by a genetic alteration in one gene. More preferably, the genetic disease is a monogenic recessive disease, i.e. is caused by genetic alterations in both alleles of a gene; thus, preferably, the amelioration of symptoms is expected by provision of at least one unaltered copy of the affected gene. Most preferably, the genetic disease is phenylketonuria, alkaptonuria, Leber's Congenital Amaurosis, Choroideremia, or Stargardt disease.

The present invention also relates to a kit comprising a polynucleotide according to the present invention and a compound mediating cell entry.

The term "kit", as used herein, refers to a collection of the aforementioned compounds, means or reagents of the present invention which may or may not be packaged together. The components of the kit may be comprised by separate vials (i.e. as a kit of separate parts) or provided in a single vial. Moreover, it is to be understood that the kit of the present invention, preferably, is to be used for practicing the methods referred to herein above. It is, preferably, envisaged that all components are provided in a ready-to-use manner for practicing the methods referred to above. Further, the kit, preferably, contains instructions for carrying out said methods. The instructions can be provided by a user's manual in paper or electronic form. In addition, the manual may comprise instructions for interpreting the results obtained when carrying out the aforementioned methods using the kit of the present invention. As will be understood from the above, the description of the kit comprising polynucleotides, preferably, relates to a kit comprising corresponding vectors mutatis mutandis.

Preferably, the kit further comprises at least one compound mediating cell entry for the polynucleotide it comprises, the term "compound mediating cell entry" relating to any means suitable to cause a polynucleotide of the kit to enter the interior of a host cell, preferably a host cell. Suitable compound mediating cell entry (delivery means) are known in the art and include in particular transfection means, packaging compositions, and the like. Preferably, the polynucleotide of the present invention is pre-packaged in a delivery means, e.g. in viral particles, more preferably in replication-defective viral particles, most preferably in virus-like particles (VLPs). The skilled person is aware of delivery means providing different specificities for cellular receptors, such that delivery means appropriate for a given target host cell may be selected.

The present invention further relates to a device comprising a polynucleotide according to the present invention, a composition according to the present invention, and/or a host cell according to the present invention.

The term "device", as used herein relates to a system of means comprising at least the means operatively linked to each other as to allow administration of the compound or of the composition of the present invention. Preferred means for administering polynucleotides, compositions, host cells are well known in the art. How to link the means in an operating manner will depend on the type of means included into the device and on the kind of administration envisaged. Preferably, the means are comprised by a single device in such a case. Said device may accordingly include a delivery unit for the administration of the compound or composition and a storage unit for storing said compound or composition until administration. However, it is also contemplated that the means of the current invention may appear as separate devices in such an embodiment and are, preferably, packaged together as a kit. The person skilled in the art will realize how to link the means without further ado. Preferred devices are those which can be applied without the particular knowledge of a specialized technician. In a preferred embodiment, the device is a syringe, more preferably with a needle, comprising the compound or composition of the invention. In another preferred embodiment, the device is an intravenous infusion (IV) equipment comprising the compound or composition. In another preferred embodiment, the device is an endoscopic device comprising the compound or medicament for flushing a site of administration, or further comprising a needle for topical application of the compound or composition, e.g. to a tumor. In still another preferred embodiment the device is an inhaler comprising the compound of the present invention, wherein, more preferably, said compound is formulated for administration as an aerosol.

The present application also relates to a method for stably transfecting a host cell, comprising
a) contacting said host cell with a polynucleotide according to the present invention, a composition according to the present invention, and/or a host cell according to the present invention, and,
b) thereby, stably transfecting a host cell.

The method for stably transfecting a host cell of the present invention, preferably, is an in vitro method. Moreover, it may comprise steps in addition to those explicitly mentioned above. For example, further steps may relate, e.g., to providing a host cell or a sample comprising the same for step a), and/or applying selective pressure to the host cells contacted. Moreover, one or more of said steps may be performed by automated equipment.

The term stably transfecting a host cell is understood by the skilled person to relate to introducing a polynucleotide, preferably a heterologous polynucleotide into a cell such that the polynucleotide is stable replicated by the host cell as specified herein above. Preferably, stable transfection comprises stable episomal replication of the polynucleotide. Preferably, stable transfecting comprises, after contacting, applying selective pressure to the host cell to select for the presence of a selectable marker. The selective pressure is applied after contacting, optionally excluding a first time frame allowing the polynucleotide to establish within the host cell; the duration of said first time frame allowing the polynucleotide to establish within the host cell will depend mostly on the type of host cell contacted and on the kind of selectable marker used; preferably, the duration of said first time frame allowing the polynucleotide to establish within the host cell is of from 1 h to 48 h, more preferably of from 2 h to 24, most preferably of from 3 h to 16 h. However, the duration of said first time frame allowing the polynucleotide to establish within the host cell may also be zero, i.e. selective pressure may be applied immediately after contacting or even during contacting. Selective pressure may be applied continuously, i.e. at essentially all time points after the first time frame allowing the polynucleotide to establish within the host cell, more preferably to prevent host cells not comprising the polynucleotide from proliferating; or it may be applied transiently, more preferably to remove cells not having received the polynucleotide. Preferably, transient application of selective pressure is used in cases where cells are transferred back into an organism after said contacting. It is, however, also envisaged that no selective pressure is applied, in particular in cases where it is known that the efficiency of transfer of the polynucleotide into target host cells is sufficiently high and/or where a pure population of transgenic host cells is not of major importance.

The term "contacting", as used in the context of the methods of the present invention, is understood by the skilled person. Preferably, the term relates to bringing at least one polynucleotide, vector, and/or host cell of the present invention in physical contact with a host cell, e.g. allowing the host cell and the compound(s) to interact. Preferably, contacting includes delivery of at least one polynucleotide of the present invention into the interior of a host cell, preferably via a delivery means as specified above.

The present invention also relates to a method for treating genetic disease in a subject, comprising
a) contacting said subject with a polynucleotide according to the present invention, a composition according to the present invention, and/or a host cell according to the present invention, and,
b) thereby, treating genetic disease in said subject.

The method for treating genetic disease of the present invention, preferably, is an in vivo method. Moreover, it may comprise steps in addition to those explicitly mentioned above. For example, further steps may relate, e.g., to providing a host cell or a sample comprising the same for step a), and/or re-administering said sample or host cell into the subject. Thus, the method for treating genetic disease, comprise the steps of the method for stably transfecting a host cell as specified above. Moreover, one or more of said steps may be performed by automated equipment.

Further, the present invention relates to a use of a polynucleotide of the present invention for stably genetically modifying a host cell.

Also, the present invention relates to a use of a polynucleotide according to the present invention, a composition according to the present invention, and/or a host cell according to the present invention, for the manufacture of a medicament. And to a use of a polynucleotide according to the present invention, a composition according to the present invention, and/or a host cell according to the present invention, for the manufacture of a medicament for treating genetic disease, preferably monogenic disease, more preferably monogenic recessive disease, most preferably phenylketonuria, alkaptonuria, Leber's Congenital Amaurosis, Choroideremia, or Stargardt disease.

Also, the present invention relates to a use of a polynucleotide according to the present invention, a composition according to the present invention, and/or a host cell according to the present invention, for the genetic modification of a primary cell, preferably a primary dermal fibroblast, for the generation of an Induced Pluripotent Stem Cells (IPSCs). Preferably, said primary cell is a mouse or a human primary cell.

The term "primary cell" is understood by the skilled person as opposed to a cell of a cultured cell line; thus, preferably, a primary cell is a cell derived from a living organism and having been cultured for at most 20 passages, more preferably at most 15 passages, even more preferably at most 10 passages, still more preferably at most 5 passages. Most preferably, primary cells are cells derived directly from tissue of a living being, preferably a mouse or a human.

The term "stem cell" is also understood by the skilled person to relate to an un- or low-differentiated cell with the potential for differentiation into at least two cell types, preferably at least five cell types, more preferably at least one complete cell lineage. Preferably, the stem cell is a totipotent stem cell, more preferably a pluripotent stem cell. The term "Induced Pluripotent Stem Cell" or "IPSC" relates to a pluripotent stem cell derived from a differentiated cell, preferably a differentiated primary cell. Methods of generating IPSCs are known in the art and include, preferably, expression of four transcription factors in the cell (e.g from Takahashi et al. (2006), Cell. 126 (4):663).

The present invention also relates to a use of a polynucleotide according to the present invention, a composition according to the present invention, and/or a host cell according to the present invention, for the genetic modification of embryonic stem cells. The present invention also relates to a use of a polynucleotide according to the present invention, a composition according to the present invention, and/or a host cell according to the present invention, for the manufacture of a medicament for treating genetic disease, preferably monogenic disease, more preferably monogenic recessive disease, most preferably phenylketonuria, alkaptonuria, Leber's Congenital Amaurosis, Choroideremia, or Stargardt disease, wherein said medicament comprises host cells comprising a polynucleotide of the present invention.

The present invention also relates to a use of a polynucleotide according to the present invention, a composition according to the present invention, and/or a host cell according to the present invention, for the genetic modification of stem cells for generating a transgenic animal. The present invention further relates to a use of a polynucleotide according to the present invention, a composition according to the present invention, and/or a host cell according to the present invention, for the production of a transgenic animal.

The term "transgenic animal" as used herein, relates to an animal comprising at least one heterologous polynucleotide, preferably introduced into said animal by methods of genetic engineering. Preferably, the transgenic animal comprises at least one, more preferably at least 10, still more preferably at least 1000, even more preferably at least 10000 cells comprising at least one polynucleotide according to the present invention.

Also, the present invention relates to a use of a polynucleotide according to the present invention, a composition according to the present invention, and/or a host cell according to the present invention, for the genetic modification of single cell embryos by pronuclear injection.

As is understood by the skilled person, the term "pronuclear injection" relates to injecting genetic material, preferably a polynucleotide of the present invention, into the nucleus of a fertilized oocyte, preferably to create a transgenic animal.

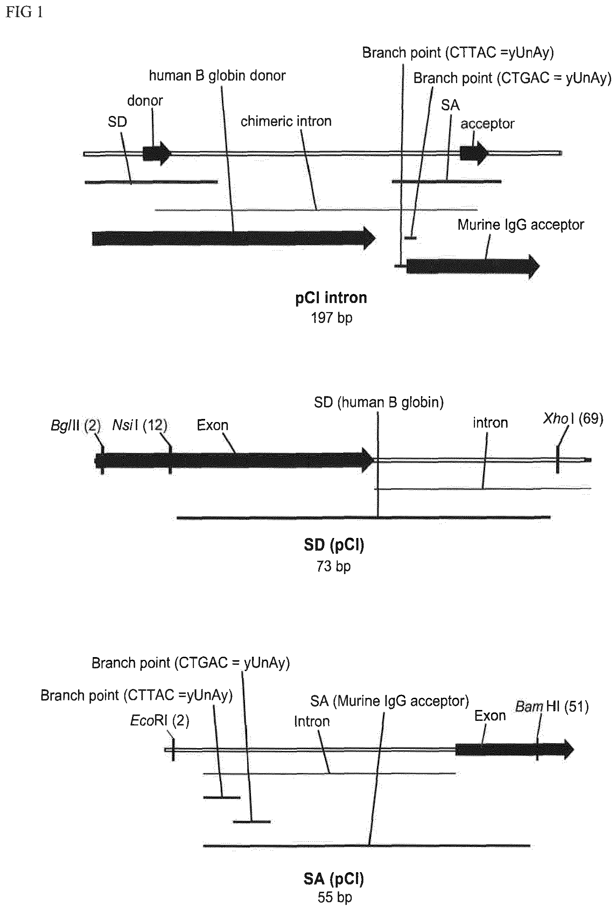

Further definitions:
AF: Antibiotic-free.
amp: Ampicillin.
ampR: Ampicillin Resistance gene.
Antibiotic selectable marker: A gene that confers resistance to an antibiotic, e.g. ampicillin resistance gene, kanamycin resistance gene, chloramphenicol resistance gene, puromycin resistance gene, tetracycline resistance gene.
ApoB: Apolipoprotein B
Approximately: As used herein, the term "approximately" or "about," as applied to one or more values of interest, refers to a value that is the same or similar to a stated reference value.
Bacterial region: Region of a plasmid vector required for propagation and selection in the bacterial host.
bp: basepairs
ccc: Covalently Closed Circular.
cI: Lambda repressor.
cITs857: Lambda repressor further incorporating a C to T (Ala to Thr) mutation that confers temperature sensitivity. cITs857 is a functional repressor at 28-30°C but is mostly inactive at 37-42°C. Also called cI857.
CatR: Chloramphenicol resistance gene.
cmv: Cytomegalovirus.
E. coli: Escherichia coli, a gram negative bacteria.
EGFP: Enhanced green fluorescent protein.
ELE40: anti-repressor element Element 40, STAR40 disclosed in Kwaks et al., 2003, Nat Biotechnol. 21:553
EP: Electroporation.
Establishment efficiency: The percentage of cells in which a self-replicating non-integrative episomal S/MAR expression vector is stably retained as an episome after transfection.
Eukaryotic expression vector: A vector for expression of mRNA, protein antigens, protein therapeutics, shRNA, RNA or microRNA genes in a target eukaryotic cell or organism using RNA Polymerase I, II or III promoters.
Eukaryotic region: The region of a plasmid that encodes eukaryotic sequences and/or sequences required for plasmid function in the target organism. This includes the region of a plasmid vector required for expression of one or more transgenes in the target organism including RNA Pol II enhancers, promoters, transgenes and polyA sequences. This also includes the region of a plasmid vector required for expression of one or more transgenes in the target organism using RNA Pol I or RNA Pol III promoters, RNA Pol I or RNA Pol III expressed transgenes or RNAs. The eukaryotic region may optionally include other functional sequences, such as eukaryotic transcriptional terminators, supercoiling-induced DNA duplex destabilized (SIDD) structures, S/MARs, boundary elements, etc.
Exon: A nucleotide sequence encoded by a gene that is transcribed and present within a mature mRNA product after RNA splicing to remove introns has been completed. Expression enhancer: A DNA sequence that improves the expression of an adjacent promoter. For example, Ele40, UCOE, anti-repressor elements, or Stabilising Anti Repressor (STAR) elements as reviewed in Saunders et al., 2015 PloS One 10:e0120096
Expression vector: A vector for expression of mRNA, protein antigens, protein therapeutics, shRNA, RNA or microRNA genes in a target organism.
g: Gram, kg for kilogram
gene of interest: gene to be expressed in the target organism. Includes mRNA genes that encode protein or peptide antigens, protein or peptide therapeutics, and mRNA, shRNA, RNA or microRNA that encode RNA therapeutics, and mRNA, shRNA, RNA or microRNA that encode RNA vaccines, etc.
GFP: Green fluorescent protein.
Hr(s): Hour(s).
ID: Intradermal.
IM:Intramuscular.
immune response: Antigen reactive cellular (e.g. antigen reactive T cells) or antibody (e.g. antigen reactive IgG) responses.
Intron: A nucleotide sequence encoded by a gene that is transcribed and subsequently removed from a mature mRNA product by RNA splicing.
ITR: Inverted Terminal Repeat.
kan: Kanamycin.
kanR: Kanamycin Resistance gene.
Kd:Kilodalton.
kozak sequence: Optimized consensus DNA sequence gccRccATG (R = G or A) immediately upstream of an ATG start codon that ensures efficient tranlation initiation.
MFI: Medium Fluorescent Intensity.
minicircle: Covalently closed circular plasmid derivatives in which the bacterial region has been removed from the parent plasmid by in vivo or in vitro site-specific recombination or in vitro restriction digestion/ligation. Minicircle vectors are replication incompetent in bacterial cells.
mRNA: Messenger RNA.
mSEAP: Murine secreted alkaline phosphatase.
NA: Not Applicable.
NanoplasmidTM vector: Vector with a bacterial region combining an RNA selectable marker with a R6K, ColE2 or ColE2 related replication origin. For example, NTC9385C, NTC9685C, NTC9385R, NTC9685R vectors and modifications described in Williams, 2014. DNA plasmids with improved expression. World Patent Application WO2014035457 and included herein by reference.
Non-integrating lentiviral vector: A lentiviral vector with mutated integrase and a S/MAR for maintenance of episomal LTR circles such as those described in Verghese et al., 2014 Nucleic Acids Research 42:e53.
NP: Nanoplasmid.
NTC8385: NTC8385, NTC8485 and NTC8685 plasmids are antibiotic-free pUC origin vectors that contain a short RNA (RNA-OUT) selectable marker instead of an antibiotic resistance marker such as kanR. The creation and application of these RNA-OUT based antibiotic-free vectors are described in Williams, JA 2008 World Patent Application WO2008153733 and included herein by reference.
NTC8485: NTC8485 is an antibiotic-free pUC origin vector that contains a short RNA (RNA-OUT) selectable marker instead of an antibiotic resistance marker such as kanR. The creation and application of NTC8485 is described in Williams, JA 2010 US Patent Application 20100184158 and included herein by reference.
NTC8685: NTC8685 is an antibiotic-free pUC origin vector that contains a short RNA (RNA-OUT) selectable marker instead of an antibiotic resistance marker such as kanR. The creation and application of NTC8685 is described in Williams, Supra, 2010 and included herein by reference.
NTC9385R: The NTC9385R NanoplasmidTM vector described in Williams, Supra, 2014 included herein by reference has a spacer region encoded NheI- trpA terminator-R6K origin RNA-OUT -KpnI bacterial region (SEQ ID NO:8) linked through the flanking NheI and KpnI sites to the eukaryotic region.
OD600: optical density at 600 nm.
PBS: Phosphate buffered Saline.
PCR: Polymerase Chain Reaction.
pDNA: Plasmid DNA.
pINT pR pL vector: The pINT pR pL attHK022 integration expression vector is described in Luke et al., 2011 Mol Biotechnol 47:43 and included herein by reference. The target gene to be expressed is cloned downstream of the pL promoter. The vector encodes the temperature inducible cI857 repressor, allowing heat inducible target gene expression.
PL promoter: Lambda promoter left. PL is a strong promoter that is repressed by the cI repressor binding to OL1, OL2 and OL3 repressor binding sites. The temperature sensitive cI857 repressor allows control of gene expression by heat induction since at 30°C the cI857 repressor is functional and it represses gene expression, but at 37-42 °C the repressor is inactivated so expression of the gene ensues.
PL (OL1 G to T) promoter: Lambda promoter left. PL is a strong promoter that is repressed by the cI repressor binding to OL1, OL2 and OL3 repressor binding sites. The temperature sensitive cI857 repressor allows control of gene expression by heat induction since at 30°C the cI857 repressor is functional and it represses gene expression, but at 37-42 °C the repressor is inactivated so expression of the gene ensues. The cI repressor binding to OL1 is reduced by the OL1 G to T mutation resulting in increased promoter activity at 30°C and 37-42 °C as described in Williams, Supra, 2014.
Plasmid: An extra chromosomal DNA molecule separate from the chromosomal DNA which is capable of replicating independently from the chromosomal DNA
Plasmid copy number: the number of copies of a plasmid per cell. Increases in plasmid copy number increase plasmid production yield.
Pol:Polymerase.
polyA: Polyadenylation signal or site. Polyadenylation is the addition of a poly(A) tail to an RNA molecule. The polyadenylation signal contains the sequence motif recognized by the RNA cleavage complex. Most human polyadenylation signals contain an AAUAAA motif and conserved sequences 5' and 3' to it. Commonly utilized polyA signals are derived from the rabbit β globin (RBG), bovine growth hormone (BGH), SV40 early, or SV40 late polyA signals.
pUC origin: pBR322-derived replication origin, with G to A transition that increases copy number at elevated temperature and deletion of the ROP negative regulator.
pUC free: Plasmid that does not contain the pUC origin. Non-replicative fragments of the pUC origin may be included, for example the RNAI selectable marker.
pUC plasmid: Plasmid containing the pUC origin.
PuroR: Puromycin Resistance gene.
R6K plasmid: NTC9385R, NTC9685R, NTC9385R2-O1, NTC9385R2-O2, NTC9385R2a-01, NTC9385R2a-O2, NTC9385R2b-O1, NTC9385R2b-O2, NTC9385Ra-O1, NTC9385Ra-O2, NTC9385RaF, and NTC9385RbF vectors as well as modifications and alternative vectors containing a R6K replication origin that were described in Williams, Supra, 2014 and included herein by reference. Alternative R6K vectors known in the art including, but not limited to, pCOR vectors (Gencell), pCpGfree vectors (Invivogen), and CpG free University of Oxford vectors including pGM169.
R6K replication origin: a region which is specifically recognized by the R6K Rep protein to initiate DNA replication. Includes but not limited to R6K gamma replication origin sequence disclosed as SEQ ID NO:1, SEQ ID NO:2 and SEQ ID NO:4, and CpG free versions (e.g. SEQ ID NO:3) as described in Drocourt et al., United States Patent 7244609 and incorporated herein by reference R6K replication origin-RNA-OUT bacterial region: Contains a R6K replication origin for propagation and the RNA-OUT selectable marker (e.g. SEQ ID NO:8; SEQ ID NO:9; SEQ ID NO:10; SEQ ID NO:11; SEQ ID NO:12; SEQ ID NO:13; SEQ ID NO:14; SEQ ID NO:15; SEQ ID NO:16; SEQ ID NO:17).
Rep: Replication
Replication intermediates: Linear DNA fragments resulting from premature termination of plasmid replication
Rep protein dependent plasmid: A plasmid in which replication is dependent on a replication (Rep) protein provided in Trans. For example, R6K replication origin, ColE2-P9 replication origin and ColE2 related replication origin plasmids in which the Rep protein is expressed from the host strain genome. Numerous additional Rep protein dependent plasmids are known in the art, many of which are summarized in del Solar et al., Supra, 1998 which is included herein by reference
Retroviral vector: Integrative viral vector that can infect dividing cells. Also call transfer plasmid. Plasmid encodes Retroviral LTR flanked expression unit. Transfer plasmid is transfected into production cells along with envelope and packaging plasmids required to make viral particles.
RNA-IN: Insertion sequence 10 (IS10) encoded RNA-IN, an RNA complementary and antisense to a portion of RNA RNA-OUT. When RNA-IN is cloned in the untranslated leader of a mRNA, annealing of RNA-IN to RNA-OUT reduces translation of the gene encoded downstream of RNA-IN.
RNA-IN regulated selectable marker: A chromosomally expressed RNA-IN regulated selectable marker. In the presence of plasmid borne RNA-OUT antisense repressor RNA (SEQ ID NO:6), expression of a protein encoded downstream of RNA-IN is repressed. An RNA-IN regulated selectable marker is configured such that RNA-IN regulates either 1) a protein that is lethal or toxic to said cell per se or by generating a toxic substance (e.g. SacB), or 2) a repressor protein that is lethal or toxic to said bacterial cell by repressing the transcription of a gene that is essential for growth of said cell (e.g. murA essential gene regulated by RNA-IN tetR repressor gene). For example, chromosomally expressed RNA-IN-SacB cell lines for RNA-OUT plasmid selection/propagation are described in Williams, Supra, 2008 and included herein by reference. Alternative selection markers described in the art may be substituted for SacB.
RNA-OUT: Insertion sequence 10 (IS10) encoded RNA-OUT, an antisense RNA that hybridizes to, and reduces translation of, the transposon gene expressed downstream of RNA-IN. The sequence of the RNA-OUT RNA (SEQ ID NO:6) and complementary RNA-IN SacB chromosomally expressed RNA-IN-SacB cell lines can be modified to incorporate alternative functional RNA-IN/RNA-OUT binding pairs such as those described in Mutalik et al., 2012 Nat Chem Biol 8:447, including, but not limited to, the RNA-OUT A08/RNA-IN S49 pair, the RNA-OUT A08/RNA-IN S08 pair, and CpG free modifications of RNA-OUT A08 that modify the CG in the RNA-OUT 5' TTCGC sequence to non-CpG sequence. An example of a CpG free RNA-OUT selection marker, in which the two CpG motifs in the RNA-OUT RNA (one of which is present in the RNA-IN complementary region) are removed, was described in Williams 2015. Replicative minicircle vectors with improved expression. US Patent Application US 2015/0275221 and included herein by reference. A multitude of alternative substitutions to remove the two CpG motifs (mutating each CpG to either CpA, CpC, CpT, ApG, GpG, or TpG) may be utilized to make a CpG free RNA-OUT
RNA-OUT Selectable marker: An RNA-OUT selectable marker DNA fragment including E. coli transcription promoter and terminator sequences flanking an RNA-OUT RNA. An RNA-OUT selectable marker, utilizing the RNA-OUT promoter and terminator sequences, that is flanked by DraIII and KpnI restriction enzyme sites, and designer chromosomally expressed RNA-IN-SacB cell lines for RNA-OUT plasmid propagation, are described in Williams, Supra, 2008 and included herein by reference. The RNA-OUT promoter and terminator sequences in SEQ ID NO: 5 that flank the RNA-OUT RNA (SEQ ID NO:6) may be replaced with heterologous promoter and terminator sequences. For example, the RNA-OUT promoter may be substituted with a CpG free promoter known in the art, for example the I-EC2K promoter or the P5/6 5/6 or P5/6 6/6 promoters described in Williams, Supra, 2008 and included herein by reference. A 2 CpG RNA-OUT selectable marker in which the two CpG motifs in the RNA-OUT promoter are removed is given as SEQ ID NO: 7. An example of a CpG free RNA-OUT transcription unit, in which the two CpG motifs in the RNA-OUT RNA (one of which is present in the RNA-IN complementary region) and the two CpG motifs in the RNA-OUT promoter are removed was described in Williams, Supra, 2015 and included herein by reference. Vectors incorporating CpG free RNA-OUT selectable marker may be selected for sucrose resistance using the RNA-IN-SacB cell lines for RNA-OUT plasmid propagation described in Williams, Supra, 2008. Alternatively, the RNA-IN sequence in these cell lines can be modified to incorporate the 1 bp change needed to perfectly match the CpG free RNA-OUT region complementary to RNA-IN.
RNA polymerase II promoter: Promoter that recruits RNA Polymerase II to synthesize mRNAs, most small nuclear RNAs and microRNAs. For example, constitutive promoters such as the human or murine CMV promoter, elongation factor 1 (EF1) promoter, the chicken β -actin promoter, the β - actin promoter from other species, the elongation factor-1 α (EF1 α) promoter, the phosphoglycerokinase (PGK) promoter, the Rous sarcoma virus (RSV) promoter, the human serum albumin (SA) promoter, the spleen focus-forming virus (SFFV) promoter, the α -1 antitrypsin (AAT) promoter, the thyroxine binding globulin (TBG) promoter, the cytochrome P450 2E1 (CYP2E1) promoter, etc. The vectors may also utilize combination promoters such as the chicken β -actin/CMV enhancer (CAG) promoter, the human or murine CMV-derived enhancer elements combined with the elongation factor 1α (EF1α) promoters, CpG free versions of the human or murine CMV-derived enhancer elements combined with the elongation factor 1α (EF1α) promoters, the albumin promoter combined with an α -fetoprotein MERII enhancer, etc., or the diversity of tissue specific or inducible promoters know in the art such as the muscle specific promoters muscle creatine kinase (MCK), and C5-12 or the liver-specific promoters ApoE-hAAT, apolipoprotein A-I (ApoAI), etc.
RNA polymerase III promoter: Promoter that recruits RNA Polymerase III to synthesize tRNAs, 5S ribosomal RNA, and other small RNAs. For example, Class I promoters such as the 5s rRNA promoter, Class II promoter such as tRNA promoters, Class III promoters such as the U6 small nuclear RNA promoter or the H1 nuclear RNase P promoter, etc.
RNA selectable marker: An RNA selectable marker is a plasmid borne expressed non-translated RNA that regulates a chromosomally expressed target gene to afford selection. This may be a plasmid borne nonsense suppressing tRNA that regulates a nonsense suppressible selectable chromosomal target as described by Crouzet J and Soubrier F 2005 US Patent 6,977,174 included herein by reference. This may also be a plasmid borne antisense repressor RNA, a non limiting list included herein by reference includes RNA-OUT that represses RNA-IN regulated targets (Williams, Supra, 2008), pMB1 plasmid origin encoded RNAI that represses RNAII regulated targets (Grabherr R, Pfaffenzeller I. 2006 US patent application US20060063232; Cranenburgh RM. 2009; US Patent 7,611,883), IncB plasmid pMU720 origin encoded RNAI that represses RNA II regulated targets (Wilson IW, Siemering KR, Praszkier J, Pittard AJ. 1997. J Bacteriol 179:742-53), ParB locus Sok of plasmid R1 that represses Hok regulated targets, Flm locus FlmB of F plasmid that represses flmA regulated targets (Morsey MA, 1999 US patent US5922583). An RNA selectable marker may be another natural antisense repressor RNAs known in the art such as those described in Wagner EGH, Altuvia S, Romby P. 2002. Adv Genet 46:361-98 and Franch T, and Gerdes K. 2000. Current Opin Microbiol 3:159-64. An RNA selectable marker may also be an engineered repressor RNAs such as synthetic small RNAs expressed SgrS, MicC or MicF scaffolds as described in Na D, Yoo SM, Chung H, Park H, Park JH, Lee SY. 2013. Nat Biotechnol 31:170-4. An RNA selectable marker may also be an engineered repressor RNA as part of a selectable marker that represses a target RNA fused to a target gene to be regulated such as SacB as described in Williams, Supra, 2015
ROP: Repressor of primer.
RSM: RNA selectable marker.
SA: Splice Acceptor, consensus sequence YYYYYYYYYYYAGRW is presented as SEQ ID NO:29. To create an efficiently spliced SA site, a splice branch point (consensus sequence YTNAY) is included upstream of the splice acceptor site (see Figure 1).
SacB: Structural gene encoding Bacillus subtilis levansucrase. Expression of SacB in gram negative bacteria is toxic in the presence of sucrose.
SD: Splice Donor, consensus sequence AGGTRAGT.
SEAP: Secreted alkaline phosphatase.
Selectable marker: A selectable marker, for example a kanamycin resistance gene or an RNA selectable marker.
Selection marker: A selectable marker, for example a kanamycin resistance gene or an RNA selectable marker.
SIDD: supercoiling-induced DNA duplex destabilized (SIDD) structures. These sites, when incorporated into a vector, may alter the susceptibility of other sequences within the vector to be destabilized. This can alter function. For example, addition of a SIDD site to an expression vector may reduce the helical destabilization of a promoter. This may increase or decrease promoter activity, depending on the promoter since some promoters have increased expression with promoter helical destabilization, while others will have reduced expression with promoter helical destabilization.
shRNA: Short hairpin RNA.
S/MAR: Scaffold/matrix attachment region as specified elsewhere herein. Eukaryotic sequences that mediate DNA attachment to the nuclear matrix.
Spacer region: As used herein, spacer region is the region linking the 5' and 3' ends of the eukaryotic region sequences. The eukaryotic region 5' and 3' ends are typically separated by the bacterial replication origin and bacterial selectable marker in plasmid vectors.
SR: Spacer region.
SV40 origin: Simian Virus 40 genomic DNA that contains the origin of replication.
SV40 enhancer: Simian Virus 40 genomic DNA that contains the 72 bp and optionally the 21 bp enhancer repeats.
target antigen: Immunogenic protein or peptide epitope, or combination of proteins and epitopes, against which an immune response can be mounted. Target antigens may by derived from a pathogen for infectious disease or allergy applications or derived from a host organism for applications such as cancer, allergy, or autoimmune diseases. Target antigens are well defined in the art. Some examples are described in Williams, Supra, 2008 and are included herein by reference.
TE buffer: A solution containing approximately 10mM Tris pH 8 and 1 mM EDTA.
TetR: Tetracycline resistance gene.
Transcription terminator: Bacterial: A DNA sequence that marks the end of a gene or operon for transcription. This may be an intrinsic transcription terminator or a Rho-dependent transcriptional terminator. For an intrinsic terminator, such as the trpA terminator, a hairpin structure forms within the transcript that disrupts the mRNA-DNA-RNA polymerase ternary complex. Alternatively, Rho-dependent transcriptional terminators require Rho factor, an RNA helicase protein complex, to disrupt the nascent mRNA-DNA-RNA polymerase ternary complex. Eukaryotic: PolyA signals are not 'terminators', instead internal cleavage at PolyA sites leaves an uncapped 5'end on the 3'UTR RNA for nuclease digestion. Nuclease catches up to RNA Pol II and causes termination. Termination can be promoted within a short region of the poly A site by introduction of RNA Pol II pause sites (eukaryotic transcription terminator). Pausing of RNA Pol II allows the nuclease introduced into the 3' UTR mRNA after PolyA cleavage to catch up to RNA Pol II at the pause site. A nonlimiting list of eukaryotic transcription terminators know in the art include the C2x4 and the gastrin terminator. Eukaryotic transcription terminators may elevate mRNA levels by enhancing proper 3'-end processing of mRNA.
transfection: Method to deliver nucleic acids into cells [e.g. poly(lactide-co-glycolide) (PLGA), ISCOMs, liposomes, niosomes, virosomes, chitosan, and other biodegradable polymers, microparticles, microspheres, nanoparticles, nanocapsules, electroporation, nucleofection, piezoelectric permeabilization, sonoporation, iontophoresis, ultrasound, SQZ high speed cell deformation mediated membrane disruption, corona plasma, plasma facilitated delivery, tissue tolerable plasma, laser microporation, shock wave energy, magnetic fields, contactless magneto- permeabilization, gene gun, microneedles, microdermabrasion, hydrodynamic delivery, high pressure tail vein injection, etc] as known in the art and included herein by reference.
Transgene: Gene of interest that is cloned into a vector for expression in a target organism. ts: Temperature sensitive
µg: Microgram
µl: Microliter
UCOE: Ubiquitous Chromatin Opening Element, such as the A2UCOE or minimal derivatives as disclosed in Muller-Kuller et al., 2015, Nucleic Acids Research 43:1577.
UTR: Untranslated region of a mRNA (5' or 3' to the coding region).
Vector: A gene delivery vehicle, including viral (e.g. Alphavirus, Poxvirus, Lentivirus, Retrovirus, Adenovirus, Adenovirus related virus, Integration-Deficient Lentiviral vectors, etc.) and non-viral (e.g. plasmid, Nanoplasmid, MIDGE, transcriptionally active PCR fragment, minicircles, bacteriophage, etc.) vectors. These are well known in the art and are included herein by reference.
Vector backbone: Eukaryotic region and bacterial region of a vector, without the transgene or target antigen coding region.
Vertebrate expression vector: A vector for expression of mRNA, protein antigens, protein therapeutics, shRNA, RNA or microRNA genes in a target vertebrate cell or organism using RNA Polymerase I, II or III promoters.

### DETAILED DESCRIPTION OF THE PREFERRED EMBODIMENTS

The current technology relates generally to self-replicating non-integrative episomal vertebrate expression vector methods and compositions that improve episomal replication and transgene expression. The current technology can be practiced to improve expression and episomal replication of vectors such as non-viral vectors and viral vectors (e.g. episomal Integration-Deficient Lentivirus vector, Non-integrating Lentiviral vectors, episomal Retroviral vector, etc.).

Improved episomal replication is defined herein as improved non-integrative episomal vector establishment and/or maintenance in vitro or in vivo compared to a plasmid that does not incorporate the current technology. Improved plasmid expression is defined herein as improved transgene expression level and/or expression duration in vitro or in vivo compared to a transgene encoding plasmid that does not incorporate the current technology. It is to be understood that all references cited herein are incorporated by reference in their entirety.

The methods of plasmid modification of the present current technology have been surprisingly found to provide a solution to provide self-replicating non-integrative episomal vectors with efficient establishment.

The vector methods and compositions disclosed herein are 3' UTR SD-SMAR-SA compositions with improved expression and or episomal establishment (improved performance) compared to non SD-SA versions. Improved performance is not S/MAR specific since performance improvement is observed with various S/MARs. Improved performance is also not vector transcription unit specific, since performance improvement is observed with SD-SMAR-SA linked to various promoters, 5' UTRs, transgenes, and polyA signals. Improved performance is observed with or without upstream introns. Thus, the 3' UTR SD-SMAR-SA vectors of the disclosure are broadly applicable to improve self-replicating non-integrative episomal vertebrate expression vector performance.

The disclosed improved performance of 3' UTR SD-SMAR-SA compared to non SD-SA versions is surprising in light of the prior art. For example, Le Hir et al., 2003 Trends in Biochemical Sciences 28:215 teaches 'Matsumoto et al. [51] found these translational effects to be highly dependent on intron position. In their study, an intron placed in the 5' UTR was highly stimulatory, whereas the same intron placed in the 3' UTR repressed translation to below the level of the corresponding intronless mRNA.' .......'Nonetheless, for researchers interested in optimizing the expression of transgenes, it is important to note that intron position is an important variable. In addition to potentially inhibiting translation, introns in the 3' UTR can trigger nonsense-mediated decay (NMD) of the mRNA as described below, resulting in even lower protein expression.' Barrett et al., 2012 Cell. Mol. Life Sci. 69:3613 teaches 'In contrast to 5'UTRs, 3'UTRs were found to have relatively few introns (5 %) [21]. A study looking at rare cases of intron acquisition in retroposed mammalian genes found that the presence of an intron in the 3'UTR of these genes resulted in down regulation of gene expression by nonsense-mediated decay [52]. This negative effect on expression offers an explanation for the low prevalence of 3'UTR introns.' While not limiting the application of the invention, adding flanking splice donor and splice acceptor splice sites may have an unexpected benefit in the disclosed invention in which the 3' UTR encodes an S/MAR sequence.

As used herein, the term "sequence identity" refers to the degree of identity between any given query sequence, e.g. SEQ ID NO: 2, and a subject sequence. A subject sequence may, for example, have at least 90 percent, at least 95 percent, or at least 99 percent sequence identity to a given query sequence. To determine percent sequence identity, a query sequence (e.g. a nucleic acid sequence) is aligned to one or more subject sequences using any suitable sequence alignment program that is well known in the art, for instance, the computer program ClustalW (version 1.83, default parameters), which allows alignments of nucleic acid sequences to be carried out across their entire length (global alignment). Chema et al., 2003 Nucleic Acids Res., 31:3497-500. In a preferred method, the sequence alignment program (e.g. ClustalW) calculates the best match between a query and one or more subject sequences, and aligns them so that identities, similarities, and differences can be determined. Gaps of one or more nucleotides can be inserted into a query sequence, a subject sequence, or both, to maximize sequence alignments. For fast pair-wise alignments of nucleic acid sequences, suitable default parameters can be selected that are appropriate for the particular alignment program. The output is a sequence alignment that reflects the relationship between sequences. To further determine percent identity of a subject nucleic acid sequence to a query sequence, the sequences are aligned using the alignment program, the number of identical matches in the alignment is divided by the length of the query sequence, and the result is multiplied by 100. It is noted that the percent identity value can be rounded to the nearest tenth. For example, 78.11, 78.12, 78.13, and 78.14 are rounded down to 78.1, while 78.15, 78.16, 78.17, 78.18, and 78.19 are rounded up to 78.2.

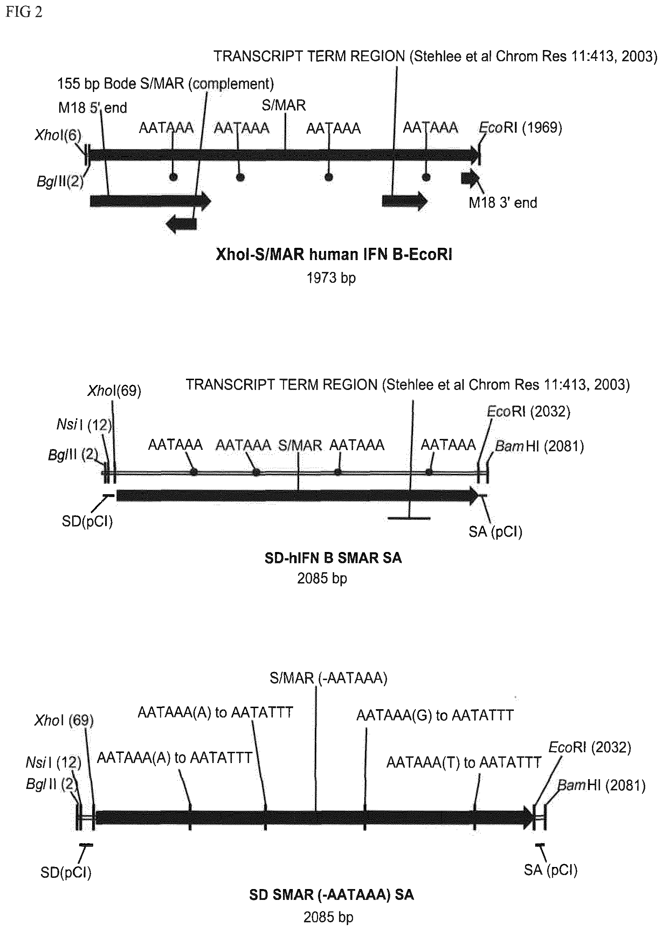

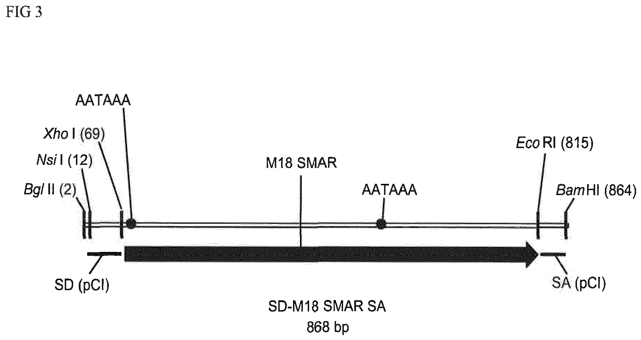

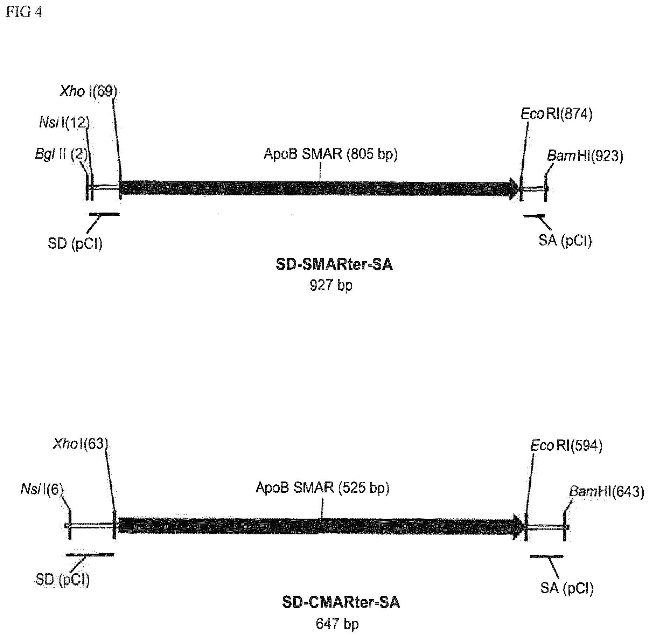

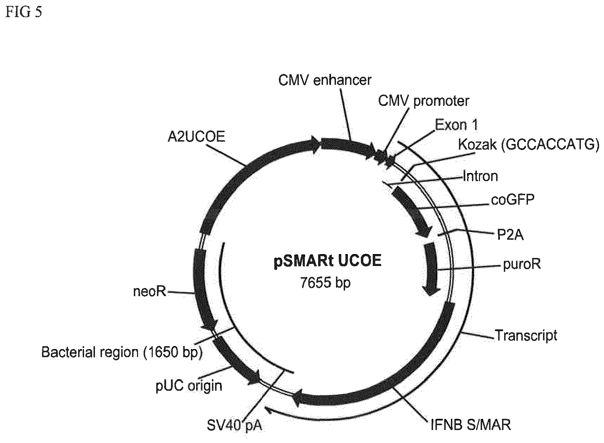

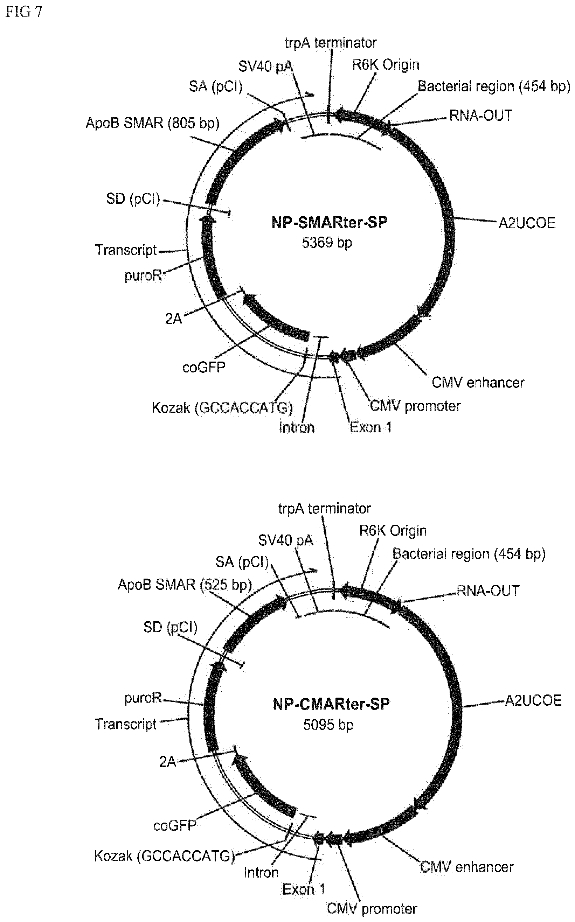

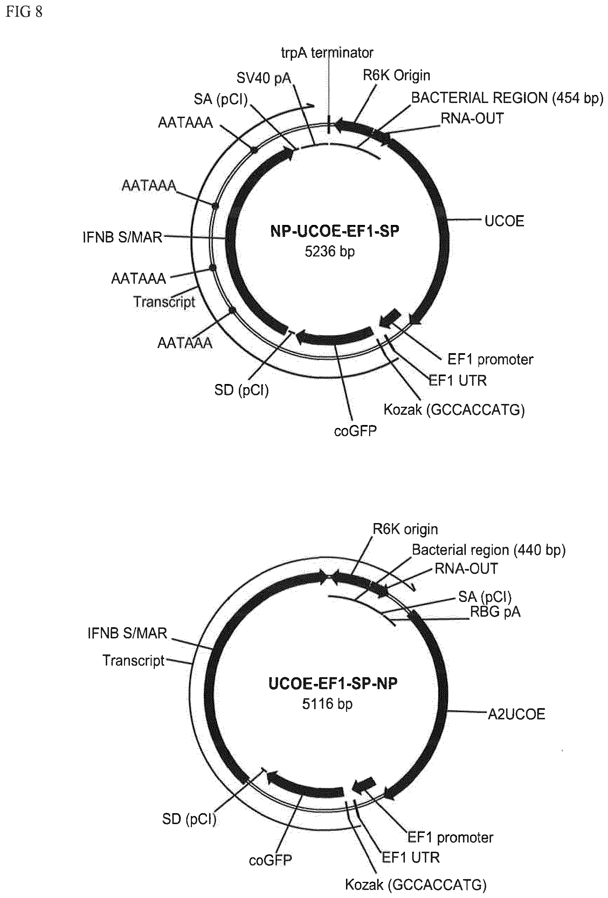

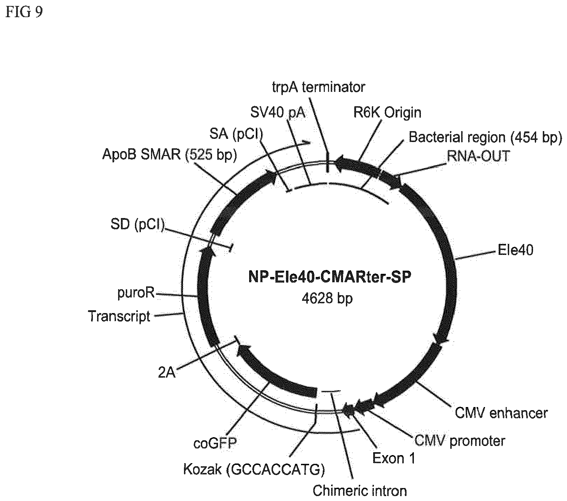

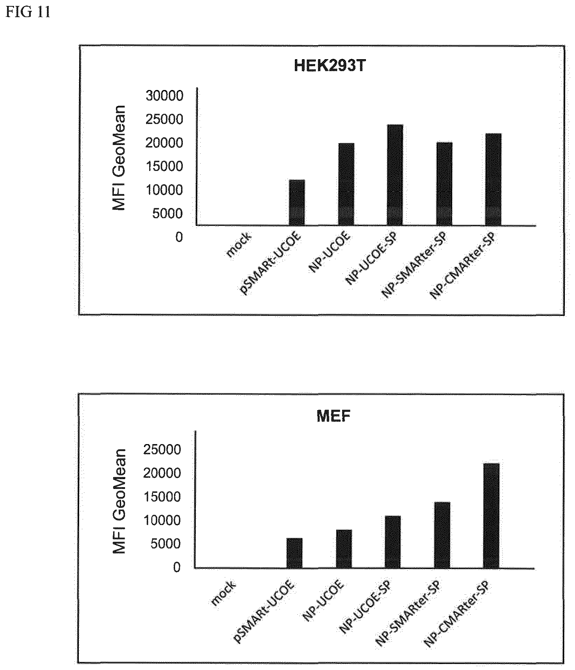

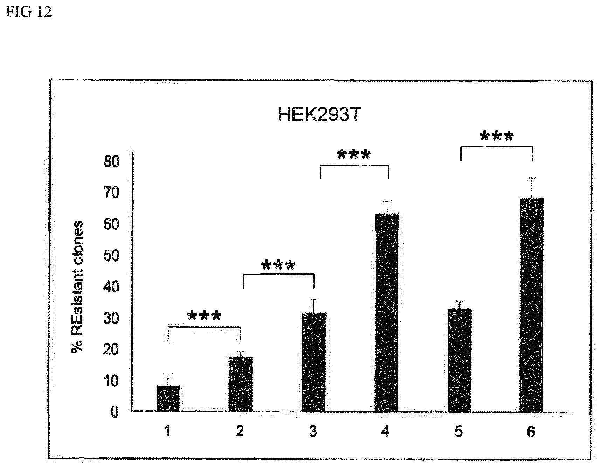

Turning now to the drawings, FIG. 1. shows annotated maps of the pCI intron (top), splice donor (SD) region (middle) and branch point and splice acceptor (SA) region (bottom). FIG. 2 shows annotated maps of the interferon beta S/MAR (top), and a SD interferon beta S/MAR SA derivative (middle), as well as a SD interferon beta S/MAR SA derivative in which the internal AATAAA polyadenylation signals were mutated (bottom). FIG. 3 shows annotated maps of the interferon beta S/MAR derivative M18 with flanking SD and SA sites. FIG. 4 shows annotated maps of the 805 bp (top) or 525 bp (bottom) apoB S/MAR with flanking SDand SA sites. FIG. 5 shows an annotated map of the pMAX-UCOE-coGFP P2A-PuroR-NP (pSMARt UCOE) vector FIG. 6 shows annotated maps of the NTC9385R-UCOE-CMV-coGFP P2A-PuroR -SMAR-SV40 pA (NP-UCOE) and NTC9385R-UCOE-CMV- coGFP P2A-PuroR - SD SMAR- SA SV40 pA (NP-UCOE-SP) vectors. FIG. 7 shows annotated maps of the NTC9385R-SP-UCOE-CMV-GFP SMARter (NP-SMARter-SP) and NTC9385R-SP-UCOE-CMV-GFP CMARter (NP-CMARter-SP) vectors. FIG. 8 shows annotated maps of the NTC9385R- UCOE EF1 -coGFP SD -SMAR SA SV40 pA (NP-UCOE-EF1-SP) and NTC9385R- UCOE EF1-coGFP-SD SMAR R6K-R-OUT-SA pA (UCOE-EF1-SP-NP) vectors. FIG. 9 shows annotated maps of the NTC9385R-SP-ELE40-CMV-GFP CMARter (NP-Ele40-CMARter-SP) vector. FIG. 10 shows improved expression of established S/MAR vectors with and without flanking SD and SA sites. Left panel: MFI of HEK293T cells established with a S/MAR vector with and without splice junctions. The vectors contain NP bacterial region, the genomic insulator UCOE, the expression cassette GFP -2A-PuroR driven by the CMV promoter and the interferon beta S/MAR in the 3' UTR with (Nano-S/MAR-splice = NP-UCOE-SP; NTC9385R-UCOE-CMV- coGFP P2A-PuroR - SD SMAR- SA SV40 pA Figure 6) or without (NP-UCOE; NTC9385R-UCOE-CMV- coGFP P2A-PuroR -SMAR-SV40 pA, Figure 6) S/MAR flanking SD and SA sites. Right panel: the improved transcription expression is confirmed by real time PCR analysis. The expression of the transgene GFP was normalized to the housekeeping gene GAPDH. FIG. 11 shows improved expression of established S/MAR vectors with and without flanking SD and SA sites. MFI of established cells (HEK293T and primary Mouse Embryonic Fibroblast) with vectors harboring different S/MARs flanked by splicing junctions. Vector names are as in Figures 5, 6 and 7. FIG. 12 shows improved establishment of S/MAR vectors with and without flanking SD and SA sites. Colony forming assay conducted in HEK293T with vectors harboring two different S/MARs (interferon beta S/MAR; ApoB S/MAR, 805 bp) with and without flanking SD and SA sites. pEPI is a CMV promoter plasmid vector with a 3' UTR interferon beta S/MAR.

### EXAMPLES

The methods of the current technology are further illustrated by the following examples. These are provided by way of illustration and are not intended in any way to limit the scope of the disclosure.

### Example 1: pUC, and R6K replication origin plasmid production

RNA-OUT antibiotic free selectable marker background: Antibiotic-free selection is performed in E. coli strains containing phage lambda attachment site chromosomally integrated pCAH63-CAT RNA-IN-SacB (P5/6 6/6) as described in Williams, Supra, 2008. SacB (Bacillus subtilis levansucrase) is a counter selectable marker which is lethal to E. coli cells in the presence of sucrose. Translation of SacB from the RNA-IN-SacB transcript is inhibited by plasmid encoded RNA-OUT. This facilitates plasmid selection in the presence of sucrose, by inhibition of SacB mediated lethality.

R6K origin vector replication and production background: The R6K gamma plasmid replication origin requires a single plasmid replication protein 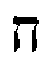 that binds as a replication initiating monomer to multiple repeated 'iteron'. Use of a conditional replication origin such as R6K gamma that requires a specialized cell line for propagation adds a safety margin since the vector will not replicate if transferred to a patient's endogenous flora.

A highly minimalized R6K gamma derived replication origin (SEQ ID NO:1) that contains core sequences required for replication was described in Williams, Supra, 2014 and included herein by reference. The NTC9385R NanoplasmidTM backbone including this minimalized R6K origin and the RNA-OUT AF selectable marker in the spacer region, was described in Williams, Supra, 2014 and included herein by reference. Williams, Supra, 2014 describes host strains expressing phage HK022 attachment site integrated pL promoter heat inducible 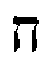 P42L, P106L and F107S high copy mutant replication (Rep) protein for selection and propagation of R6K origin NanoplasmidTM vectors. This is an additional NanoplasmidTM safety factor since R6K origin vectors can only replicate within the engineered Rep protein-expressing E. coli host strain.

Shake flask production : pUC origin plasmid production was performed in E. coli strain DH5α [F- Φ80lacZΔM15 Δ(lacZYA-argF) U169 recA1 endA1 hsdR17 (rK-, mK+) phoA supE44 λ- thi-1 gyrA96 relA1] (Invitrogen, Carlsbad CA). R6K origin-RNA-OUT sucrose selection NanoplasmidTM vectors was performed in host strains NTC940211 DH5α attλ::P5/6 6/6-RNA-IN- SacB, catR; attHK022::pL (OL1-G to T) P42L-P106I-F107S or NTC1050811 DH5α attλ::P5/6 6/6-RNA-IN- SacB, catR; attHK022::pL (OL1-G to T) P42L-P106I-F107S P113S (P3-), SpecR StrepR; attϕ80::pARA-CI857ts, tetR. Shake flask production was performed using proprietary Plasmid+ shake culture medium. The seed cultures were started from glycerol stocks or colonies and streaked onto LB medium agar plates containing 50 µg/mL antibiotic (for ampR or kanR selection plasmids) or 6% sucrose (for RNA-OUT selection plasmids). The plates were grown at 30-32°C; cells were resuspended in media and used to provide approximately 2.5 OD600 inoculums for the 500 mL Plasmid+ shake flasks that contained 50 µg/mL antibiotic for ampR or kanR selection plasmids or 0.5% sucrose to select for RNA-OUT plasmids. Flask were grown with shaking to saturation.

### Example 2: S/MAR vector construction

The pNTC-NP1, pNTC-NP2, pNTC-NP3, pNTC-NP4, pNTC-NP5, pNTC-NP6, pNTC-NP7, vectors encode the R6K gamma origin-RNA-OUT bacterial replication-selection region (SEQ ID NO:8) cloned into the polylinker region of a pUC57 based vector. The pNTC-3xCpG NP1 vector encode the 1 CpG R6K gamma origin- 2 CpG RNA-OUT bacterial replication-selection region (SEQ ID NO:9) cloned into the polylinker region of a pUC57 based vector. Each vector has different flanking restriction sites that can be used to retrofit a target vector to R6K replication-RNA-OUT selection. The 5' and 3' polylinker sequences flanking the R6K-RNA-OUT insert in the pNTC-NP 1-7 vectors and pNTC-3xCpG NP1 are shown in Table 1.

**Table 1: pNTC multiple cloning site flanked R6K Origin-RNA-OUT selection marker vectors**

| **Vector** | **R6K 5' flanking restriction sites** | **trpA term** | **R6K origin** | **Linker site** | **RNA OUT Selection marker** | **RNA-OUT 3' flanking restriction site** |
|---|---|---|---|---|---|---|
| pNTC-NP1 (SEQ ID NO:10) | EcoRI, SacI, KpnI, NruI, NsiI, XmaIII, NotI, *NheI* | Yes | SEQ ID NO: 1 | DraIII^{a} | SEQ ID NO: 5 | *NheI* BamHI, Xmal, ApaI, SalI, HincII, PstI, StuI, AatI, SphI, *HindIII* (in R6K) |
| pNTC-NP2 (SEQ ID NO:11) | EcoRI, SacI, KpnI, NruI, NsiI, XmaIII, NotI, NheI | Yes | SEQ ID NO: 1 | DraIII^{a} | SEQ ID NO: 5 | SpeI, *XmaI,* SspI BamHI, *XmaI*, ApaI, Sail, HincII, PstI, StuI, AatI, SphI, *HindIII* (in R6K) |
| pNTC-NP3 (SEQ ID NO:12) | EcoRI, *SacI, KpnI,* NruI, NsiI, XmaIII, NotI, NheI | Yes | SEQ ID NO: 1 | DraIII^{a} | SEQ ID NO: 5 | *KpnI, SacI* BamHI, XmaI, ApaI, Sail, HincII, PstI, StuI, AatI, SphI, *HindIII* (in R6K) |
| pNTC-NP4 (SEQ ID NO:13) | NheI, XmaIII, NotI, NsiI, NruI, *KpnI, SacI* BamHI, XmaI, ApaI, Sail, HincII, SfcI, PstI, StuI, AatI, SphI, *HindIII* (in R6K) | Yes | SEQ ID NO: 1 | DraIII^{a} | SEQ ID NO: 5 | EcoRI, *SacI, KpnI* |
| pNTC-NP5 (SEQ ID NO:14) | KasI, NheI | Yes | SEQ ID NO: 1 | DraIII^{a} | SEQ ID NO: 5 | KpnI AflIII PstI, AatI, SphI, *HindIII* (in R6K) |
| pNTC-NP6 (SEQ ID NO:15) | EcoRI, PstI, EcoRV, BstXI, NotI, NheI | Yes | SEQ ID NO: | DraIII^{a} | SEQ ID NO: 5 | KpnI, ApaI, PvuI, SalI, SacI |
| pNTC-NP7 (SEQ ID NO:16) | BssHII PacI NheI | Yes | SEQ ID NO: | DraIII^{a} | SEQ ID NO: 5 | KpnI PacI BssHII |
| | | | | | | |
| pNTC-3x CpG NP1 (SEQ ID NO:17) | XhoI, XbaI, ApaI, SalI, HincII, PstI, StuI, AatI, SphI, | No | SEQ ID NO: 2 | BsrGI | SEQ ID NO: 7 | EcoRI, SacI, *KpnI,* NruI, NsiI, XmaIII, NotI, NheI, *KpnI* |

| | | | | | | |
|---|---|---|---|---|---|---|
| a Non-palindromic unique 3 bp NNN sticky end DraIII site (CACNNNGTG) separating R6K and RNA-OUT of sequence CACGTTGTG can be used to assemble R6K and RNA-OUT from separate bpNTC vectors in directional multi-fragment ligation reactions S/MAR vector pUC origin-antibiotic selection bacterial backbone retrofits to R6K-RNA-OUT (i.e., Nanoplasmid, NP, vectors) were performed by: 1) selecting restriction sites that flank the pUC origin and antibiotic selection marker region in the target S/MAR vector; 2) Identifying a pNTC-NP compatible polylinker -R6K-RNA-OUT polylinker cassette (either pNTC- NP1, 2, 3, 4, 5, 6, or 7; Table 1); 3) Excising the pUC origin antibiotic selection marker region and replacing with the selected R6K origin RNA-OUT region using the selected restriction digestion approach and standard ligase mediated cloning. | | | | | | |

In some cases, the R6K origin and RNA-OUT units were assembled in multi-fragment ligations from separate restriction fragments using the non-palindromic DraIII linker site (see Table 1).

Example vector maps and vector characteristics of the original pUC origin- antibiotic selection marker vector (e.g. pSMARt UCOE; Figure 5) and the retrofitted R6K origin-RNA-OUT antibiotic free selection marker vector (e.g. NP-UCOE: Figure 6) are shown.

The SD -S/MAR-SA 3' UTRs were made as synthetic genes as follows. A splice donor site (SEQ ID NO: 25) with 5' BglII and NsiI cloning sites and a 3' XhoI cloning site (Figure 1) was incorporated 5' to the S/MAR, while a splice acceptor site (SEQ ID NO: 26) with 5' EcoRI and 3' BamHI cloning sites (Figure 1) was incorporated 3' to the S/MAR. The genes were synthesized at Genscript (Piscataway, NJ) and cloned in place of the S/MAR in existing SMAR-NP vectors using standard restriction fragment ligation mediated cloning. For example, the interferon beta S/MAR (SEQ ID NO: 18) (e.g. NP-UCOE vector, Figure 6) was replaced with the splice donor-interferon beta S/MAR-splice acceptor (SEQ ID NO:19) (e.g. NP-UCOE-SP vector, Figure 6; NP-UCOE-EF1-SP, Figure 8) or splice donor-interferon beta S/MAR (-AATAAA)-splice acceptor (SEQ ID NO:20) or splice donor-interferon beta M18 S/MAR-splice acceptor (SEQ ID NO:21). Splice donor-interferon beta S/MAR (-AATAAA) was designed to remove S/MAR encoded AATAAA(N) transcription termination signals with an AATATT(T) MAR motif (Figure 2). The 805 bp ApoB S/MAR was replaced with the splice donor-805 bp ApoB S/MAR-splice acceptor version (SEQ ID NO: 22) (e.g. NP-SMARter-SP, Figure 7) while the 525 bp ApoB S/MAR was replaced with the splice donor-525bp ApoB S/MAR-splice acceptor version (SEQ ID NO: 23) (e.g. NP-CMARter-SP, Figure 7; NP-Ele40-CMARter-SP, Figure 9). Additional NP constructs with alternative transgenes, promoters, 5' UTR introns, or ELE40 or UCOE elements were made by standard restriction fragment ligation mediated cloning. All constructs were verified correct by restriction digestion and sequencing.

### Example 3: S/MAR vector expression after transient transfection

Adherent HEK293 (human embryonic kidney) and A549 (human lung carcinoma), cell lines were obtained from the American Type Culture Collection (Manassas, VA, USA). Cell lines were propagated in Dulbecco's modified Eagle's medium/F12 containing 10% fetal bovine serum

and split (0.25% trypsin-EDTA) using Invitrogen (Carlsbad, CA, USA) reagents and conventional methodologies. For transfections, cells were plated on 24-well tissue culture dishes. plasmids were transfected into cell lines using Lipofectamine 2000 following the manufacturer's instructions (Invitrogen).

Total cellular lysates for EGFP determination were prepared by resuspending cells in cell lysis buffer (CelLytic M, Sigma, St Louis, MO, USA), lysing cells by incubating for 30 min at 37°C, followed by a freeze-thaw cycle at -80 °C. Lysed cells were clarified by centrifugation and the supernatants assayed for EGFP by FLX800 microplate fluorescence reader (Bio-Tek, Winooski, VT, USA). The results are summarized in Tables 2-4.

**Table 2: Transient expression of S/MAR vectors after transfection into A549 and HEK293 cell lines**

| **Plasmid** | **Promoter** | **Intron** | **3-UTR** | **A549 GFP^{a}** | **HEK GFP^{a}** |
|---|---|---|---|---|---|
| NTC9385R- UCOE EF1 - coGFP | UCOE EF1 | None | hIFNB SMAR- SV40 | 552 ± 95 | 5168 ± 202 |
| NTC9385R- UCOE EF1 - coGFP SD -SMAR SA SV40 pA | UCOE EF1 | None | SD hIFNB SMAR SA -SV40 pA | 1139 ± 181 | 13909 ± 1068 |
| NTC9385R- UCOE EF1-coGFP- SMAR R6K-ROUT- pA | UCOE EF1 | None | hIFNB SMAR-R6K-R-OUT RBG | 607 ± 217 | *7552* ± *1754* |
| NTC9385R- UCOE EF1-coGFP- SD SMAR R6K-R-OUT-SA pA **(UCOE-EF1-SP-NP-****Figure 8****)** | UCOE EF1 | None | SD hIFNB SMAR-R6K-R-OUT SA RBG pA | 961 ± 83 | 12956 ± 848 |
| NTC9385R- UCOE EF1-coGFP-SD M18 SMAR R6K-R-OUT- | UCOE EF1 | None | SD M18 SMAR-R6K-R-OUT SA RBG pA | 2088 ± 449 | 16761 ± 954 |
| NTC9385R- UCOE EF1 - coGFP SD -M18 SMAR SA SV40 pA | UCOE EF1 | None | SD M18 SMAR SA-SV40 pA | 3190 ± 386 | 22640 ± 1129 |

| | | | | | |
|---|---|---|---|---|---|
| a Results presented are mean fluorescent units ± standard deviation at 2 days post transfection | | | | | |

**Table 3: Transient expression of S/MAR vectors after transfection into A549 and HEK293 cell lines**

| **Plasmid** | **Promoter** | **Intron** | **3-UTR** | **A549 GFP** | **HEK GFP** |
|---|---|---|---|---|---|
| NTC9385R- EF1-coGFP - SMAR SV40 pA | EF1 | None | hIFNB SMAR-SV40 pA | 1221 ± | 2038 ± |
| NTC9385R- UCOE EF1 - coGFP -SMAR SV40 pA | UCOE EF1 | None | hIFNB SMAR-SV40 pA | 2251 ± | 7339 ± |
| NTC9385R- UCOE EF1 - coGFP SD -SMAR SA SV40 pA **(NP-UCOE-EF1-SP-****Figure 8****)** | UCOE EF1 | None | SD hIFNB SMAR SA -SV40 pA | 6205 ± 420 | 24507 ± 2501 |
| NTC9385R- UCOE EF1 - coGFP SD -SMAR(-AATAAA) SA SV40 pA | UCOE EF1 | None | SD hIFNB SMAR-AATAAA SA - SV40 pA | 4708 ± 359 | 18910 ± 1278 |
| NTC9385R- EF1-coGFP-SMAR R6K-R-OUT- pA | EF1 | None | hIFNB SMAR-R6K-R-OUT RBG pA | 1240 ± 164 | 1896 ± 189 |
| NTC9385R- UCOE EF1-coGFP-SMAR R6K-R-OUT-pA | UCOE EF1 | None | hIFNB SMAR-R6K-R-OUT RBG pA | 1540 ± 180 | 4996 ± 322 |
| NTC9385R- UCOE EF1-coGFP-SD SMAR R6K-R-OUT-SA pA **(UCOE-EF1-SP-NP-****Figure 8****)** | UCOE EF1 | None | SD hIFNB SMAR-R6K-R-OUT SA RBG pA | 4843 ± 604 | 19247 ± 1693 |
| NTC9385R- UCOE EF1-coGFP-SD M18 SMAR R6K-R-OUT-SA pA | UCOE EF1 | None | SD M18 SMAR-R6K-R-OUT SA RBG pA | 10021 ± 753 | 27981 ± 1121 |
| NTC9385R- UCOE EF1-coGFP SD -M18 SMAR SA SV40 pA | UCOE EF1 | None | SD M18 SMAR SA -SV40 pA | 9751 ± 821 | 29019 ± 2744 |
| NTC9385R-UCOE-CMV-coGFP P2A-PuroR -SMAR-SV40 pA (NP-UCOE -Figure 6) | UCOE CMV | pCI | hIFNB SMAR-SV40 pA | 2104 ± 74 | 8478 ± 320 |
| NTC9385R-UCOE-CMV-coGFP P2A-PuroR - SD SMAR- SA SV40 pA (NP-UCOE-SP **-****Figure 6****)** | UCOE CMV | pCI | SD hIFNB SMAR SA -SV40 pA | 3526 ± 102 | 14278 ± 2664 |
| NTC9385R-UCOE-CMV-coGFP P2A-PuroR - SD SMAR(-AATAAA)- SA SV40 pA | UCOE CMV | pCI | SD hIFNB SMAR-AATAAA SA - SV40 pA | 2876 ± 376 | 13425 ± 1331 |

| | | | | | |
|---|---|---|---|---|---|
| a Results presented are mean fluorescent units ± standard deviation at 2 days post transfection | | | | | |

The results presented in Tables 2 and 3 demonstrate that with a UCOE-EF1 promoter no intron coGFP transgene transcription unit the human IFNB SMAR flanked by SD/SA improves expression in both HEK293 and A549 cell lines compared to human IFNB SMAR without SD/SA sites. Improved expression was observed in 2 SD/SA configurations (flanking SMAR, or flanking SMAR+R6K-RNA-OUT NP bacterial region). The M18 SMAR (derived from human IFNB SMAR) flanked by SD/SA has higher expression than the equivalent human IFNB SMAR flanked by SD/SA.

In addition, the results in Table 3 show improved expression in UCOE-CMV promoter pCI intron coGFP transgene transcription unit (i.e., improved expression with two different promoters, with or without a 5' UTR encoded intron). Improved expression is also observed with different polyadenylation signals (SV40 or RBG derived).

**Table 4: Transient expression of S/MAR vectors after transfection into A549 and HEK293 cell lines**

| **Plasmid** | **Promoter** | **5' UTR Intron** | **3' UTR** | **T=2 day A549 GFP** | **T=2 day HEK GFP** |
|---|---|---|---|---|---|
| NTC9385R- EF1-coGFP - SMAR SV40 pA | EF1 | None | hIFNB SMAR-SV40 | 525 ± 37 | 1377 ± 111 |
| NTC9385R- UCOE EF1 - coGFP -SMAR SV40 pA | UCOE EF1 | None | hIFNB SMAR-SV40 | 1848 ± 163 | 12980 ± 1005 |
| NTC9385R- UCOE EF1-coGFP SD - SMAR SA SV40 pA | UCOE EF1 | None | SD hIFNB SMARSA -SV40 pA | 3091 ± 169 | 22354 ± 1686 |
| NTC9385R- UCOE EF1-coGFP SD - SMAR(-AATAAA) SA SV40 pA | UCOE EF1 | None | SD hIFNB SMAR-AATAAA SA - | 2311 ± 413 | 14768 ± 1628 |
| NTC9385R- UCOE EF1 -coGFP SD- | UCOE EF1 | None | SD M18 SMAR SA- | 4833 ±462 | 21254 ± 6296 |
| NTC9385R-SP-UCOE-EF1-GFP SMARter = | UCOE EF1 | None | SD SMARter SA - SV40 | 2878 ± 233 | 13688 ± 1873 |
| NC9385R-SP-Ele40-EF1-GFP SMARter = coGFP | ELE40 EF1 | None | SD SMARter SA - SV40 pA | 990 ± 175 | 3349 ± 341 |
| )MAX-UCOE-coGFP P2A-PuroR-NP | UCOE CMV | pCI | SMAR-SV40 pA | 933 ± 117 | 6193 ± 533 |
| NTC9385R-UCOE-CMV-coGFP P2A- PuroR -SMAR-SV40 pA | UCOE CMV | pCI | SMAR-SV40 pA | 1081 ± 85 | 8216 ± 211 |
| NTC9385R-UCOE-CMV-coGFP P2A- PuroR - SD SMAR- SA SV40 pA | UCOE CMV | pCI | SD SMAR SA-SV40 pA | 1857 ±207 | 12596 ± 1531 |
| NTC9385R-UCOE-CMV-coGFP P2A- PuroR - SD SMAR(-AATAAA)- SA SV40 pA | UCOE CMV | pCI | SD SMAR-AATAAA SA-SV40 pA | 2204 ± 70 | 13901 ± 1024 |
| NTC9385R-SP-UCOE-CMV-GFP SMARter = coGFP P2A-PuroR **(NP-** | UCOE CMV | pCI | SD SMARter SA - SV40 pA | 917 ± 113 | 8091 ±449 |
| NTC9385R-SP-UCOE-CMV-GFP CMARter = coGFP P2A-PuroR **(NP-** | UCOE CMV | pCI | SD CMARter SA-SV40 pA | 3875 ± 230 | 12020 ± 624 |
| NTC9385R-SP-Ele40-CMV-GFP SMARter = coGFP P2A-PuroR | ELE40 CMV | pCI | SD SMARter SA - SV40 pA | 1524 ± 59 | 5483 ± 393 |

| | | | | | |
|---|---|---|---|---|---|
| a Results presented are mean fluorescent units ± standard deviation at 2 days post transfection | | | | | |

The results presented in Table 4 further demonstrates human IFNB SMAR flanked by SD/SA improves expression in both HEK293 and A549 cell lines compared to human IFNB SMAR without SD/SA site with the UCOE-EF1 promoter no intron coGFP transgene transcription unit and the UCOE-CMV promoter pCI intron coGFP transgene transcription unit (i.e., improved expression with two different promoters, with or without a 5' UTR encoded intron). Additionally, CMARter SMAR flanked by SD/SA has higher expression than human IFNB SMAR flanked by SD/SA. Further, replacement of S/MAR AATAAA(N) transcription termination signals with an AATATT(T) MAR motif resulted in a functional S/MAR, demonstrating that this approach can be used to remove transcription terminator signals from S/MAR elements described in the art. Alternative motifs can be substituted for AATATT(T), for example, AT rich motifs enriched in S/MARs as described by Liebeich et al., Supra, 2002. This AATAAA motif replacement method allows adaption of S/MARs in the art to be utilized in 3' UTRs of the invention, without reducing expression through AATAAA motif-mediated premature transcription termination.

Collectively, the results demonstrate the vectors of the current invention solve the suboptimal expression level limitation of S/MAR based vectors described in the art.

### Example 4: S/MAR vector expression after episome establishment

Expression from NP-UCOE (Figure 6) and NP-UCOE-SP (Figure 6) was determined after episomal establishment in cell line HEK293. Cells were established with the standard protocols which required the application of Puromycin (0.5 □g/ml) for one week before expansion for at least 30 days (Wong and Harbottle, 2013 Mol Ther Nucleic Acids 2:e115). The established populations were analysed for the expression of the reporter gene GFP via FACS and the GFP RNA levels were evaluated via qPCR. The results (Figure 10) demonstrate that human IFNB SMAR flanked by SD/SA improves mRNA transcription and GFP transgene expression compared to human IFNB SMAR without SD/SA site after episomal establishment in the HEK293 cell line. A second experiment demonstrated GFP transgene expression of SD-S/MAR-SA vectors NP-UCOE-SP (Figure 6), NP- SMARter-SP (Figure 7) and NP-CMARter-SP (Figure 7) were improved compared to non SD-SA vector NP-UCOE (Figure 6) after episomal establishment in HEK293 cell line and primary Mouse Embryonic Fibroblast cells.

These results with established cell lines demonstrate the vectors of the current invention solve the gene silencing limitation of S/MAR based vectors described in the art.

### Example 5: S/MAR vector expression after episome establishment

The efficacy in establishing cells was also tested in HEK293T through colony forming assay (Wong and Harbottle, Supra, 2013) with vectors harboring two different S/MARs (interferon beta S/MAR; ApoB S/MAR, 805 bp) with and without flanking SD and SA sites. The results demonstrated (Figure 12) that with both the interferon beta S/MAR and the ApoB S/MAR flanking SD and SA sites dramatically improved efficacy in generating established cells (i.e., producing the highest number of colonies). These results demonstrate the vectors of the current invention solve the low establishment rate limitation of S/MAR based vectors described in the art.

### Example 6: Efficiency of establishment and analysis of the genetically modified cell population (Fig. 1)

The efficacy in generating stably expressing cells was evaluated in a colony forming assay using pS/MARt (Fig. 4, SEQ ID NO:41). Upon DNA delivery, cells positive for GFP transgene expression were isolated via FACS sorting (FACS Aria II) and 100 cells were plated into a 6 cm cell culture dish. They were then cultured for 4 weeks in presence of 0.5 µg/ml Puromycin. After 4 weeks the cells were fixed with PFA and the colonies stained with Crystal Violet. The number of colonies is considered as the efficiency of vector establishment, i.e. the number of colonies forming per number of FACS sorted cells plated. The generation of stable cells lines is very effective with over 40 % of transfected cells becoming established (Fig. 1A)). The number of transgene (GFP) expressing cells was estimated by Flow Cytometry. As shown in Fig. 1 b), pS/MARt generates modified populations in which the expression of the transgene is homogenous without significant numbers of negative cells.

### Example 7: Plasmid rescue of pS/MARt vectors from established cell populations (Fig. 2)

An effective method to determine if DNA vectors are maintained episomally with integrity within modified cells is to verify that they can be rescued intact into naive bacteria. To do so, persistently established cell lines modified with the plasmid pS/MARt were cultured in the presence of the antibiotic Puromycin (0.5 µg/ml) for 1 week and expanded for at least 30 days without antibiotic to evaluate vector integrity. Total DNA was prepared from the cells using the Blood&Tissue DNAeasy kit (Qiagen) and transformed into DH10B E. coli cells. Bacteria were grown on LB-Agar plates with Kanamycin (50µg/ml). 12 colonies were grown in liquid LB medium with Kanamycin (50 µg/ml) overnight and plasmid DNA was extracted with the MiniprepKit (Qiagen). For the analysis the DNA mini preparations were digested with the restriction enzyme BamHI (Thermo Fisher) for 10 min at 37°C and the restriction pattern was addressed on a 1% agarose gel. As control the DNA used for transfecting the cells at the beginning of the establishment procedure was digested with the same enzyme and run as a reference. These gels illustrate that intact pS/MARt DNA could be isolated form stable modified cell lines and that in every instance the DNA was identical to the originally transfected vectors.

### Example 8: pS/MARt vectors are maintained episomally in modified cells (Fig. 3)

To further demonstrate that the pS/MARt vectors were modifying the mammalian cells as an episome, structure was physically determined by Southern Blot analysis. Hek293T cell populations cultured for at least 30 days after DNA transfection were analyzed. The genomic DNA was extracted with the Blood&Tissue DNAeasy kit (Qiagen) and digested over night at 37°C with the restriction enzyme BamHI (NEB). The total cellular DNA was then separated on a 1% agarose gel and transferred to a nylon membrane. Oligonucleotides corresponding to the vector's GFP gene were used to generate the radioactive probe used to detected the pS/MARt DNA within cellular DNA. The presence in the samples of a single band that has the same size of the control vector demonstrates the episomal status of pS/MARt in the established mammalian cell populations. The absence of smears and/or alternative bands demonstrates that the vectors did not rearrange nor integrate into the cellular genome.

The vector methods and compositions disclosed herein and evaluations presented above demonstrate 3' UTR SD-SMAR-SA compositions improved expression and or episomal establishment compared to non SD-SA versions. Improved performance is not S/MAR specific since performance improvement is observed with various S/MARs. Improved performance is also not vector transcription unit specific, since performance improvement is observed with SD-SMAR-SA linked to various promoters, 5' UTRs, transgenes, and polyA signals. Improved performance is observed with or without upstream introns. Thus, the 3' UTR SD-SMAR-SA vectors of the disclosure are broadly applicable to improve self-replicating non-integrative episomal vertebrate expression vector performance.

The vectors of the current technology any intronic splice donor site described in the art could be substituted for the pCI intron derived splice donor. Likewise, any intronic splice acceptor site described in the art could be substituted for the pCI intron derived splice acceptor. For example, splice donors and acceptors may be derived from the HTLV- IR-Rabbit β globin hybrid intron, HTLV- IR CMV hybrid intron, CMV intron, CpG free intron I 140, Human β globin Murine IgG chimeric intron, Adenovirus leader- Murine IgG chimeric intron, Rabbit β globin intron, Truncated CMV intron, CAG (Chicken β Actin-rabbit β globin) intron, CMV-Rabbit β globin hybrid intron disclosed in Williams, Supra, 2014 or other introns described in the art.

The various alternative S/MARs described in the art could also be used in the vectors of the current technology. If necessary, internal polyA sites can be removed by motif replacement as described herein.

The vectors may encode a diversity of transgenes different from the examples provided herein, for example, antigen genes for a variety of pathogens, or therapeutic genes such as hypoxia inducible factor, keratinocyte growth factor, factor IX, factor VIII, Fanconi anemia complementation group A protein, homogentisate dioxygenase, etc or polyproteins such as a reprogramming factor polyprotein.

Likewise, the vectors may utilize a diversity of RNA Pol II promoters different from the CMV and elongation factor 1 (EF1) promoter examples provided herein, for example, constitutive promoters such as the chicken β -actin promoter, the β -actin promoter from other species, the phosphoglycerokinase (PGK) promoter, the spleen focus-forming virus (SFFV) promoter, the Rous sarcoma virus (RSV) promoter, the human serum albumin (SA) promoter, the thyroxine binding globulin (TBG) promoter, the cytochrome P450 2E1 (CYP2E1) promoter, etc. The vectors may also utilize combination promoters such as the chicken β - actin/CMV enhancer (CAG) promoter, the human or murine CMV-derived enhancer elements combined with the elongation factor 1α (EF1α) promoters, CpG free versions of the human or murine CMV-derived enhancer elements combined with the elongation factor 1α (EF1α) promoters, the albumin promoter combined with an α - fetoprotein MERII enhancer, etc, or the diversity of tissue specific or inducible promoters know in the art such as the muscle specific promoters muscle creatine kinase (MCK), and C5-12 or the liver-specific promoters apolipoprotein A-I (ApoAI), α -1 antitrypsin (AAT) promoter, AAT-TTR promoter, SERP-TTR promoter, and ApoE-hAAT, or T-cell promoters such as hTCR8.1, CD4 and WASp. Additionally, in the Nanoplasmid backbone, various orientations of the R6K replication origin, and the RNA selectable marker, may be utilized. For example, any of the eight orientations of the R6K replication origin, and the RNA selectable marker in vectors of the current technology may be used (i.e., ←Pol III replication origin RSM→; Pol III replication origin ← RSM; Pol III replication origin → RSM →; Pol III replication origin → ← RSM; ← RSM Pol III replication origin →; ← RSM ← Pol III replication origin; RSM → Pol III replication origin →; RSM → ←Pol III replication origin). Further, a variety of RNA selectable markers know in the art may be substituted for RNA-OUT. Thus, the reader will see that the improved self-replicating non-integrative episomal vertebrate expression vectors of the current technology provide for an approach to improve non- integrative episomal replication plasmid encoded transgene expression.
Accordingly, the scope of the disclosure should be determined not by the embodiments illustrated, but by the appended claims.

## Claims

1. An *in vitro* method for improving the expression and establishment efficiency of a self-replicating non- integrative episomal S/MAR expression vector in a target vertebrate cell comprising the following steps:
a. providing an episomal S/MAR expression vector comprising:
i. a bacterial replication-selection region comprising a bacterial origin of replication and a selectable marker;
ii. a transcription unit for expression of a transgene in a vertebrate cell, comprising a promoter, a 5' UTR, a transgene, and a 3' UTR;
iii. an S/MAR insert located within said 3' UTR; and
b. modifying the episomal S/MAR expression vector such that the S/MAR is flanked by a 5' splice donor site and a 3' splice acceptor site within said 3' UTR, whereby the resultant self-replicating non-integrative episomal S/MAR expression vector has improved expression and establishment efficiency after transfection of a vertebrate cell.

2. The method of claim 1, wherein said S/MAR insert contains internal AATAAA transcription termination motifs.

3. The method of claim 2, wherein said AATAAA transcription termination motifs in said S/MAR are replaced with AATATT motifs.

4. The method of claim 1, wherein said S/MAR is selected from the group consisting of human Interferon beta S/MAR, M18 S/MAR, Apolipoprotein B S/MAR.

5. The method of claim 1, wherein said SMAR flanked by a 5' splice donor site and a 3' splice acceptor site has at least 95% sequence identity to a sequence selected from the group consisting of SEQ ID NO: 19, SEQ ID NO: 20, SEQ ID NO: 21, SEQ ID NO: 22, and SEQ ID NO: 23.

6. The method of claim 1, wherein said bacterial origin of replication is an R6K gamma replication origin.

7. The method of claim 1, wherein said selectable marker is an RNA-IN regulating RNA-OUT functional variant with at least 95% sequence identity to a sequence selected from the group consisting of SEQ ID NO: 5, and SEQ ID NO: 7.

8. The method of claim 1, wherein said self-replicating non-integrative episomal S/MAR expression vector is selected from the group consisting of plasmid vector, Nanoplasmid vector, Integration-Deficient Lentivirus vector, and Non-integrating Lentiviral vectors.

9. A polynucleotide comprising at least one promoter and an S/MAR element, wherein said S/MAR element is located downstream of said promoter and wherein the nucleic acid sequence of said S/MAR element (S/MAR sequence) comprises at least 3 sequence motifs ATTA per 100 nucleotides over a stretch of at most 200 nucleotides, wherein said S/MAR element is flanked by a splice donor and a splice acceptor, and wherein said polynucleotide further comprises an R6K gamma replication origin with at least 95% sequence identity to a sequence selected from the group consisting of SEQ ID NO: 1, SEQ ID NO:2, SEQ ID NO: 3 and SEQ ID NO: 4.

10. The polynucleotide of claim 9, wherein said promoter is comprised in a transcription unit for expression of a cargo polypeptide and/or a selectable marker in a host cell.

11. The polynucleotide according to claim 10, wherein said transcription unit comprises a promoter, a 5' UTR, a transgene, and a 3' UTR.

12. The polynucleotide of claim 11, wherein said S/MAR is located within said 3' UTR.

13. A covalently closed circular recombinant DNA molecule comprising:
a. an transcription unit for expression of a transgene in a vertebrate cell, comprising a promoter, a 5' UTR, a transgene, and a 3' UTR;
b. an S/MAR located within said 3' UTR wherein said S/MAR is flanked by a 5' splice donor site and a 3' splice acceptor site;
c. an R6K gamma replication origin with at least 95% sequence identity to a sequence selected from the group consisting of SEQ ID NO: 1, SEQ ID NO: 2, SEQ ID NO: 3, and SEQ ID NO: 4; and
d. an RNA-OUT RNA selectable marker comprising an RNA-IN regulating RNA-OUT functional variant with at least 95% sequence identity to a sequence selected from the group consisting of SEQ ID NO: 5, and SEQ ID NO: 7.

14. The polynucleotide of any one of claims 9 to 12 or the recombinant DNA molecule of claim 13, wherein said S/MAR is selected from the group consisting of human Interferon beta S/MAR, M18 S/MAR, Apolipoprotein B S/MAR.

15. The polynucleotide of any one of claims 9 to 12 or the recombinant DNA molecule of claim 13, wherein said SMAR flanked by a 5' splice donor site and a 3' splice acceptor site has at least 95% sequence identity to a sequence selected from the group consisting of SEQ ID NO: 19, SEQ ID NO: 20, SEQ ID NO: 21, SEQ ID NO: 22, and SEQ ID NO: 23.

## Patentansprüche

1. In-vitro-Verfahren zur Verbesserung der Expression und Etablierungseffizienz eines selbstreplizierenden nicht integrativen episomalen S/MAR-Expressionsvektors in einer Ziel-Vertebratenzelle, umfassend die folgenden Schritte:
a. Bereitstellen eines episomalen S/MAR-Expressionsvektors, der Folgendes umfasst:
i. eine bakterielle Replikations-/Selektions-Region, die einen bakteriellen Replikationsursprung und einen selektierbaren Marker umfasst;
ii. eine Transkriptionseinheit zur Expression eines Transgens in einer Vertebratenzelle, die einen Promotor, eine 5'-UTR, ein Transgen und eine 3'-UTR umfasst;
iii. ein S/MAR-Insert, das in der 3'-UTR liegt; und
b. Modifizieren des episomalen S/MAR-Expressionsvektors, so dass die S/MAR von einer 5'-Spleißdonor-Stelle und einer 3'-Spleißakzeptor-Stelle innerhalb der 3'-UTR flankiert wird, wodurch der resultierende selbstreplizierende nicht integrative episomale S/MAR-Expressionsvektor eine verbesserte Expression und Etablierungseffizienz nach Transfektion einer Vertebratenzelle aufweist.

2. Verfahren nach Anspruch 1, wobei das S/MAR-Insert interne AATAAA-Transkriptionsterminationsmotive aufweist.

3. Verfahren nach Anspruch 2, wobei die AATAAA-Transkriptionsterminationsmotive in der S/MAR durch AATATT-Motive ersetzt werden.

4. Verfahren nach Anspruch 1, wobei die S/MAR ausgewählt ist aus der Gruppe bestehend aus menschlicher Interferon-beta-S/MAR, M18-S/MAR, Apolipoprotein-B-S/MAR.

5. Verfahren nach Anspruch 1, wobei die von einer 5'-Spleißdonor-Stelle und einer 3'-Spleißakzeptor-Stelle flankierte SMAR eine Sequenzidentität von wenigstens 95% mit einer Sequenz ausgewählt aus der Gruppe bestehend aus SEQ ID NO: 19, SEQ ID NO: 20, SEQ ID NO: 21, SEQ ID NO: 22 und SEQ ID NO: 23 aufweist.

6. Verfahren nach Anspruch 1, wobei es sich bei dem bakteriellen Replikationsursprung um einen R6K-gamma-Replikationsursprung handelt.

7. Verfahren nach Anspruch 1, wobei es sich bei dem selektierbaren Marker um eine RNA-IN regulierende RNA-OUT-Funktionsvariante mit einer Sequenzidentität von wenigstens 95% mit einer Sequenz ausgewählt aus der Gruppe bestehend aus SEQ ID NO: 5 und SEQ ID NO: 7 handelt.

8. Verfahren nach Anspruch 1, wobei der selbstreplizierende nicht integrative episomale S/MAR-Expressionsvektor ausgewählt ist aus der Gruppe bestehend aus Plasmidvektor, Nanoplasmidvektor, Integrationsdefizientes-Lentivirus-Vektor und nicht integrierenden Lentivirusvektoren.

9. Polynukleotid, umfassend wenigstens einen Promotor und ein S/MAR-Element, wobei das S/MAR-Element stromabwärts vom Promotor liegt und wobei die Nukleinsäuresequenz des S/MAR-Elements (S/MAR-Sequenz) wenigstens 3 Sequenzmotive ATTA pro 100 Nukleotide über eine Strecke von höchstens 200 Nukleotiden aufweist, wobei das S/MAR-Element von einem Spleißdonor und einem Spleißakzeptor flankiert ist, und wobei das Polynukleotid ferner einen R6K-gamma-Replikationsursprung mit einer Sequenzidentität von wenigstens 95% mit einer Sequenz ausgewählt aus der Gruppe bestehend aus SEQ ID NO: 1, SEQ ID NO: 2, SEQ ID NO: 3 und SEQ ID NO: 4 umfasst.

10. Polynukleotid nach Anspruch 9, wobei der Promotor in einer Transkriptionseinheit zur Expression eines Cargo-Polypeptids und/oder eines selektierbaren Markers in einer Wirtszelle enthalten ist.

11. Polynukleotid gemäß Anspruch 10, wobei die Transkriptionseinheit einen Promotor, eine 5'-UTR, ein Transgen und eine 3'-UTR umfasst.

12. Polynukleotid nach Anspruch 11, wobei die S/MAR innerhalb der 3'-UTR liegt.

13. Kovalent geschlossenes zirkuläres rekombinantes DNA-Molekül, umfassend:
a. eine Transkriptionseinheit zur Expression eines Transgens in einer Vertebratenzelle, die einen Promotor, eine 5'-UTR, ein Transgen und eine 3'-UTR umfasst;
b. eine innerhalb der 3'-UTR liegende S/MAR, wobei die S/MAR von einer 5'-Spleißdonor-Stelle und einer 3'-Spleißakzeptor-Stelle flankiert ist;
c. einen R6K-gamma-Replikationsursprung mit einer Sequenzidentität von wenigstens 95% mit einer Sequenz ausgewählt aus der Gruppe bestehend aus SEQ ID NO: 1, SEQ ID NO: 2, SEQ ID NO: 3 und SEQ ID NO: 4; und
d. einen selektierbaren RNA-OUT-RNA-Marker, der eine RNA-IN regulierende RNA-OUT-Funktionsvariante mit einer Sequenzidentität von wenigstens 95% mit einer Sequenz ausgewählt aus der Gruppe bestehend aus SEQ ID NO: 5 und SEQ ID NO: 7 umfasst.

14. Polynukleotid nach einem der Ansprüche 9 bis 12 oder rekombinantes DNA-Molekül nach Anspruch 13, wobei die S/MAR ausgewählt ist aus der Gruppe bestehend aus menschlicher Interferon-beta-S/MAR, M18-S/MAR, Apolipoprotein-B-S/MAR.

15. Polynukleotid nach einem der Ansprüche 9 bis 12 oder rekombinantes DNA-Molekül nach Anspruch 13, wobei die von einer 5'-Spleißdonor-Stelle und einer 3'-Spleißakzeptor-Stelle flankierte SMAR eine Sequenzidentität von wenigstens 95% mit einer Sequenz ausgewählt aus der Gruppe bestehend aus SEQ ID NO: 19, SEQ ID NO: 20, SEQ ID NO: 21, SEQ ID NO: 22 und SEQ ID NO: 23 aufweist.

## Revendications

1. Procédé *in vitro* destiné à améliorer l'expression et l'efficacité d'implantation d'un vecteur d'expression à S/MAR épisomique auto-réplicable, non intégrant dans une cellule cible de vertébré, comprenant les étapes suivantes :
a. fournir un vecteur d'expression à S/MAR épisomique comprenant :
i. une région à sélection de réplication bactérienne comprenant une origine de réplication bactérienne et un marqueur sélectionnable ;
ii. une unité de transcription destinée à l'expression d'un transgène dans une cellule de vertébré, comprenant un promoteur, une UTR en 5', un transgène et une UTR en 3' ;
iii. un insert S/MAR situé au sein de ladite UTR en 3' ; et
b. modifier le vecteur d'expression à S/MAR épisomique de sorte que le S/MAR soit flanqué par un site donneur d'épissage en 5' et un site accepteur d'épissage en 3' au sein de ladite UTR en 3', moyennant quoi le vecteur d'expression à S/MAR épisomique auto-réplicable, non intégrant résultant a une expression et une efficacité d'implantation améliorées après une transfection d'une cellule de vertébré.

2. Procédé de la revendication 1, dans lequel ledit insert S/MAR contient des motifs internes de terminaison de la transcription AATAAA.

3. Procédé de la revendication 2, dans lequel lesdits motifs de terminaison de la transcription AATAAA dans ledit S/MAR sont remplacés par des motifs AATATT.

4. Procédé de la revendication 1, dans lequel ledit S/MAR est choisi dans le groupe constitué par un S/MAR de l'interféron bêta humain, un S/MAR du M18, un S/MAR de l'apolipoprotéine B.

5. Procédé de la revendication 1, dans lequel ledit SMAR, flanqué par un site donneur d'épissage en 5' et un site accepteur d'épissage en 3', a une identité de séquence d'au moins 95% avec une séquence choisie dans le groupe constitué par les SEQ ID n° : 19, SEQ ID n° : 20, SEQ ID n° : 21, SEQ ID n° : 22 et SEQ ID n° : 23.

6. Procédé de la revendication 1, dans lequel ladite origine de réplication bactérienne est une origine de réplication R6K gamma.

7. Procédé de la revendication 1, dans lequel ledit marqueur sélectionnable est un variant fonctionnel à "RNA-OUT" régulant le "RNA-IN", avec une identité de séquence d'au moins 95% avec une séquence choisie dans le groupe constitué par les SEQ ID n° : 5 et SEQ ID n° : 7.

8. Procédé de la revendication 1, dans lequel ledit vecteur d'expression à S/MAR épisomique auto-réplicable, non intégrant est choisi dans le groupe constitué par un vecteur plasmidique, un vecteur nano-plasmidique, un vecteur lentiviral à intégration défectueuse et des vecteurs lentiviraux non intégrants.

9. Polynucléotide comprenant au moins un promoteur et un élément S/MAR, dans lequel ledit élément S/MAR est situé en aval dudit promoteur et dans lequel la séquence d'acides nucléiques dudit élément S/MAR (séquence S/MAR) comprend au moins 3 motifs à séquence ATTA par 100 nucléotides sur un allongement d'au plus 200 nucléotides, dans lequel ledit élément S/MAR est flanqué par un donneur d'épissage et un accepteur d'épissage, et ledit polynucléotide comprenant en outre une origine de réplication R6K gamma avec une identité de séquence d'au moins 95% avec une séquence choisie dans le groupe constitué par les SEQ ID n° : 1, SEQ ID n° : 2, SEQ ID n° : 3, et SEQ ID n° : 4.

10. Polynucléotide de la revendication 9, dans lequel ledit promoteur est compris dans une unité de transcription destinée à l'expression d'un polypeptide cargo et/ou d'un marqueur sélectionnable dans une cellule hôte.

11. Polynucléotide selon la revendication 10, dans lequel ladite unité de transcription comprend un promoteur, une UTR en 5', un transgène et une UTR en 3'.

12. Polynucléotide de la revendication 11, dans lequel ledit S/MAR est situé au sein de ladite UTR en 3'.

13. Molécule d'ADN recombinante circulaire fermée par covalence comprenant :
a. une unité de transcription destinée à l'expression d'un transgène dans une cellule de vertébré, comprenant un promoteur, une UTR en 5', un transgène et une UTR en 3' ;
b. un S/MAR situé au sein de ladite UTR en 3', dans laquelle ledit S/MAR est flanqué par un site donneur d'épissage en 5' et un site accepteur d'épissage en 3';
c. une origine de réplication R6K gamma avec une identité de séquence d'au moins 95% avec une séquence choisie dans le groupe constitué par les SEQ ID n° : 1, SEQ ID n° : 2, SEQ ID n° : 3 et SEQ ID n° : 4 ; et
d. un marqueur sélectionnable d'ARN à "RNA-OUT" comprenant un variant fonctionnel à "RNA-OUT" régulant le "RNA-IN" avec une identité de séquence d'au moins 95% avec une séquence choisie dans le groupe constitué par les SEQ ID n° : 5 et SEQ ID n° : 7.

14. Polynucléotide de l'une quelconque des revendications 9 à 12 ou molécule d'ADN recombinante de la revendication 13, dans lequel/laquelle ledit S/MAR est choisi dans le groupe constitué par un S/MAR de l'interféron bêta humain, un S/MAR du M18, un S/MAR de l'apolipoprotéine B.

15. Polynucléotide de l'une quelconque des revendications 9 à 12 ou molécule d'ADN recombinante de la revendication 13, dans lequel/laquelle ledit S/MAR flanqué par un site donneur d'épissage en 5' et un site accepteur d'épissage en 3' a une identité de séquence d'au moins 95% avec une séquence choisie dans le groupe constitué par les SEQ ID n° : 19, SEQ ID n° : 20, SEQ ID n° : 21, SEQ ID n° : 22 et SEQ ID n° : 23.


### SEQUENCE LISTING

<110> Deutsches Krebsforschungszentrum
   Nature Technology Coropration
   Bozza, Matthias
   Harbottle, Richard
   Williams, James
<120> NON-INTEGRATING DNA VECTORS FOR THE GENETIC MODIFICATION OF CELLS
<130> DK14791PC1
<160> 41
<170> BiSSAP 1.3.6
<210> 1
   <211> 281
   <212> DNA
   <213> Artificial Sequence
<220>
   <223> R6K gamma origin
<400> 1
   ggcttgttgt ccacaaccgt taaaccttaa aagctttaaa agccttatat attctttttt 60
   ttcttataaa acttaaaacc ttagaggcta tttaagttgc tgatttatat taattttatt 120
   gttcaaacat gagagcttag tacgtgaaac atgagagctt agtacgttag ccatgagagc 180
   ttagtacgtt agccatgagg gtttagttcg ttaaacatga gagcttagta cgttaaacat 240
   gagagcttag tacgtactat caacaggttg aactgctgat c 281
<210> 2
   <211> 281
   <212> DNA
   <213> Artificial Sequence
<220>
   <223> 1 CpG R6K gamma origin
<400> 2
   ggcttgttgt ccacaaccat taaaccttaa aagctttaaa agccttatat attctttttt 60
   ttcttataaa acttaaaacc ttagaggcta tttaagttgc tgatttatat taattttatt 120
   gttcaaacat gagagcttag tacgtgaaac atgagagctt agtacattag ccatgagagc 180
   ttagtacatt agccatgagg gtttagttca ttaaacatga gagcttagta cattaaacat 240
   gagagcttag tacatactat caacaggttg aactgctgat c 281
<210> 3
   <211> 260
   <212> DNA
   <213> Artificial Sequence
<220>
   <223> CpG free R6K gamma origin
<400> 3
   aaaccttaaa acctttaaaa gccttatata ttcttttttt tcttataaaa cttaaaacct 60
   tagaggctat ttaagttgct gatttatatt aattttattg ttcaaacatg agagcttagt 120
   acatgaaaca tgagagctta gtacattagc catgagagct tagtacatta gccatgaggg 180
   tttagttcat taaacatgag agcttagtac attaaacatg agagcttagt acatactatc 240
   aacaggttga actgctgatc 260
<210> 4
   <211> 389
   <212> DNA
   <213> Artificial Sequence
<220>
   <223> Extended R6K gamma origin
<400> 4
   tgtcagccgt taagtgttcc tgtgtcactg aaaattgctt tgagaggctc taagggcttc 60
   tcagtgcgtt acatccctgg cttgttgtcc acaaccgtta aaccttaaaa gctttaaaag 120
   ccttatatat tctttttttt cttataaaac ttaaaacctt agaggctatt taagttgctg 180
   atttatatta attttattgt tcaaacatga gagcttagta cgtgaaacat gagagcttag 240
   tacgttagcc atgagagctt agtacgttag ccatgagggt ttagttcgtt aaacatgaga 300
   gcttagtacg ttaaacatga gagcttagta cgtgaaacat gagagcttag tacgtactat 360
   caacaggttg aactgctgat cttcagatc 389
<210> 5
   <211> 139
   <212> DNA
   <213> Artificial Sequence
<220>
   <223> RNA-OUT selectable marker
<400> 5
   gtagaattgg taaagagagt cgtgtaaaat atcgagttcg cacatcttgt tgtctgatta 60
   ttgatttttg gcgaaaccat ttgatcatat gacaagatgt gtatctacct taacttaatg 120
   attttgataa aaatcatta 139
<210> 6
   <211> 69
   <212> DNA
   <213> Artificial Sequence
<220>
   <223> RNA-OUT antisense repressor RNA
<400> 6
   tcgcacatct tgttgtctga ttattgattt ttggcgaaac catttgatca tatgacaaga      60
   tgtgtatct                                                              69
<210> 7
   <211> 139
   <212> DNA
   <213> Artificial Sequence
<220>
   <223> 2 CpG RNA-OUT selectable marker
<400> 7
   gtagaattgg taaagagagt tgtgtaaaat attgagttcg cacatcttgt tgtctgatta      60
   ttgatttttg gcgaaaccat ttgatcatat gacaagatgt gtatctacct taacttaatg     120
   attttgataa aaatcatta                                                  139
<210> 8
   <211> 466
   <212> DNA
   <213> Artificial Sequence
<220>
   <223> R6K gamma origin -RNA-OUT bacterial replication-selection region flanked by NheI and KpnI restriction sites
<400> 8
   gctagcccgc ctaatgagcg ggcttttttt tggcttgttg tccacaaccg ttaaacctta      60
   aaagctttaa aagccttata tattcttttt tttcttataa aacttaaaac cttagaggct     120
   atttaagttg ctgatttata ttaattttat tgttcaaaca tgagagctta gtacgtgaaa     180
   catgagagct tagtacgtta gccatgagag cttagtacgt tagccatgag ggtttagttc     240
   gttaaacatg agagcttagt acgttaaaca tgagagctta gtacgtacta tcaacaggtt     300
   gaactgctga tccacgttgt ggtagaattg gtaaagagag tcgtgtaaaa tatcgagttc     360
   gcacatcttg ttgtctgatt attgattttt ggcgaaacca tttgatcata tgacaagatg     420
   tgtatctacc ttaacttaat gattttgata aaaatcatta ggtacc                    466
<210> 9
   <211> 439
   <212> DNA
   <213> Artificial Sequence
<220>
   <223> 1 CpG R6K gamma origin - 2 CpG RNA-OUT bacterial replication-selection region flanked by NheI and KpnI restriction sites
<400> 9
   gctagctggc ttgttgtcca caaccattaa accttaaaag ctttaaaagc cttatatatt      60
   cttttttttc ttataaaact taaaacctta gaggctattt aagttgctga tttatattaa     120
   ttttattgtt caaacatgag agcttagtac gtgaaacatg agagcttagt acattagcca     180
   tgagagctta gtacattagc catgagggtt tagttcatta aacatgagag cttagtacat     240
   taaacatgag agcttagtac atactatcaa caggttgaac tgctgatctg tacagtagaa     300
   ttggtaaaga gagttgtgta aaatattgag ttcgcacatc ttgttgtctg attattgatt     360
   tttggcgaaa ccatttgatc atatgacaag atgtgtatct accttaactt aatgattttg     420
   ataaaaatca ttaggtacc                                                  439
<210> 10
   <211> 565
   <212> DNA
   <213> Artificial Sequence
<220>
   <223> pNTC-NP1 polylinker trpA R6K-RNA-OUT polylinker cloning cassette: EcoRI-HindIII
<400> 10
   gaattcgagc tcggtacctc gcgaatgcat ctaggggacg gccgctagcc cgcctaatga      60
   gcgggctttt ttttggcttg ttgtccacaa ccgttaaacc ttaaaagctt taaaagcctt     120
   atatattctt ttttttctta taaaacttaa aaccttagag gctatttaag ttgctgattt     180
   atattaattt tattgttcaa acatgagagc ttagtacgtg aaacatgaga gcttagtacg     240
   ttagccatga gagcttagta cgttagccat gagggtttag ttcgttaaac atgagagctt     300
   agtacgttaa acatgagagc ttagtacgta ctatcaacag gttgaactgc tgatccacgt     360
   tgtggtagaa ttggtaaaga gagtcgtgta aaatatcgag ttcgcacatc ttgttgtctg     420
   attattgatt tttggcgaaa ccatttgatc atatgacaag atgtgtatct accttaactt     480
   aatgattttg ataaaaatca ttaggagcta gcattgggtc atcggatccc gggcccgtcg     540
   actgcagagg cctgcatgca agctt                                           565
<210> 11
   <211> 584
   <212> DNA
   <213> Artificial Sequence
<220>
   <223> pNTC-NP2 polylinker trpA R6K-RNA-OUT polylinker cloning cassette: EcoRI-HindIII
<400> 11
   gaattcgagc tcggtacctc gcgaatgcat ctaggggacg gccgctagcc cgcctaatga 60
   gcgggctttt ttttggcttg ttgtccacaa ccgttaaacc ttaaaagctt taaaagcctt 120
   atatattctt ttttttctta taaaacttaa aaccttagag gctatttaag ttgctgattt 180
   atattaattt tattgttcaa acatgagagc ttagtacgtg aaacatgaga gcttagtacg 240
   ttagccatga gagcttagta cgttagccat gagggtttag ttcgttaaac atgagagctt 300
   agtacgttaa acatgagagc ttagtacgta ctatcaacag gttgaactgc tgatccacgt 360
   tgtggtagaa ttggtaaaga gagtcgtgta aaatatcgag ttcgcacatc ttgttgtctg 420
   attattgatt tttggcgaaa ccatttgatc atatgacaag atgtgtatct accttaactt 480
   aatgattttg ataaaaatca ttaggactag tcccgggcgc tagttattaa tattgggtca 540
   tcggatcccg ggcccgtcga ctgcagaggc ctgcatgcaa gctt 584
<210> 12
   <211> 557
   <212> DNA
   <213> Artificial Sequence
<220>
   <223> pNTC-NP3 polylinker trpA R6K-RNA-OUT polylinker cloning cassette: EcoRI-HindIII
<400> 12
   gaattcgagc tcggtacctc gcgaatgcat ctaggggacg gccgctagcc cgcctaatga 60
   gcgggctttt ttttggcttg ttgtccacaa ccgttaaacc ttaaaagctt taaaagcctt 120
   atatattctt ttttttctta taaaacttaa aaccttagag gctatttaag ttgctgattt 180
   atattaattt tattgttcaa acatgagagc ttagtacgtg aaacatgaga gcttagtacg 240
   ttagccatga gagcttagta cgttagccat gagggtttag ttcgttaaac atgagagctt 300
   agtacgttaa acatgagagc ttagtacgta ctatcaacag gttgaactgc tgatccacgt 360
   tgtggtagaa ttggtaaaga gagtcgtgta aaatatcgag ttcgcacatc ttgttgtctg 420
   attattgatt tttggcgaaa ccatttgatc atatgacaag atgtgtatct accttaactt 480
   aatgattttg ataaaaatca ttaggtaccg agctcggatc ccgggcccgt cgactgcaga 540
   ggcctgcatg caagctt 557
<210> 13
   <211> 557
   <212> DNA
   <213> Artificial Sequence
<220>
   <223> pNTC-NP4 polylinker trpA R6K-RNA-OUT polylinker cloning cassette: HindIII-EcoRI
<400> 13
   aagcttgcat gcaggcctct gcagtcgacg ggcccgggat ccgagctcgg tacctcgcga 60
   atgcatctag gggacggccg ctagcccgcc taatgagcgg gctttttttt ggcttgttgt 120
   ccacaaccgt taaaccttaa aagctttaaa agccttatat attctttttt ttcttataaa 180
   acttaaaacc ttagaggcta tttaagttgc tgatttatat taattttatt gttcaaacat 240
   gagagcttag tacgtgaaac atgagagctt agtacgttag ccatgagagc ttagtacgtt 300
   agccatgagg gtttagttcg ttaaacatga gagcttagta cgttaaacat gagagcttag 360
   tacgtactat caacaggttg aactgctgat ccacgttgtg gtagaattgg taaagagagt 420
   cgtgtaaaat atcgagttcg cacatcttgt tgtctgatta ttgatttttg gcgaaaccat 480
   ttgatcatat gacaagatgt gtatctacct taacttaatg attttgataa aaatcattag 540
   gtaccgagct cgaattc 557
<210> 14
   <211> 503
   <212> DNA
   <213> Artificial Sequence
<220>
   <223> pNTC-NP5 polylinker trpA R6K-RNA-OUT polylinker cloning cassette: KasI-HindIII
<400> 14
   ggcgccgcta gcccgcctaa tgagcgggct tttttttggc ttgttgtcca caaccgttaa 60
   accttaaaag ctttaaaagc cttatatatt cttttttttc ttataaaact taaaacctta 120
   gaggctattt aagttgctga tttatattaa ttttattgtt caaacatgag agcttagtac 180
   gtgaaacatg agagcttagt acgttagcca tgagagctta gtacgttagc catgagggtt 240
   tagttcgtta aacatgagag cttagtacgt taaacatgag agcttagtac gtactatcaa 300
   caggttgaac tgctgatcca cgttgtggta gaattggtaa agagagtcgt gtaaaatatc 360
   gagttcgcac atcttgttgt ctgattattg atttttggcg aaaccatttg atcatatgac 420
   aagatgtgta tctaccttaa cttaatgatt ttgataaaaa tcattaggta ccacatgtcc 480
   tgcagaggcc tgcatgcaag ctt 503
<210> 15
   <211> 539
   <212> DNA
   <213> Artificial Sequence
<220>
   <223> pNTC-NP6 polylinker trpA R6K-RNA-OUT polylinker cloning cassette: EcoRI-SacI
<400> 15
   gaattctgca gatatccatc acactggcgg ccgctagccc gcctaatgag cgggcttttt 60
   tttggcttgt tgtccacaac cgttaaacct taaaagcttt aaaagcctta tatattcttt 120
   tttttcttat aaaacttaaa accttagagg ctatttaagt tgctgattta tattaatttt 180
   attgttcaaa catgagagct tagtacgtga aacatgagag cttagtacgt tagccatgag 240
   agcttagtac gttagccatg agggtttagt tcgttaaaca tgagagctta gtacgttaaa 300
   catgagagct tagtacgtac tatcaacagg ttgaactgct gatccacgtt gtggtagaat 360
   tggtaaagag agtcgtgtaa aatatcgagt tcgcacatct tgttgtctga ttattgattt 420
   ttggcgaaac catttgatca tatgacaaga tgtgtatcta ccttaactta atgattttga 480
   taaaaatcat taggtaccgg gccccccctc gatcgaggtc gacggtatcg ggggagctc 539
<210> 16
   <211> 500
   <212> DNA
   <213> Artificial Sequence
<220>
   <223> pNTC-NP7 polylinker trpA R6K-RNA-OUT polylinker cloning cassette: BssHII-BssHII
<400> 16
   gcgcgcagcc ttaattaagc tagcccgcct aatgagcggg cttttttttg gcttgttgtc 60
   cacaaccgtt aaaccttaaa agctttaaaa gccttatata ttcttttttt tcttataaaa 120
   cttaaaacct tagaggctat ttaagttgct gatttatatt aattttattg ttcaaacatg 180
   agagcttagt acgtgaaaca tgagagctta gtacgttagc catgagagct tagtacgtta 240
   gccatgaggg tttagttcgt taaacatgag agcttagtac gttaaacatg agagcttagt 300
   acgtactatc aacaggttga actgctgatc cacgttgtgg tagaattggt aaagagagtc 360
   gtgtaaaata tcgagttcgc acatcttgtt gtctgattat tgatttttgg cgaaaccatt 420
   tgatcatatg acaagatgtg tatctacctt aacttaatga ttttgataaa aatcattagg 480
   taccttaatt aactgcgcgc 500
<210> 17
   <211> 530
   <212> DNA
   <213> Artificial Sequence
<220>
   <223> pNTC-3xCpG NP1 polylinker R6K-RNA-OUT polylinker cloning cassette: HindIII-EcoRI
<400> 17
   aagcttgcat gcaggcctct gcagtcgacg ggccctctag actcgagctg gcttgttgtc 60
   cacaaccatt aaaccttaaa agctttaaaa gccttatata ttcttttttt tcttataaaa     120
   cttaaaacct tagaggctat ttaagttgct gatttatatt aattttattg ttcaaacatg     180
   agagcttagt acgtgaaaca tgagagctta gtacattagc catgagagct tagtacatta     240
   gccatgaggg tttagttcat taaacatgag agcttagtac attaaacatg agagcttagt     300
   acatactatc aacaggttga actgctgatc tgtacagtag aattggtaaa gagagttgtg     360
   taaaatattg agttcgcaca tcttgttgtc tgattattga tttttggcga aaccatttga     420
   tcatatgaca agatgtgtat ctaccttaac ttaatgattt tgataaaaat cattaggtac     480
   cgctagcggc cgtcccctag atgcattcgc gaggtaccga gctcgaattc                530
<210> 18
   <211> 1973
   <212> DNA
   <213> Artificial Sequence
<220>
   <223> human Interferon beta S/MAR flanked by 5' BglII-XhoI site and 3' EcoRI restriction enzyme sites
<400> 18
   agatctcgag ctcgatgata atgaatgtct aagttaatgc agaaacggag agacatacta 60
   tattcatgaa ctaaaagact taatattgtg aaggtatact ttctttccac ataaatttgt 120
   agtcaatatg ttcaccccaa aaaagctgtt tgttaacttg ccaacctcat tctaaaatgt 180
   atatagaagc ccaaaagaca ataacaaaaa tattcttgta gaacaaaatg ggaaagaatg 240
   ttccactaaa tatcaagatt tagagcaaag catgagatgt gtggggatag acagtgaggc 300
   tgataaaata gagtagagct cagaaacaga cccattgata tatgtaagtg acctatgaaa 360
   aaaatatggc attttacaat gggaaaatga tgatcttttt cttttttaga aaaacaggga 420
   aatatattta tatgtaaaaa ataaaaggga acccatatgt cataccatac acacaaaaaa 480
   attccagtga attataagtc taaatggaga aggcaaaact ttaaatcttt tagaaaataa 540
   tatagaagca tgccatcatg acttcagtgt agagaaaaat ttcttatgac tcaaagtcct 600
   aaccacaaag aaaagattgt taattagatt gcatgaatat taagacttat ttttaaaatt 660
   aaaaaaccat taagaaaagt caggccatag aatgacagaa aatatttgca acaccccagt 720
   aaagagaatt gtaatatgca gattataaaa agaagtctta caaatcagta aaaaataaaa 780
   ctagacaaaa atttgaacag atgaaagaga aactctaaat aatcattaca catgagaaac 840
   tcaatctcag aaatcagaga actatcattg catatacact aaattagaga aatattaaaa 900
   ggctaagtaa catctgtggc aatattgatg gtatataacc ttgatatgat gtgatgagaa 960
   cagtacttta ccccatgggc ttcctcccca aacccttacc ccagtataaa tcatgacaaa 1020
   tatactttaa aaaccattac cctatatcta accagtactc ctcaaaactg tcaaggtcat 1080
   caaaaataag aaaagtctga ggaactgtca aaactaagag gaacccaagg agacatgaga    1140
   attatatgta atgtggcatt ctgaatgaga tcccagaaca gaaaaagaac agtagctaaa    1200
   aaactaatga aatataaata aagtttgaac tttagttttt tttaaaaaag agtagcatta    1260
   acacggcaaa gccattttca tatttttctt gaacattaag tacaagtcta taattaaaaa    1320
   ttttttaaat gtagtctgga acattgccag aaacagaagt acagcagcta tctgtgctgt    1380
   cgcctaacta tccatagctg attggtctaa aatgagatac atcaacgctc ctccatgttt    1440
   tttgttttct ttttaaatga aaaactttat tttttaagag gagtttcagg ttcatagcaa    1500
   aattgagagg aaggtacatt caagctgagg aagttttcct ctattcctag tttactgaga    1560
   gattgcatca tgaatgggtg ttaaattttg tcaaatgctt tttctgtgtc tatcaatatg    1620
   accatgtgat tttcttcttt aacctgttga tgggacaaat tacgttaatt gattttcaaa    1680
   cgttgaacca cccttacata tctggaataa attctacttg gttgtggtgt atattttttg    1740
   atacattctt ggattctttt tgctaatatt ttgttgaaaa tgtttgtatc tttgttcatg    1800
   agagatattg gtctgttgtt ttcttttctt gtaatgtcat tttctagttc cggtattaag    1860
   gtaatgctgg cctagttgaa tgatttagga agtattccct ctgcttctgt cttctgaaag    1920
   agattgtaga aagttgatac aatttttttt tctttaaata tttgatagaa ttc          1973
<210> 19
   <211> 2085
   <212> DNA
   <213> Artificial Sequence
<220>
   <223> Splice donor-human Interferon beta S/MAR-splice acceptor flanked by 5' BglII site and 3' BamHI restriction enzyme sites
<400> 19
   agatctatgc atgcagaagt tggtcgtgag gcactgggca ggtaagtatc aaggttacaa      60
   gacaggtctc gagctcgatg ataatgaatg tctaagttaa tgcagaaacg gagagacata     120
   ctatattcat gaactaaaag acttaatatt gtgaaggtat actttctttc cacataaatt     180
   tgtagtcaat atgttcaccc caaaaaagct gtttgttaac ttgccaacct cattctaaaa     240
   tgtatataga agcccaaaag acaataacaa aaatattctt gtagaacaaa atgggaaaga     300
   atgttccact aaatatcaag atttagagca aagcatgaga tgtgtgggga tagacagtga     360
   ggctgataaa atagagtaga gctcagaaac agacccattg atatatgtaa gtgacctatg     420
   aaaaaaatat ggcattttac aatgggaaaa tgatgatctt tttctttttt agaaaaacag     480
   ggaaatatat ttatatgtaa aaaataaaag ggaacccata tgtcatacca tacacacaaa     540
   aaaattccag tgaattataa gtctaaatgg agaaggcaaa actttaaatc ttttagaaaa     600
   taatatagaa gcatgccatc atgacttcag tgtagagaaa aatttcttat gactcaaagt 660
   cctaaccaca aagaaaagat tgttaattag attgcatgaa tattaagact tatttttaaa 720
   attaaaaaac cattaagaaa agtcaggcca tagaatgaca gaaaatattt gcaacacccc 780
   agtaaagaga attgtaatat gcagattata aaaagaagtc ttacaaatca gtaaaaaata 840
   aaactagaca aaaatttgaa cagatgaaag agaaactcta aataatcatt acacatgaga 900
   aactcaatct cagaaatcag agaactatca ttgcatatac actaaattag agaaatatta 960
   aaaggctaag taacatctgt ggcaatattg atggtatata accttgatat gatgtgatga 1020
   gaacagtact ttaccccatg ggcttcctcc ccaaaccctt accccagtat aaatcatgac 1080
   aaatatactt taaaaaccat taccctatat ctaaccagta ctcctcaaaa ctgtcaaggt 1140
   catcaaaaat aagaaaagtc tgaggaactg tcaaaactaa gaggaaccca aggagacatg 1200
   agaattatat gtaatgtggc attctgaatg agatcccaga acagaaaaag aacagtagct 1260
   aaaaaactaa tgaaatataa ataaagtttg aactttagtt ttttttaaaa aagagtagca 1320
   ttaacacggc aaagccattt tcatattttt cttgaacatt aagtacaagt ctataattaa 1380
   aaatttttta aatgtagtct ggaacattgc cagaaacaga agtacagcag ctatctgtgc 1440
   tgtcgcctaa ctatccatag ctgattggtc taaaatgaga tacatcaacg ctcctccatg 1500
   ttttttgttt tctttttaaa tgaaaaactt tattttttaa gaggagtttc aggttcatag 1560
   caaaattgag aggaaggtac attcaagctg aggaagtttt cctctattcc tagtttactg 1620
   agagattgca tcatgaatgg gtgttaaatt ttgtcaaatg ctttttctgt gtctatcaat 1680
   atgaccatgt gattttcttc tttaacctgt tgatgggaca aattacgtta attgattttc 1740
   aaacgttgaa ccacccttac atatctggaa taaattctac ttggttgtgg tgtatatttt 1800
   ttgatacatt cttggattct ttttgctaat attttgttga aaatgtttgt atctttgttc 1860
   atgagagata ttggtctgtt gttttctttt cttgtaatgt cattttctag ttccggtatt 1920
   aaggtaatgc tggcctagtt gaatgattta ggaagtattc cctctgcttc tgtcttctga 1980
   aagagattgt agaaagttga tacaattttt ttttctttaa atatttgata gaattcttac 2040
   tgacatccac tttgcctttc tctccacagg tgtccactcg gatcc 2085
<210> 20
   <211> 2085
   <212> DNA
   <213> Artificial Sequence
<220>
   <223> Splice donor-human Interferon beta S/MAR (-AATAAA)-splice acceptor flanked by 5' BglII site and 3' BamHI restriction enzyme sites
<400> 20
   agatctatgc atgcagaagt tggtcgtgag gcactgggca ggtaagtatc aaggttacaa      60
   gacaggtctc gagctcgatg ataatgaatg tctaagttaa tgcagaaacg gagagacata     120
   ctatattcat gaactaaaag acttaatatt gtgaaggtat actttctttc cacataaatt     180
   tgtagtcaat atgttcaccc caaaaaagct gtttgttaac ttgccaacct cattctaaaa     240
   tgtatataga agcccaaaag acaataacaa aaatattctt gtagaacaaa atgggaaaga     300
   atgttccact aaatatcaag atttagagca aagcatgaga tgtgtgggga tagacagtga     360
   ggctgataaa atagagtaga gctcagaaac agacccattg atatatgtaa gtgacctatg     420
   aaaaaaatat ggcattttac aatgggaaaa tgatgatctt tttctttttt agaaaaacag     480
   ggaaatatat ttatatgtaa aaaatatttg ggaacccata tgtcatacca tacacacaaa     540
   aaaattccag tgaattataa gtctaaatgg agaaggcaaa actttaaatc ttttagaaaa     600
   taatatagaa gcatgccatc atgacttcag tgtagagaaa aatttcttat gactcaaagt     660
   cctaaccaca aagaaaagat tgttaattag attgcatgaa tattaagact tatttttaaa     720
   attaaaaaac cattaagaaa agtcaggcca tagaatgaca gaaaatattt gcaacacccc     780
   agtaaagaga attgtaatat gcagattata aaaagaagtc ttacaaatca gtaaaaaata     840
   tttctagaca aaaatttgaa cagatgaaag agaaactcta aataatcatt acacatgaga     900
   aactcaatct cagaaatcag agaactatca ttgcatatac actaaattag agaaatatta     960
   aaaggctaag taacatctgt ggcaatattg atggtatata accttgatat gatgtgatga    1020
   gaacagtact ttaccccatg ggcttcctcc ccaaaccctt accccagtat aaatcatgac    1080
   aaatatactt taaaaaccat taccctatat ctaaccagta ctcctcaaaa ctgtcaaggt    1140
   catcaaaaat aagaaaagtc tgaggaactg tcaaaactaa gaggaaccca aggagacatg    1200
   agaattatat gtaatgtggc attctgaatg agatcccaga acagaaaaag aacagtagct    1260
   aaaaaactaa tgaaatataa atattttttg aactttagtt ttttttaaaa aagagtagca    1320
   ttaacacggc aaagccattt tcatattttt cttgaacatt aagtacaagt ctataattaa    1380
   aaatttttta aatgtagtct ggaacattgc cagaaacaga agtacagcag ctatctgtgc    1440
   tgtcgcctaa ctatccatag ctgattggtc taaaatgaga tacatcaacg ctcctccatg    1500
   ttttttgttt tctttttaaa tgaaaaactt tattttttaa gaggagtttc aggttcatag     1560
   caaaattgag aggaaggtac attcaagctg aggaagtttt cctctattcc tagtttactg    1620
   agagattgca tcatgaatgg gtgttaaatt ttgtcaaatg ctttttctgt gtctatcaat    1680
   atgaccatgt gattttcttc tttaacctgt tgatgggaca aattacgtta attgattttc     1740
   aaacgttgaa ccacccttac atatctggaa tattttctac ttggttgtgg tgtatatttt    1800
   ttgatacatt cttggattct ttttgctaat attttgttga aaatgtttgt atctttgttc    1860
   atgagagata ttggtctgtt gttttctttt cttgtaatgt cattttctag ttccggtatt    1920
   aaggtaatgc tggcctagtt gaatgattta ggaagtattc cctctgcttc tgtcttctga 1980
   aagagattgt agaaagttga tacaattttt ttttctttaa atatttgata gaattcttac 2040
   tgacatccac tttgcctttc tctccacagg tgtccactcg gatcc 2085
<210> 21
   <211> 868
   <212> DNA
   <213> Artificial Sequence
<220>
   <223> Splice donor-human Interferon beta M18 S/MAR-splice acceptor flanked by 5' BglII site and 3' BamHI restriction enzyme sites
<400> 21
   agatctatgc atgcagaagt tggtcgtgag gcactgggca ggtaagtatc aaggttacaa 60
   gacaggtctc gagaagcttg atctaaataa acttataaat tgtgagagaa attaatgaat 120
   gtctaagtta atgcagaaac ggagagacat actatattca tgaactaaaa gacttaatat 180
   tgtgaaggta tactttcttt ccacataaat ttgtagtcaa tatgttcacc ccaaaaaagc 240
   tgtttgttaa cttgccaacc tcattctaaa atgtatatag aagcccaaaa gacaataaca 300
   aaaatattct tgtagaacaa aatgggaaag aatgttccac taaatatcaa gatttagagc 360
   aaagcatgag atgtgtgggg atagacagtg aggctgataa aatagagtag agctcagaaa 420
   cagacccatt gatatatgta agtgacctat gaaaaaaata tggcatttta caatgggaaa 480
   atgatgatct ttttcttttt tagaaaaaca gggaaatata tttatatgta aaaaataaaa 540
   gggaacccat atgtcatacc atacacacaa aaaaattcca gtgaattata agtctaaatg 600
   gagaaggcaa aactttaaat cttttagaaa ataatataga agcatgccat catgacttca 660
   gtgtagagaa aaatttctta tgactcaaag tcctaaccac aaagaaaaga ttgttaatta 720
   tgaatgattt aggaagtatt ccctctgctt ctgtcttctg aaagagattg tagaaagttg 780
   atacaatttt tttttcttta aatatttgat agagaattct tactgacatc cactttgcct 840
   ttctctccac aggtgtccac tcggatcc 868
<210> 22
   <211> 927
   <212> DNA
   <213> Artificial Sequence
<220>
   <223> Splice donor-805 bp human Apolipoprotein B S/MAR-splice acceptor flanked by 5' BglII site and 3' BamHI restriction enzyme sites
<400> 22
   agatctatgc atgcagaagt tggtcgtgag gcactgggca ggtaagtatc aaggttacaa      60
   gacaggtctc gaggcctacc ggagagcctt gccttgcaaa ggcagacagt cagtgaggaa     120
   gactatgtgg cacatgaaga caccagaggt gttcctcagg atcaaagtat gtacaagcct     180
   ttgtgaatat tttttccttc tcacttggca aatacaattc ctgagatcaa taacctcgtc     240
   tttttaattt tttcctcgtc tttttaacta tttataaaat attgaattat aaaatatgta     300
   attataaata ctttaattat aaaatatgta attataaata ctttaattat aaaatatgta     360
   attataaata ctttataaaa tatgtaatta taaaatatgt aattataaac attttaatta     420
   taaaatatgt aattataaac attttaatta taaaatatgt aattataaac attttaatta     480
   taaaatatgt aattataaac attttaatta taaaatatgt aattataaac attttaatta     540
   taaaatattt aattataaac attttaatta taaaatattt aattataaat attttaatta     600
   taaaatattt aattataaat attttaatta taaaatattt aattataaat attttaatta     660
   taaaatattt aattataaat actttaatta taaaatattt aattataaat attttaatta     720
   taaaatattt aattataaat attttaatta taaatatttt aattataaaa tatttaatta     780
   taaaaacaca attacctcat ctttttaaat atttttgcaa aatatttccc tccataattt     840
   ctccgtttcc atttttattc tgttacttaa atgaattctt actgacatcc actttgcctt     900
   tctctccaca ggtgtccact cggatcc                                         927
<210> 23
   <211> 647
   <212> DNA
   <213> Artificial Sequence
<220>
   <223> Splice donor-525 bp human Apolipoprotein B S/MAR-splice acceptor flanked by 5' NsiI site and 3' BamHI restriction enzyme sites
<400> 23
   atgcatgcag aagttggtcg tgaggcactg ggcaggtaag tatcaaggtt acaagacagg      60
   tctcgagatt tataaaatat tgaattataa aatatgtaat tataaatact ttaattataa     120
   aatatgtaat tataaatact ttaattataa aatatgtaat tataaatact ttataaaata     180
   tgtaattata aaatatgtaa ttataaacat tttaattata aaatatgtaa ttataaacat     240
   tttaattata aaatatgtaa ttataaacat tttaattata aaatatgtaa ttataaacat     300
   tttaattata aaatatgtaa ttataaacat tttaattata aaatatttaa ttataaacat     360
   tttaattata aaatatttaa ttataaatat tttaattata aaatatttaa ttataaatat     420
   tttaattata aaatatttaa ttataaatat tttaattata aaatatttaa ttataaatac     480
   tttaattata aaatatttaa ttataaatat tttaattata aaatatttaa ttataaatat     540
   tttaattata aatattttaa ttataaaata tttaattata aaaacacaat tagaattctt     600
   actgacatcc actttgcctt tctctccaca ggtgtccact cggatcc                   647
<210> 24
   <211> 197
   <212> DNA
   <213> Artificial Sequence
<220>
   <223> pCI intron
<400> 24
   gcagaagttg gtcgtgaggc actgggcagg taagtatcaa ggttacaaga caggtttaag      60
   gagaccaata gaaactgggc ttgtcgagac agagaagact cttgcgtttc tgataggcac     120
   ctattggtct tactgacatc cactttgcct ttctctccac aggtgtccac tcccagttca     180
   attacagctc ttaaggc                                                    197
<210> 25
   <211> 42
   <212> DNA
   <213> Artificial Sequence
<220>
   <223> pCI Splice donor
<400> 25
   gcagaagttg gtcgtgaggc actgggcagg taagtatcaa gg 42
<210> 26
   <211> 44
   <212> DNA
   <213> Artificial Sequence
<220>
   <223> pCI Splice acceptor (murine IgG)
<400> 26
   cttactgaca tccactttgc ctttctctcc acaggtgtcc actc 44
<210> 27
   <211> 1025
   <212> DNA
   <213> Artificial Sequence
<220>
   <223> Ele40 expression enhancer
<400> 27
   gatcaagaaa gcactccggg ctccagaagg agccttccag gccagctttg agcataagct 60
   gctgatgagc agtgagtgtc ttgagtagtg ttcagggcag catgttacca ttcatgcttg 120
   acttctagcc agtgtgatga gaggctggag tcaggtctct agagagttga gcagctccag 180
   ccttagatct cccagtctta tgcggtgtgc ccattcgctt tgtgtctgca gtcccctggc 240
   cacacccagt aacagttctg ggatctatgg gagtagcttc cttagtgagc tttcccttca 300
   aatactttgc aaccaggtag agaagtttgg agtgaaggtt ttgttctttg tttcttcaca 360
   atatggatat gcatcttctt ttgaaaatgt taaagtaaat tacctctctt ttcagatact 420
   gtcttcatgc gaacttggta tcctgtttcc atcccagcct tctataaccc agtaacatct 480
   tttttgaaac cagtgggtga gaaagacacc tggtcaggaa cgcggaccac aggacaactc 540
   aggctcaccc acggcatcag actaaaggca aacaaggact ctgtataaag taccggtggc 600
   atgtgtatta gtggagatgc agcctgtgct ctgcagacag ggagtcacac agacactttt 660
   ctataatttc ttaagtgctt tgaatgttca agtagaaagt ctaacattaa atttgattga 720
   acaattgtat attcatggaa tattttggaa cggaatacca aaaaatggca atagtggttc 780
   tttctggatg gaagacaaac ttttcttttt aaattttatc ttatatattt gaggttgacc 840
   acatgacctt aaggatacat atagacagta aactggttac tacagtgaag caaattaaca 900
   tatctaccat cttacatagt tacatttttt tgtgtgacag gaacagctaa aatctacgta 960
   tttaacaaaa atcctaaaga caatacattt ttattaacta tagccctcat gatgtacatt 1020
   agatc 1025
<210> 28
   <211> 1503
   <212> DNA
   <213> Artificial Sequence
<220>
   <223> A2UCOE expression enhancer
<400> 28
   gcctacagct caagccacat ccgaaggggg agggagccgg gagctgcgcg cggggccgcc 60
   ggggggaggg gtggcaccgc ccacgccggg cggccacgaa gggcggggca gcgggcgcgc 120
   gcgcggcggg gggaggggcc ggcgccgcgc ccgctgggaa ttggggccct agggggaggg 180
   cggaggcgcc gacgaccgcg gcacttaccg ttcgcggcgt ggcgcccggt ggtccccaag 240
   gggagggaag ggggaggcgg ggcgaggaca gtgaccggag tctcctcagc ggtggctttt 300
   ctgcttggca gcctcagcgg ctggcgccaa aaccggactc cgcccacttc ctcgcccgcc 360
   ggtgcgaggg tgtggaatcc tccagacgct gggggagggg gagttgggag cttaaaaact 420
   agtacccctt tgggaccact ttcagcagcg aactctcctg tacaccaggg gtcagttcca 480
   cagacgcggg ccaggggtgg gtcattgcgg cgtgaacaat aatttgacta gaagttgatt 540
   cgggtgtttc cggaaggggc cgagtcaatc cgccgagttg gggcacggaa aacaaaaagg 600
   gaaggctact aagatttttc tggcgggggt tatcattggc gtaactgcag ggaccacctc 660
   ccgggttgag ggggctggat ctccaggctg cggattaagc ccctcccgtc ggcgttaatt 720
   tcaaactgcg cgaccgtttc tcacctgcct tgcgccaagg cagggggcgg gaccctattc 780
   caagaggtag taactagcag gactctagcc ttccgcaatt cattgagcgc atttacggaa 840
   gtaacgtcgg gtactgtctc tggccgcaag ggtgggagga gtacgcattt ggcgtaaggt 900
   ggggcgtaga gccttcccgc cattggcggc ggatagggcg tttacgcgac ggcctgacgt 960
   agcggaagac gcgttagtgg gggggaaggt tctagaaaag cggcggcagc ggctctagcg 1020
   gcagtagcag cagcgccggg tcccgtgcgg aggtgctcct cgcagagttg tttctcgagc 1080
   agcggcagtt ctcactacag cgccaggacg agtccggttc gtgttcgtcc gcggagatct 1140
   ctctcatctc gctcggctgc gggaaatcgg gctgaagcga ctgagtccgc gatggaggta 1200
   acgggtttga aatcaatgag ttattgaaaa gggcatggcg aggccgttgg cgcctcagtg 1260
   gaagtcggcc agccgcctcc gtgggagaga ggcaggaaat cggaccaatt cagtagcagt 1320
   ggggcttaag gtttatgaac ggggtcttga gcggaggcct gagcgtacaa acagcttccc 1380
   caccctcagc ctcccggcgc catttccctt cactgggggt gggggatggg gagctttcac 1440
   atggcggacg ctgccccgct ggggtgaaag tggggcgcgg aggcgggaat tcttattccc 1500
   ttt 1503
<210> 29
   <211> 15
   <212> DNA
   <213> Artificial Sequence
<220>
   <223> Splice acceptor consensus sequence
<400> 29
   yyyyyyyyyy yagrw 15
<210> 30
   <211> 11
   <212> DNA
   <213> Homo sapiens
<400> 30
   taaatatttt a 11
<210> 31
   <211> 19
   <212> DNA
   <213> Homo sapiens
<400> 31
   attataaata ttttaatta 19
<210> 32
   <211> 24
   <212> DNA
   <213> Homo sapiens
<400> 32
   atttaattat aaatatttta atta 24
<210> 33
   <211> 525
   <212> DNA
   <213> Homo sapiens
<400> 33
   atttataaaa tattgaatta taaaatatgt aattataaat actttaatta taaaatatgt 60
   aattataaat actttaatta taaaatatgt aattataaat actttataaa atatgtaatt 120
   ataaaatatg taattataaa cattttaatt ataaaatatg taattataaa cattttaatt 180
   ataaaatatg taattataaa cattttaatt ataaaatatg taattataaa cattttaatt 240
   ataaaatatg taattataaa cattttaatt ataaaatatt taattataaa cattttaatt 300
   ataaaatatt taattataaa tattttaatt ataaaatatt taattataaa tattttaatt 360
   ataaaatatt taattataaa tattttaatt ataaaatatt taattataaa tactttaatt 420
   ataaaatatt taattataaa tattttaatt ataaaatatt taattataaa tattttaatt 480
   ataaatattt taattataaa atatttaatt ataaaaacac aatta 525
<210> 34
   <211> 825
   <212> DNA
   <213> Homo sapiens
<400> 34
   gcaggctgag tgaaataaag gacttgttat ttcatctcga ggcctaccgg agagccttgc 60
   cttgcaaagg cagacagtca gtgaggaaga ctatgtggca catgaagaca ccagaggtgt 120
   tcctcaggat caaagtatgt acaagccttt gtgaatattt tttccttctc acttggcaaa 180
   tacaattcct gagatcaata acctcgtctt tttaattttt tcctcgtctt tttaactatt 240
   tataaaatat tgaattataa aatatgtaat tataaatact ttaattataa aatatgtaat 300
   tataaatact ttaattataa aatatgtaat tataaatact ttataaaata tgtaattata 360
   aaatatgtaa ttataaacat tttaattata aaatatgtaa ttataaacat tttaattata 420
   aaatatgtaa ttataaacat tttaattata aaatatgtaa ttataaacat tttaattata 480
   aaatatgtaa ttataaacat tttaattata aaatatttaa ttataaacat tttaattata 540
   aaatatttaa ttataaatat tttaattata aaatatttaa ttataaatat tttaattata 600
   aaatatttaa ttataaatat tttaattata aaatatttaa ttataaatac tttaattata 660
   aaatatttaa ttataaatat tttaattata aaatatttaa ttataaatat tttaattata 720
   aatattttaa ttataaaata tttaattata aaaacacaat tacctcatct ttttaaatat 780
   ttttgcaaaa tatttccctc cataatttct ccgtttccat tttta 825
<210> 35
   <211> 854
   <212> DNA
   <213> Homo sapiens
<400> 35
   cttctccact cctggcaggc tgagtgaaat aaaggacttg ttatttcatc tcgaggccta      60
   ccggagagcc ttgccttgca aaggcagaca gtcagtgagg aagactatgt ggcacatgaa     120
   gacaccagag gtgttcctca ggatcaaagt atgtacaagc ctttgtgaat attttttcct      180
   tctcacttgg caaatacaat tcctgagatc aataacctcg tctttttaat tttttcctcg     240
   tctttttaac tatttataaa atattgaatt ataaaatatg taattataaa tactttaatt     300
   ataaaatatg taattataaa tactttaatt ataaaatatg taattataaa tactttataa     360
   aatatgtaat tataaaatat gtaattataa acattttaat tataaaatat gtaattataa     420
   acattttaat tataaaatat gtaattataa acattttaat tataaaatat gtaattataa     480
   acattttaat tataaaatat gtaattataa acattttaat tataaaatat ttaattataa     540
   acattttaat tataaaatat ttaattataa atattttaat tataaaatat ttaattataa     600
   atattttaat tataaaatat ttaattataa atattttaat tataaaatat ttaattataa     660
   atactttaat tataaaatat ttaattataa atattttaat tataaaatat ttaattataa     720
   atattttaat tataaatatt ttaattataa aatatttaat tataaaaaca caattacctc     780
   atctttttaa atatttttgc aaaatatttc cctccataat ttctccgttt ccatttttat     840
   tctgttactt aaat                                                       854
<210> 36
   <211> 199
   <212> PRT
   <213> Artificial Sequence
<220>
   <223> puromycin acetyltransferase, synthetic construct
<400> 36
   Met Thr Glu Tyr Lys Pro Thr Val Arg Leu Ala Thr Arg Asp Asp Val
   1               5                   10                  15
   Pro Arg Ala Val Arg Thr Leu Ala Ala Ala Phe Ala Asp Tyr Pro Ala
               20                  25                  30
   Thr Arg His Thr Val Asp Pro Asp Arg His Ile Glu Arg Val Thr Glu
               35                  40                  45
   Leu Gln Glu Leu Phe Leu Thr Arg Val Gly Leu Asp Ile Gly Lys Val
           50                  55                  60
   Trp Val Ala Asp Asp Gly Ala Ala Val Ala Val Trp Thr Thr Pro Glu
   65                  70                  75                  80
   Ser Val Glu Ala Gly Ala Val Phe Ala Glu Ile Gly Pro Arg Met Ala
                   85                  90                  95
   Glu Leu Ser Gly Ser Arg Leu Ala Ala Gln Gln Gln Met Glu Gly Leu
               100                 105                 110
   Leu Ala Pro His Arg Pro Lys Glu Pro Ala Trp Phe Leu Ala Thr Val
           115                 120                 125
   Gly Val Ser Pro Asp His Gln Gly Lys Gly Leu Gly Ser Ala Val Val
       130                 135                 140
   Leu Pro Gly Val Glu Ala Ala Glu Arg Ala Gly Val Pro Ala Phe Leu
   145                 150                 155                 160
   Glu Thr Ser Ala Pro Arg Asn Leu Pro Phe Tyr Glu Arg Leu Gly Phe
                   165                 170                 175
   Thr Val Thr Ala Asp Val Glu Val Pro Glu Gly Pro Arg Thr Trp Cys
               180                 185                 190
   Met Thr Arg Lys Pro Gly Ala
           195
<210> 37
   <211> 600
   <212> DNA
   <213> Artificial Sequence
<220>
   <223> puromycin acetyltransferase encoding sequence, synthetic
<400> 37
   atgaccgagt acaagcccac ggtgcgcctc gccacccgcg acgacgtccc ccgggccgta      60
   cgcaccctcg ccgccgcgtt cgccgactac cccgccacgc gccacaccgt cgacccggac     120
   cgccacatcg agcgggtcac cgagctgcaa gaactcttcc tcacgcgcgt cgggctcgac     180
   atcggcaagg tgtgggtcgc ggacgacggc gccgcggtgg cggtctggac cacgccggag     240
   agcgtcgaag cgggggcggt gttcgccgag atcggcccgc gcatggccga gttgagcggt     300
   tcccggctgg ccgcgcagca acagatggaa ggcctcctgg cgccgcaccg gcccaaggag     360
   cccgcgtggt tcctggccac cgtcggcgtc tcgcccgacc accagggcaa gggtctgggc     420
   agcgccgtcg tgctccccgg agtggaggcg gccgagcgcg ccggggtgcc cgccttcctg     480
   gagacctccg cgccccgcaa cctccccttc tacgagcggc tcggcttcac cgtcaccgcc     540
   gacgtcgagg tgcccgaagg accgcgcacc tggtgcatga cccgcaagcc cggtgcctga     600
<210> 38
   <211> 1031
   <212> DNA
   <213> Artificial Sequence
<220>
   <223> anti-repressive element40
<400> 38
   gatcaagaaa gcactccggg ctccagaagg agccttccag gccagctttg agcataagct      60
   gctgatgagc agtgagtgtc ttgagtagtg ttcagggcag catgttacca ttcatgcttg     120
   acttctagcc agtgtgacga gaggctggag tcaggtctct agagagttga gcagctccag     180
   ccttagatct cccagtctta tgcggtgtgc ccattcgctt tgtgtctgca gtcccctggc     240
   cacacccagt aacagttctg ggatctatgg gagtagcttc cttagtgagc tttcccttca     300
   aatactttgc aaccaggtag agaagtttgg agtgaaggtt ttgttcttcg tttcttcaca     360
   atatggatat gcatcttctt ttgaaaatgt taaagtaaat tacctctctt ttcagatact     420
   gtcttcatgc gaacttggta tcctgtttcc atcccagcct tctataaccc agtaacatct     480
   tttttgaaac cagtgggtga gaaagacacc tggtcaggaa cgcggaccac aggacaactc     540
   aggctcaccc acggcatcag actaaaggca aacaaggact ctgtataaag taccggtggc     600
   atgtgtatta gtggagatgc agcctgtgct ctgcagacag ggagtcacac agacactttt     660
   ctataatttc ttaagtgctt tgaatgttca agtagaaagt ctaacattaa atttgattga     720
   acaattgtat attcatggaa tattttggaa cggaatacca aaaaatggca atagtggttc     780
   tttctggatg gaagacaaac ttttcttctt taaaataaat tttattttat atatttgagg     840
   ttgaccacat gaccttaagg atacatatag acagtaaact ggttactaca gtgaagcaaa     900
   ttaacatatc taccatcgta catagttaca tttttttgtg tgacaggaac agctaaaatc      960
   tacgtattta acaaaactcc taaagacaat acatttttat taactatagc cctcatgatg    1020
   tacattagat c                                                         1031
<210> 39
   <211> 1438
   <212> DNA
   <213> Artificial Sequence
<220>
   <223> CMV Promoter - S/MAR sequence
<400> 39
   ggcattgatt attgactagt tattaatagt aatcaattac ggggtcatta gttcatagcc 60
   catatatgga gttccgcgtt acataactta cggtaaatgg cccgcctggc tgaccgccca 120
   acgacccccg cccattgacg tcaataatga cgtatgttcc catagtaacg ccaataggga 180
   ctttccattg acgtcaatgg gtggagtatt tacggtaaac tgcccacttg gcagtacatc 240
   aagtgtatca tatgccaagt ccgcccccta ttgacgtcaa tgacggtaaa tggcccgcct 300
   ggcattatgc ccagtacatg accttacggg actttcctac ttggcagtac atctacgtat 360
   tagtcatcgc tattaccatg gtgatgcggt tttggcagta caccaatggg cgtggatagc 420
   ggtttgactc acggggattt ccaagtctcc accccattga cgtcaatggg agtttgtttt 480
   ggcaccaaaa tcaacgggac tttccaaaat gtcgtaataa ccccgccccg ttgacgcaaa 540
   tgggcggtag gcgtgtacgg tgggaggtct atataagcag aggtcttctc cactcctggc 600
   aggctgagtg aaataaagga cttgttattt catctcgagg cctaccggag agccttgcct 660
   tgcaaaggca gacagtcagt gaggaagact atgtggcaca tgaagacacc agaggtgttc 720
   ctcaggatca aagtatgtac aagcctttgt gaatattttt tccttctcac ttggcaaata 780
   caattcctga gatcaataac ctcgtctttt taattttttc ctcgtctttt taactattta 840
   taaaatattg aattataaaa tatgtaatta taaatacttt aattataaaa tatgtaatta 900
   taaatacttt aattataaaa tatgtaatta taaatacttt ataaaatatg taattataaa 960
   atatgtaatt ataaacattt taattataaa atatgtaatt ataaacattt taattataaa 1020
   atatgtaatt ataaacattt taattataaa atatgtaatt ataaacattt taattataaa 1080
   atatgtaatt ataaacattt taattataaa atatttaatt ataaacattt taattataaa 1140
   atatttaatt ataaatattt taattataaa atatttaatt ataaatattt taattataaa 1200
   atatttaatt ataaatattt taattataaa atatttaatt ataaatactt taattataaa 1260
   atatttaatt ataaatattt taattataaa atatttaatt ataaatattt taattataaa 1320
   tattttaatt ataaaatatt taattataaa aacacaatta cctcatcttt ttaaatattt 1380
   ttgcaaaata tttccctcca taatttctcc gtttccattt ttattctgtt acttaaat 1438
<210> 40
   <211> 2038
   <212> DNA
   <213> Artificial Sequence
<220>
   <223> CMV Promoter - Puromycin - S/MAR sequence
<400> 40
   ggcattgatt attgactagt tattaatagt aatcaattac ggggtcatta gttcatagcc      60
   catatatgga gttccgcgtt acataactta cggtaaatgg cccgcctggc tgaccgccca     120
   acgacccccg cccattgacg tcaataatga cgtatgttcc catagtaacg ccaataggga     180
   ctttccattg acgtcaatgg gtggagtatt tacggtaaac tgcccacttg gcagtacatc     240
   aagtgtatca tatgccaagt ccgcccccta ttgacgtcaa tgacggtaaa tggcccgcct     300
   ggcattatgc ccagtacatg accttacggg actttcctac ttggcagtac atctacgtat     360
   tagtcatcgc tattaccatg gtgatgcggt tttggcagta caccaatggg cgtggatagc     420
   ggtttgactc acggggattt ccaagtctcc accccattga cgtcaatggg agtttgtttt     480
   ggcaccaaaa tcaacgggac tttccaaaat gtcgtaataa ccccgccccg ttgacgcaaa     540
   tgggcggtag gcgtgtacgg tgggaggtct atataagcag aggtatgacc gagtacaagc     600
   ccacggtgcg cctcgccacc cgcgacgacg tcccccgggc cgtacgcacc ctcgccgccg      660
   cgttcgccga ctaccccgcc acgcgccaca ccgtcgaccc ggaccgccac atcgagcggg      720
   tcaccgagct gcaagaactc ttcctcacgc gcgtcgggct cgacatcggc aaggtgtggg      780
   tcgcggacga cggcgccgcg gtggcggtct ggaccacgcc ggagagcgtc gaagcggggg      840
   cggtgttcgc cgagatcggc ccgcgcatgg ccgagttgag cggttcccgg ctggccgcgc      900
   agcaacagat ggaaggcctc ctggcgccgc accggcccaa ggagcccgcg tggttcctgg      960
   ccaccgtcgg cgtctcgccc gaccaccagg gcaagggtct gggcagcgcc gtcgtgctcc     1020
   ccggagtgga ggcggccgag cgcgccgggg tgcccgcctt cctggagacc tccgcgcccc     1080
   gcaacctccc cttctacgag cggctcggct tcaccgtcac cgccgacgtc gaggtgcccg     1140
   aaggaccgcg cacctggtgc atgacccgca agcccggtgc ctgacttctc cactcctggc     1200
   aggctgagtg aaataaagga cttgttattt catctcgagg cctaccggag agccttgcct     1260
   tgcaaaggca gacagtcagt gaggaagact atgtggcaca tgaagacacc agaggtgttc     1320
   ctcaggatca aagtatgtac aagcctttgt gaatattttt tccttctcac ttggcaaata     1380
   caattcctga gatcaataac ctcgtctttt taattttttc ctcgtctttt taactattta     1440
   taaaatattg aattataaaa tatgtaatta taaatacttt aattataaaa tatgtaatta     1500
   taaatacttt aattataaaa tatgtaatta taaatacttt ataaaatatg taattataaa     1560
   atatgtaatt ataaacattt taattataaa atatgtaatt ataaacattt taattataaa     1620
   atatgtaatt ataaacattt taattataaa atatgtaatt ataaacattt taattataaa     1680
   atatgtaatt ataaacattt taattataaa atatttaatt ataaacattt taattataaa     1740
   atatttaatt ataaatattt taattataaa atatttaatt ataaatattt taattataaa     1800
   atatttaatt ataaatattt taattataaa atatttaatt ataaatactt taattataaa     1860
   atatttaatt ataaatattt taattataaa atatttaatt ataaatattt taattataaa     1920
   tattttaatt ataaaatatt taattataaa aacacaatta cctcatcttt ttaaatattt     1980
   ttgcaaaata tttccctcca taatttctcc gtttccattt ttattctgtt acttaaat       2038
<210> 41
   <211> 6189
   <212> DNA
   <213> Artificial Sequence
<220>
   <223> Element40-GPF-P2A-Puromycin-S/MAR
<400> 41
   ggcattgatt attgactagt tattaatagt aatcaattac ggggtcatta gttcatagcc 60
   catatatgga gttccgcgtt acataactta cggtaaatgg cccgcctggc tgaccgccca 120
   acgacccccg cccattgacg tcaataatga cgtatgttcc catagtaacg ccaataggga 180
   ctttccattg acgtcaatgg gtggagtatt tacggtaaac tgcccacttg gcagtacatc 240
   aagtgtatca tatgccaagt ccgcccccta ttgacgtcaa tgacggtaaa tggcccgcct 300
   ggcattatgc ccagtacatg accttacggg actttcctac ttggcagtac atctacgtat 360
   tagtcatcgc tattaccatg gtgatgcggt tttggcagta caccaatggg cgtggatagc 420
   ggtttgactc acggggattt ccaagtctcc accccattga cgtcaatggg agtttgtttt 480
   ggcaccaaaa tcaacgggac tttccaaaat gtcgtaataa ccccgccccg ttgacgcaaa 540
   tgggcggtag gcgtgtacgg tgggaggtct atataagcag aggtcgttta gtgaaccgtc 600
   agatcactag tagctttatt gcggtagttt atcacagtta aattgctaac gcagtcagtg 660
   ctcgactgat cacaggtaag tatcaaggtt acaagacagg tttaaggagg ccaatagaaa 720
   ctgggcttgt cgagacagag aagattcttg cgtttctgat aggcacctat tggtcttact 780
   gacatccact ttgcctttct ctccacaggg gtaccgaagc cgctagcgct accggtcgcc 840
   accatgcccg ccatgaagat cgagtgccgc atcaccggca ccctgaacgg cgtggagttc 900
   gagctggtgg gcggcggaga gggcaccccc gagcagggcc gcatgaccaa caagatgaag 960
   agcaccaaag gcgccctgac cttcagcccc tacctgctga gccacgtgat gggctacggc 1020
   ttctaccact tcggcaccta ccccagcggc tacgagaacc ccttcctgca cgccatcaac 1080
   aacggcggct acaccaacac ccgcatcgag aagtacgagg acggcggcgt gctgcacgtg 1140
   agcttcagct accgctacga ggccggccgc gtgatcggcg acttcaaggt ggtgggcacc 1200
   ggcttccccg aggacagcgt gatcttcacc gacaagatca tccgcagcaa cgccaccgtg 1260
   gagcacctgc accccatggg cgataacgtg ctggtgggca gcttcgcccg caccttcagc 1320
   ctgcgcgacg gcggctacta cagcttcgtg gtggacagcc acatgcactt caagagcgcc 1380
   atccacccca gcatcctgca gaacgggggc cccatgttcg ccttccgccg cgtggaggag 1440
   ctgcacagca acaccgagct gggcatcgtg gagtaccagc acgccttcaa gacccccatc 1500
   gccttcgcca gatctggcag cggcgccacc aacttcagcc tgctgaagca ggccggcgac 1560
   gtggaggaaa accctggccc catgaccgag tacaagccca cggtgcgcct cgccacccgc 1620
   gacgacgtcc cccgggccgt acgcaccctc gccgccgcgt tcgccgacta ccccgccacg 1680
   cgccacaccg tcgacccgga ccgccacatc gagcgggtca ccgagctgca agaactcttc 1740
   ctcacgcgcg tcgggctcga catcggcaag gtgtgggtcg cggacgacgg cgccgcggtg 1800
   gcggtctgga ccacgccgga gagcgtcgaa gcgggggcgg tgttcgccga gatcggcccg 1860
   cgcatggccg agttgagcgg ttcccggctg gccgcgcagc aacagatgga aggcctcctg 1920
   gcgccgcacc ggcccaagga gcccgcgtgg ttcctggcca ccgtcggcgt ctcgcccgac 1980
   caccagggca agggtctggg cagcgccgtc gtgctccccg gagtggaggc ggccgagcgc 2040
   gccggggtgc ccgccttcct ggagacctcc gcgccccgca acctcccctt ctacgagcgg 2100
   ctcggcttca ccgtcaccgc cgacgtcgag gtgcccgaag gaccgcgcac ctggtgcatg 2160
   acccgcaagc ccggtgcctg aagatctatg catgcagaag ttggtcgtga ggcactgggc 2220
   aggtaagtat caaggttaca agacaggtcg acttctccac tcctggcagg ctgagtgaaa 2280
   taaaggactt gttatttcat ctcgaggcct accggagagc cttgccttgc aaaggcagac 2340
   agtcagtgag gaagactatg tggcacatga agacaccaga ggtgttcctc aggatcaaag 2400
   tatgtacaag cctttgtgaa tattttttcc ttctcacttg gcaaatacaa ttcctgagat 2460
   caataacctc gtctttttaa ttttttcctc gtctttttaa ctatttataa aatattgaat 2520
   tataaaatat gtaattataa atactttaat tataaaatat gtaattataa atactttaat 2580
   tataaaatat gtaattataa atactttata aaatatgtaa ttataaaata tgtaattata 2640
   aacattttaa ttataaaata tgtaattata aacattttaa ttataaaata tgtaattata 2700
   aacattttaa ttataaaata tgtaattata aacattttaa ttataaaata tgtaattata 2760
   aacattttaa ttataaaata tttaattata aacattttaa ttataaaata tttaattata 2820
   aatattttaa ttataaaata tttaattata aatattttaa ttataaaata tttaattata 2880
   aatattttaa ttataaaata tttaattata aatactttaa ttataaaata tttaattata 2940
   aatattttaa ttataaaata tttaattata aatattttaa ttataaatat tttaattata 3000
   aaatatttaa ttataaaaac acaattacct catcttttta aatatttttg caaaatattt 3060
   ccctccataa tttctccgtt tccattttta ttctgttact taaataattc tgcagtcgac 3120
   ggttactgac atccactttg cctttctctc cacaggtgtc cactctaccg cgggcccggg 3180
   atccaccgga tctagataac tgatcataat cagccatacc acatttgtag aggttttact 3240
   tgctttaaaa aacctcccac acctccccct gaacctgaaa cataaaatga atgcaattgt 3300
   tgttgttaac ttgtttattg cagcttataa tggttacaaa taaagcaata gcatcacaaa 3360
   tttcacaaat aaagcatttt tttcactgca ttctagttgt ggtttgtcca aactcatcaa 3420
   tgtatcttac atgtgagcaa aaggccagca aaaggccagg aaccgtaaaa aggccgcgtt 3480
   gctggcgttt ttccataggc tccgcccccc tgacgagcat cacaaaaatc gacgctcaag 3540
   tcagaggtgg cgaaacccga caggactata aagataccag gcgtttcccc ctggaagctc 3600
   cctcgtgcgc tctcctgttc cgaccctgcc gcttaccgga tacctgtccg cctttctccc 3660
   ttcgggaagc gtggcgcttt ctcatagctc acgctgtagg tatctcagtt cggtgtaggt 3720
   cgttcgctcc aagctgggct gtgtgcacga accccccgtt cagcccgacc gctgcgcctt 3780
   atccggtaac tatcgtcttg agtccaaccc ggtaagacac gacttatcgc cactggcagc 3840
   agccactggt aacaggatta gcagagcgag gtatgtaggc ggtgctacag agttcttgaa 3900
   gtggtggcct aactacggct acactagaag aacagtattt ggtatctgcg ctctgctgaa 3960
   gccagttacc ttcggaaaaa gagttggtag ctcttgatcc ggcaaacaaa ccaccgctgg 4020
   tagcggtggt ttttttgttt gcaagcagca gattacgcgc agaaaaaaag gatctcaaga 4080
   agatcctttg atcttttcta cggggtctga cgctcagtgg aacgaaaact cacgttaagg 4140
   gattttggtc atgccgtctc agaagaactc gtcaagaagg cgatagaagg cgatgcgctg 4200
   cgaatcggga gcggcgatac cgtaaagcac gaggaagcgg tcagcccatt cgccgccaag 4260
   ctcttcagca atatcacggg tagccaacgc tatgtcctga tagcggtccg ccacacccag 4320
   ccggccacag tcgatgaatc cagaaaagcg gccattttcc accatgatat tcggcaagca 4380
   ggcatcgcca tgggtcacga cgagatcctc gccgtcgggc atgctcgcct tgagcctggc 4440
   gaacagttcg gctggcgcga gcccctgatg ctcttcgtcc agatcatcct gatcgacaag 4500
   accggcttcc atccgagtac gtgctctctc gatgcgatgt ttcgcttggt ggtcgaatgg 4560
   gcaggtagcc ggatcaagcg tatgcagccg ccgcattgca tcagccatga tggatacttt 4620
   ctcggcagga gcaaggtgag atgacaggag atcctgcccc ggcacttcgc ccaatagcag 4680
   ccagtccctt cccgcttcag tgacaacgtc gagtacagct gcgcaaggaa cgcccgtcgt 4740
   ggccagccac gatagccgcg ctgcctcgtc ttgcagttca ttcagggcac cggacaggtc 4800
   ggtcttgaca aaaagaaccg ggcgcccctg cgctgacagc cggaacacgg cggcatcaga 4860
   gcagccgatt gtctgttgtg cccagtcata gccgaatagc ctctccaccc aagcggccgg 4920
   agaacctgcg tgcaatccat cttgttcaat cataatatta ttgaagcatt tatcagggtt 4980
   cgtctcgtcc cggtctcctc ccatgcatgt caatattggc cattagccat attattcatt 5040
   ggttatatag cataaatcaa tattggctat tggccattgc atacgttgta tctatatcat 5100
   aatatgtaca aagcttgatc aagaaagcac tccgggctcc agaaggagcc ttccaggcca 5160
   gctttgagca taagctgctg atgagcagtg agtgtcttga gtagtgttca gggcagcatg 5220
   ttaccattca tgcttgactt ctagccagtg tgacgagagg ctggagtcag gtctctagag 5280
   agttgagcag ctccagcctt agatctccca gtcttatgcg gtgtgcccat tcgctttgtg 5340
   tctgcagtcc cctggccaca cccagtaaca gttctgggat ctatgggagt agcttcctta 5400
   gtgagctttc ccttcaaata ctttgcaacc aggtagagaa gtttggagtg aaggttttgt 5460
   tcttcgtttc ttcacaatat ggatatgcat cttcttttga aaatgttaaa gtaaattacc 5520
   tctcttttca gatactgtct tcatgcgaac ttggtatcct gtttccatcc cagccttcta 5580
   taacccagta acatcttttt tgaaaccagt gggtgagaaa gacacctggt caggaacgcg 5640
   gaccacagga caactcaggc tcacccacgg catcagacta aaggcaaaca aggactctgt 5700
   ataaagtacc ggtggcatgt gtattagtgg agatgcagcc tgtgctctgc agacagggag 5760
   tcacacagac acttttctat aatttcttaa gtgctttgaa tgttcaagta gaaagtctaa 5820
   cattaaattt gattgaacaa ttgtatattc atggaatatt ttggaacgga ataccaaaaa     5880
   atggcaatag tggttctttc tggatggaag acaaactttt cttctttaaa ataaatttta     5940
   ttttatatat ttgaggttga ccacatgacc ttaaggatac atatagacag taaactggtt     6000
   actacagtga agcaaattaa catatctacc atcgtacata gttacatttt tttgtgtgac     6060
   aggaacagct aaaatctacg tatttaacaa aactcctaaa gacaatacat ttttattaac     6120
   tatagccctc atgatgtaca ttagatctgt acatttatat tggctcatgt ccaatatgac     6180
   cgccatgtt                                                              6189